# EUROPEAN PATENT APPLICATION

(11) **EP 4 008 719 A2**
(43) Date of publication of application: **08.06.2022**
(21) Application number: 20831960.8
(22) Date of filing: 28.08.2020
(51) Int. Cl.: C07D 487/04, C07D 519/00, C07D 471/04, A61K 31/519, A61P 35/00

(54) **PYRAZOLONE AND PYRIMIDINE COMPOUND, AND PREPARATION METHOD AND USE THEREFOR**

(30) Priority: 28.06.2019 CN 201910578299
(71) Applicant: Shanghai Pharmaceuticals Holding Co., Ltd., Shanghai 201203 (CN)
(72) Inventor: WANG, Qian, Shanghai 201203 (CN); HUO, Guoyong, Shanghai 201203 (CN); XIA, Guangxin, Shanghai 201203 (CN); LOU, Jiangsong, Shanghai 201203 (CN); SHU, Sijie, Shanghai 201203 (CN); GE, Hui, Shanghai 201203 (CN); ZHANG, Lin, Shanghai 201203 (CN); SHI, Chen, Shanghai 201203 (CN); ZHANG, Zhihui, Shanghai 201203 (CN); MAO, Yu, Shanghai 201203 (CN); ZHANG, Bingbin, Shanghai 201203 (CN); YU, Jianxin, Shanghai 201203 (CN); LIU, Yanjun, Shanghai 201203 (CN); KE, Ying, Shanghai 201203 (CN); ZHANG, Chi, Shanghai 201203 (CN)
(74) Representative: Dehns
(86) International application number: PCT/CN2020/112034
(87) International publication number: WO 2020/259724

(57) **Abstract**

Disclosed in the present invention are a pyrazolone and pyrimidine compound, and a preparation method and a use therefor. Provided in the present invention is a pyrazolone and pyrimidine compound as in formula (I), said compound having better inhibitory activity towards WEE1 kinase.

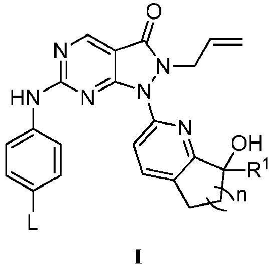

## Description

The present application claims the benefit of Chinese patent application CN201910578299.1 filed on June 28, 2019. The contents of the Chinese patent application are incorporated herein by reference in their entireties.

### Technical Field

The present disclosure relates to a pyrazolone-fused pyrimidine compound, a preparation method therefor and an application thereof.

### Background

Cell cycle is closely related to DNA damage repair process. Cell cycle refers to the whole process of cell division, which is divided into two stages: interphase and mitotic phase (M). Cell cycle checkpoint is a key point to regulate the cell cycle, its main function is to ensure that every event in the cycle can be completed on time and orderly, and to adjust the cell state to adapt to the external environment.

The main checkpoints of cells are as follows: 1) G1/S checkpoint, which is called R (restriction) point in mammals and controls cells from static G1 phase to DNA synthesis phase; 2) S-phase checkpoint: whether DNA replication is completed; 3) G2/M checkpoint: it is the control point that regulates the cell to enter the division stage; 4) middle-late checkpoint: also called spindle assembly checkpoint, if the centromere is not connected to the spindle correctly, it will cause the interruption of cell cycle. If there is abnormality in some processes of cell division cycle, such as DNA damage, the checkpoint will sense in time and start repair. P53 protein is an important protein that regulates G1 checkpoint, when DNA is damaged, P53 protein prevents cells from entering S phase and activates DNA repair mechanism, which is very important for maintaining the integrity of cell genome. However, since P53 mutation often exists in tumor cells, which makes G1 checkpoint defective, therefore, the regulation of cell division cycle in P53 mutated cells depends on G 2 /M checkpoint.

WEE1 kinase is a cell cycle regulatory protein, which can regulate the phosphorylation state of cyclin-dependent kinase 1 (CDK1), thus regulating the activity of CDK1 and cyclin B complex, realizing the regulation of cell cycle, and playing an important role in regulating DNA damage checkpoints. WEE1 is a key gene in G2/M phase block, plays an important monitoring role, and is overexpressed in some cancers, inhibition or downregulation of WEE1 kinase may trigger mitotic catastrophe, so WEE1 kinase inhibitors have a key role in anticancer therapy and have become a hot spot for the development of anticancer agents.

International patent applications WO2019037678, WO2019028008, WO2018133829, WO2010098367, WO2010067886, WO2008115742, WO2008115738, WO2007126122, WO2007126128 WO2004007499 and others have disclosed part of small molecule WEE1 kinase inhibitors, but there are no small molecule WEE1 kinase inhibitors on the market, and there is still a need in the art to develop new WEE1 kinase inhibitors with good anticancer activity and high safety.

### Content of the present invention

The technical problem to be solved by the present disclosure is that the existing compounds with inhibitory activity against WEE1 kinase have a single structure, therefore, the present disclosure provides a pyrazolone-fused pyrimidine compound, a preparation method therefor and an application thereof, and the compound has a better inhibitory activity against WEE1 kinase.

The present disclosure provides a pyrazolone-fused pyrimidine compound represented by formula I, a pharmaceutically acceptable salt thereof, a solvate thereof or a solvate of the pharmaceutically acceptable salt thereof: 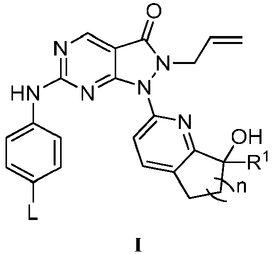
wherein R¹ is hydrogen or methyl;
n is 1 or 2;
L is -(CH₂)ₘ-R², C₃-C₂₀ cycloalkyl, "C₃-C₂₀ cycloalkyl substituted by one or two R³", C₃-C₁₄ heterocycloalkyl or "C₃-C₁₄ heterocycloalkyl substituted by one or two R⁴";
m is 0, 1, 2, 3 or 4;
R², R³ and R⁴ are independently -CN, -NR²⁻¹R²⁻², -(C=O)R²⁻³, -(C=NR²⁻⁴)R²⁻⁵, -S(=O)₂R²⁻⁶, -OR²⁻⁷, -SR²⁻⁸ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁹;
R²⁻¹ and R²⁻² are independently hydrogen, -(C=O)R²⁻¹⁻¹, -S(=O)₂R²⁻¹⁻², -(C=NR²⁻¹⁻³)R²⁻¹⁻⁴ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl, or C₁-C₇ heteroaryl" optionally substituted by one, two or three R²⁻¹⁻⁵;
or, R²⁻¹ and R²⁻² together with the nitrogen atom they are attached to form a C₃-C₁₄ heterocycloalkyl optionally substituted by one, two or three R²⁻¹⁻⁶; one or more methylenes in the C₃-C₁₄ heterocycloalkyl optionally and independently substituted by oxygen atom, sulfur atom, sulfinyl, sulfonyl, carbonyl, vinylidene or -N(R²⁻¹⁻⁷)-; R²⁻¹⁻⁷ is independently C₁-C₇ alkyl or C₃-C₁₄ cycloalkyl;
R²⁻¹⁻¹, R²⁻¹⁻² and R²⁻¹⁻⁴ are independently hydrogen, -NR²⁻¹⁻¹⁻¹R²⁻¹⁻¹⁻² or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl, or C₁-C₇ heteroaryl" optionally substituted by one, two or three R²⁻¹⁻¹⁻³;
R²⁻¹⁻¹⁻¹ and R²⁻¹⁻¹⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻¹⁻¹⁻³ is independently halogen, hydroxyl, mercapto, cyano, amino, C₁-C₇ alkyl, C₁-C₇ alkoxy, C₁-C₇ alkylthio, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻¹⁻³ is selected from hydrogen, cyano, -OH, C₁-C₇ alkyl or C₁-C₇ alkoxy;
R²⁻¹⁻⁵ and R²⁻¹⁻⁶ are independently cyano, halogen, -NR²⁻¹⁻⁵⁻¹R²⁻¹⁻⁵⁻², -OR²⁻¹⁻⁵⁻³, -SR²⁻¹⁻⁵⁻⁴ or C₁-C₇ alkyl;
R²⁻¹⁻⁵⁻¹, R²⁻¹⁻⁵⁻², R²⁻¹⁻⁵⁻³ and R²⁻¹⁻⁵⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻³ is independently hydrogen, -NR²⁻³⁻¹R²⁻³⁻², -OR²⁻³⁻³ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻³⁻⁴;
R²⁻³⁻¹ and R²⁻³⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻³⁻¹ and R²⁻³⁻² together with the nitrogen atom they are attached to form a C₃-C₁₄ heterocycloalkyl optionally substituted by one, two or three R²⁻³⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻³⁻¹⁻²)-; R²⁻³⁻¹⁻² is independently C₁-C₇ alkyl or C₃-C₁₄ cycloalkyl;
R²⁻³⁻¹⁻¹ is independently cyano, halogen, -NR²⁻³⁻¹⁻¹⁻¹R²⁻³⁻¹⁻¹⁻², -OR²⁻³⁻¹⁻¹⁻³, -SR²⁻³⁻¹⁻¹⁻⁴ or C₁-C₇ alkyl;
R²⁻³⁻¹⁻¹⁻¹, R²⁻³⁻¹⁻¹⁻², R²⁻³⁻¹⁻¹⁻³ and R²⁻³⁻¹⁻¹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻³⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻³⁻⁴ is independently cyano, halogen, -NR²⁻³⁻⁴⁻¹R²⁻³⁻⁴⁻², -OR²⁻³⁻⁴⁻³, -SR²⁻³⁻⁴⁻⁴ or C₁-C₇ alkyl;
R²⁻³⁻⁴⁻¹, R²⁻³⁻⁴⁻², R²⁻³⁻⁴⁻³ and R²⁻³⁻⁴⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁴ is independently hydrogen, -CN, -OR²⁻⁴⁻¹ or C₁-C₇ alkyl;
R²⁻⁴⁻¹ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁵ is independently -NR²⁻⁵⁻¹R²⁻⁵⁻², -OR²⁻⁵⁻³ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁵⁻⁴;
R²⁻⁵⁻¹ and R²⁻⁵⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻⁵⁻¹ and R²⁻⁵⁻² together with the nitrogen atom they are attached to form a C₃-C₁₄ heterocycloalkyl optionally substituted by one, two or three R²⁻⁵⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻⁵⁻¹⁻²)-; R²⁻⁵⁻¹⁻² is independently C₁-C₇ alkyl or C₃-C₁₄ cycloalkyl;
R²⁻⁵⁻¹⁻¹ is independently cyano, halogen, -NR²⁻⁵⁻¹⁻¹⁻¹R²⁻⁵⁻¹⁻¹⁻², -OR²⁻⁵⁻¹⁻¹⁻³, -SR²⁻⁵⁻¹⁻¹⁻⁴ or C₁-C₇ alkyl;
R²⁻⁵⁻¹⁻¹⁻¹, R²⁻⁵⁻¹⁻¹⁻², R²⁻⁵⁻¹⁻¹⁻³ and R²⁻⁵⁻¹⁻¹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁵⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁵⁻⁴ is independently cyano, halogen, -NR²⁻⁵⁻⁴⁻¹R²⁻⁵⁻⁴⁻², -OR²⁻⁵⁻⁴⁻³, -SR²⁻⁵⁻⁴⁻⁴ or C₁-C₇ alkyl;
R²⁻⁵⁻⁴⁻¹, R²⁻⁵⁻⁴⁻², R²⁻⁵⁻⁴⁻³ and R²⁻⁵⁻⁴⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁶ is independently -NR²⁻⁶⁻¹R²⁻⁶⁻², -OR²⁻⁶⁻³ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁶⁻⁴;
R²⁻⁶⁻¹ and R²⁻⁶⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻⁶⁻¹ and R²⁻⁶⁻² together with the nitrogen atom they are attached to form a C₃-C₁₄ heterocycloalkyl optionally substituted by one, two or three R²⁻⁶⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻⁶⁻¹⁻²)-; R²⁻⁶⁻¹⁻² is independently C₁-C₇ alkyl or C₃-C₁₄ cycloalkyl;
R²⁻⁶⁻¹⁻¹ is independently cyano, halogen, -NR²⁻⁶⁻¹⁻¹⁻¹R²⁻⁶⁻¹⁻¹⁻², -OR²⁻⁶⁻¹⁻¹⁻³, -SR²⁻⁶⁻¹⁻¹⁻⁴ or C₁-C₇ alkyl;
R²⁻⁶⁻¹⁻¹⁻¹, R²⁻⁶⁻¹⁻¹⁻², R²⁻⁶⁻¹⁻¹⁻³ and R²⁻⁶⁻¹⁻¹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁶⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁶⁻⁴ is independently cyano, halogen, -NR²⁻⁶⁻⁴⁻¹R²⁻⁶⁻⁴⁻², -OR²⁻⁶⁻⁴⁻³, -SR²⁻⁶⁻⁴⁻⁴ or C₁-C₇ alkyl;
R²⁻⁶⁻⁴⁻¹, R²⁻⁶⁻⁴⁻², R²⁻⁶⁻⁴⁻³ and R²⁻⁶⁻⁴⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁷ and R²⁻⁸ are independently hydrogen or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl, or C₁-C₇ heteroaryl" optionally substituted by one, two or three R²⁻⁷⁻¹;
R²⁻⁷⁻¹ is independently cyano, halogen, -NR²⁻⁷⁻¹⁻¹R²⁻⁷⁻¹⁻², -OR²⁻⁷⁻¹⁻³, -SR²⁻⁷⁻¹⁻⁴ or C₁-C₇ alkyl;
R²⁻⁷⁻¹⁻¹, R²⁻⁷⁻¹⁻², R²⁻⁷⁻¹⁻³ and R²⁻⁷⁻¹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹ is independently -OR²⁻⁹⁻¹, -NR²⁻⁹⁻²R²⁻⁹⁻³, -SR²⁻⁹⁻⁴, halogen, cyano, -(C=O)R²⁻⁹⁻⁵, -(C=NR²⁻⁹⁻⁶)R²⁻⁹⁻⁷, -S(=O)₂R²⁻⁹⁻⁸, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻¹, R²⁻⁹⁻², R²⁻⁹⁻³ and R²⁻⁹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁵ is independently hydrogen, -NR²⁻⁹⁻⁵⁻¹R²⁻⁹⁻⁵⁻², -OR²⁻⁹⁻⁵⁻³ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁹⁻⁵⁻⁴;
R²⁻⁹⁻⁵⁻¹, R²⁻⁹⁻⁵⁻² and R²⁻⁹⁻⁵⁻³ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁵⁻⁴ is independently hydrogen, halogen, hydroxyl, mercapto, cyano, amino, C₁-C₇ alkyl, C₁-C₇ alkoxy, C₁-C₇ alkylthio, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁶ is independently hydrogen, -CN, -OR²⁻⁹⁻⁶⁻¹ or C₁-C₇ alkyl;
R²⁻⁹⁻⁶⁻¹ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁷ is independently -NR²⁻⁹⁻⁷⁻¹R²⁻⁹⁻⁷⁻², -OR²⁻⁹⁻⁷⁻³, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁷⁻¹ and R²⁻⁹⁻⁷⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻⁹⁻⁷⁻¹ and R²⁻⁹⁻⁷⁻² together with the nitrogen atom they are attached to form a C₃-C₁₄ heterocycloalkyl optionally substituted by one, two or three R²⁻⁹⁻⁷⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻⁹⁻⁷⁻¹⁻²)-; R²⁻⁹⁻⁷⁻¹⁻² is independently C₁-C₇ alkyl or C₃-C₁₄ cycloalkyl;
R²⁻⁹⁻⁷⁻¹⁻¹ is independently halogen, hydroxyl, mercapto, cyano, amino, C₁-C₇ alkyl, C₁-C₇ alkoxy, C₁-C₇ alkylthio, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁷⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻¹ is independently -NR²⁻⁹⁻¹¹⁻¹R²⁻⁹⁻⁸⁻², -OR²⁻⁹⁻⁸⁻³, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻¹¹⁻¹ and R²⁻⁹⁻¹¹⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻⁹⁻⁸⁻¹ and R²⁻⁹⁻¹¹⁻² together with the nitrogen atom they are attached to form a C₃-C₁₄ heterocycloalkyl optionally substituted by one, two or three R²⁻⁹⁻⁸⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻⁹⁻⁸⁻¹⁻²)-; R²⁻⁹⁻⁸⁻¹⁻² is independently C₁-C₇ alkyl or C₃-C₁₄ cycloalkyl;
R²⁻⁹⁻⁸⁻¹⁻¹ is independently halogen, hydroxyl, mercapto, cyano, amino, C₁-C₇ alkyl, C₁-C₇ alkoxy, C₁-C₇ alkylthio, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻¹¹⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
in any one of the above cases, the heteroatoms in the C₃-C₁₄ heterocycloalkyl, C₁-C₇ heteroaryl are independently selected from one or more of boron, silicon, oxygen, sulfur, selenium, nitrogen and phosphorus; the number of heteroatoms is independently 1, 2, 3 or 4.

In a certain scheme, some substituents in the pyrazolone-fused pyrimidine compound represented by formula **I,** the pharmaceutically acceptable salt thereof, the solvate thereof or the solvate of the pharmaceutically acceptable salt thereof may further have the following definitions, and the definitions of substituents not involved below are as described in any of the above schemes (hereinafter referred to as "in a certain scheme"):
when L is C₃-C₂₀ cycloalkyl substituted by one or two R³, the C₃-C₂₀ cycloalkyl is for example C₃-C₂₀ monocyclic cycloalkyl, C₃-C₂₀ spiro cycloalkyl, C₃-C₂₀ fused cycloalkyl or C₃-C₂₀ bridged cycloalkyl.

The C₃-C₂₀ monocyclic cycloalkyl is for example C₃-C₆ monocyclic cycloalkyl, for example cyclopropyl, cyclobutyl, cyclopentyl or cyclohexyl, for example cyclohexyl.

The C₃-C₂₀ bridged cycloalkyl is for example C₅-C₈ bridged cycloalkyl, for example 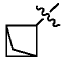 or

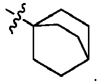

In a certain scheme:
when L is C₃-C₂₀ cycloalkyl substituted by one or two R³, the C₃-C₂₀ cycloalkyl is for example C₃-C₂₀ saturated cycloalkyl.

In a certain scheme:
when R²⁻¹ and R²⁻² are independently C₁-C₇ alkyl, the C₁-C₇ alkyl is for example C₁-C₃ alkyl, for example methyl, ethyl, *n*-propyl or isopropyl, for example methyl.

In a certain scheme:
when L is C₃-C₂₀ cycloalkyl substituted by one R³, R³ is -NR²⁻¹R²⁻²; the "C₃-C₂₀ cycloalkyl substituted by one R^{3"} is, for example

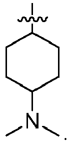

In a certain scheme:
when L is C₃-C₁₄ heterocycloalkyl, the C₃-C₁₄ heterocycloalkyl is for example C₃-C₁₄ monocyclic heterocycloalkyl, C₃-C₁₄ spiro heterocycloalkyl, C₃-C₁₄ fused heterocycloalkyl or C₃-C₁₄ bridged heterocycloalkyl.

The C₃-C₁₄ monocyclic heterocycloalkyl is, for example, "C₃-C₉ monocyclic heterocycloalkyl having one or two heteroatoms selected from one or two of N, O and S", for example, "C₃-C₅ monocyclic heterocycloalkyl having one or two heteroatoms selected from one or two of N, O and S", for example, "C₃-C₅ monocyclic heterocycloalkyl having one or two heteroatoms selected from one or two of N, O and S" and which is connected to a benzene ring by a nitrogen atom, for example, morpholinyl, piperidinyl or piperazinyl.

The morpholinyl is for example 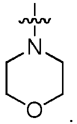 The piperidinyl is for example 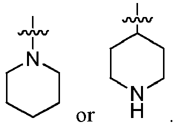 The piperazinyl is for example

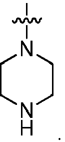

The C₃-C₁₄ spiro heterocycloalkyl is, for example, "C₅-C₉ spiro heterocycloalkyl having one or two heteroatoms selected from one or two of N, O and S", for example, "C₇-C₉ spiro heterocycloalkyl having one or two heteroatoms selected from one or two of N, O and S", for example, "C₇-C₉ spiro heterocycloalkyl having one or two heteroatoms selected from one or two of N, O and S" and which is connected to a benzene ring by a nitrogen atom, for example, 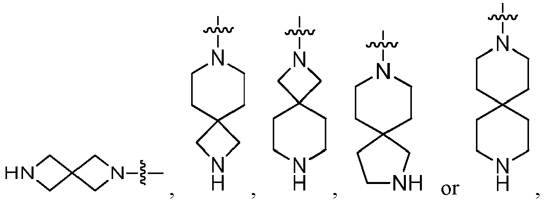 for example,

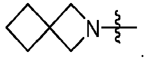

The C₃-C₁₄ fused heterocycloalkyl is, for example, "C₆-C₈ fused heterocycloalkyl having one or two heteroatoms selected from one or two of N, O and S", for example, "C₆-C₈ fused heterocycloalkyl having one or two heteroatoms selected from one or two of N, O and S" and which is connected to a benzene ring by a nitrogen atom, for example, 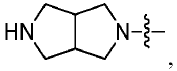 for example,

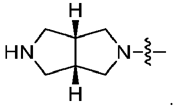

The C₃-C₁₄ bridged heterocycloalkyl is, for example, "C₄-C₆ bridged heterocycloalkyl having one or two heteroatoms selected from one or two of N, O and S", for example, "C₄-C₆ bridged heterocycloalkyl having one or two heteroatoms selected from one or two of N, O and S" and which is connected to a benzene ring by a nitrogen atom, for example,

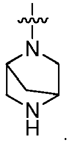

In a certain scheme:
when L is C₃-C₁₄ heterocycloalkyl, the C₃-C₁₄ heterocycloalkyl is for example C₃-C₁₄ saturated heterocycloalkyl.

In a certain scheme:
when L is C₃-C₁₄ heterocycloalkyl, the heteroatom of the C₃-C₁₄ heterocycloalkyl may not be substituted.

In a certain scheme:
when L is independently C₃-C₁₄ heterocycloalkyl, the methylene in the C₃-C₁₄ heterocycloalkyl may not be substituted or replaced.

In a certain scheme:
when L is C₃-C₁₄ heterocycloalkyl substituted by one or two R⁴, the C₃-C₁₄ heterocycloalkyl is for example C₃-C₁₄ monocyclic heterocycloalkyl, C₃-C₁₄ spiro heterocycloalkyl, C₃-C₁₄ fused heterocycloalkyl or C₃-C₁₄ bridged heterocycloalkyl.

The C₃-C₁₄ monocyclic heterocycloalkyl is, for example, "C₃-C₉ monocyclic heterocycloalkyl having one or two heteroatoms selected from one or two of N, O and S", for example, "C₃-C₅ monocyclic heterocycloalkyl having one or two heteroatoms selected from one or two of N, O and S", for example, "C₃-C₅ monocyclic heterocycloalkyl having one or two heteroatoms selected from one or two of N, O and S" and which is connected to a benzene ring by a nitrogen atom, for example, morpholinyl, piperidinyl or piperazinyl.

The morpholinyl is for example 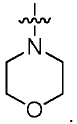 The piperidinyl is for example 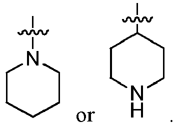 The piperazinyl is for example

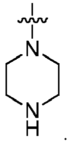

The C₃-C₁₄ spiro heterocycloalkyl is, for example, "C₅-C₉ spiro heterocycloalkyl having one or two heteroatoms selected from one or two of N, O and S", for example, "C₇-C₉ spiro heterocycloalkyl having one or two heteroatoms selected from one or two of N, O and S", for example, "C₇-C₉ spiro heterocycloalkyl having one or two heteroatoms selected from one or two of N, O and S" and which is connected to a benzene ring by a nitrogen atom, for example, 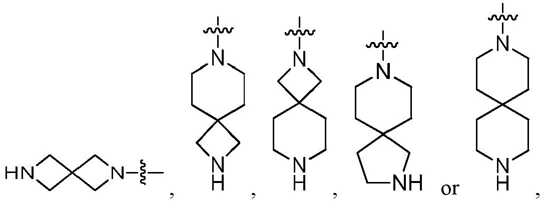 for example,

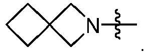

The C₃-C₁₄ fused heterocycloalkyl is, for example, "C₆-C₈ fused heterocycloalkyl having one or two heteroatoms selected from one or two of N, O and S", for example, "C₆-C₈ fused heterocycloalkyl having one or two heteroatoms selected from one or two of N, O and S" and which is connected to a benzene ring by a nitrogen atom, for example, 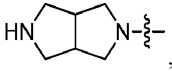 , for example,

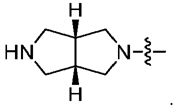

The C₃-C₁₄ bridged heterocycloalkyl is, for example, "C₄-C₆ bridged heterocycloalkyl having one or two heteroatoms selected from one or two of N, O and S", for example, "C₄-C₆ bridged heterocycloalkyl having one or two heteroatoms selected from one or two of N, O and S" and which is connected to a benzene ring by a nitrogen atom, for example,

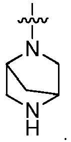

In a certain scheme:
when L is C₃-C₁₄ heterocycloalkyl substituted by one or two R⁴, the C₃-C₁₄ heterocycloalkyl is for example C₃-C₁₄ saturated heterocycloalkyl.

In a certain scheme:
when L is C₃-C₁₄ heterocycloalkyl optionally substituted by one or two R⁴, the heteroatom of the C₃-C₁₄ heterocycloalkyl may not be substituted except R⁴.

In a certain scheme:
when L is C₃-C₁₄ heterocycloalkyl optionally substituted by one or two R⁴, the methylene in the C₃-C₁₄ heterocycloalkyl may not be substituted or replaced.

In a certain scheme:
when R⁴ is C₁-C₇ alkyl optionally substituted by one R²⁻⁹, the C₁-C₇ alkyl is for example C₁-C₃ alkyl, for example methyl, ethyl, *n*-propyl or isopropyl, for example methyl or ethyl.

In a certain scheme:
when R⁴ is C₃-C₁₄ cycloalkyl, the C₃-C₁₄ cycloalkyl is for example C₃-C₇ cycloalkyl, for example C₃-C₇ monocyclic cycloalkyl, for example cyclobutyl.

In a certain scheme:
when R⁴ is C₃-C₁₄ cycloalkyl, the C₃-C₁₄ cycloalkyl is for example C₃-C₁₄ saturated cycloalkyl.

In a certain scheme:
when R⁴ is C₃-C₁₄ heterocycloalkyl optionally substituted by one R²⁻⁹, the C₃-C₁₄ heterocycloalkyl is for example C₃-C₇ heterocycloalkyl, for example C₃-C₇ monocyclic heterocycloalkyl, for example azetidinyl, and for example

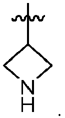

In a certain scheme:
when R⁴ is C₃-C₁₄ heterocycloalkyl optionally substituted by one R²⁻⁹, the C₃-C₁₄ heterocycloalkyl is for example C₃-C₁₄ saturated heterocycloalkyl.

In a certain scheme:
when R⁴ is C₃-C₁₄ heterocycloalkyl optionally substituted by one R²⁻⁹, the heteroatom of the C₃-C₁₄ heterocycloalkyl may not be substituted except R²⁻⁹.

In a certain scheme:
when R⁴ is C₃-C₁₄ heterocycloalkyl optionally substituted by one R²⁻⁹, the methylene in the C₃-C₁₄ heterocycloalkyl may not be substituted or replaced.

In a certain scheme:
when R²⁻⁹ is independently C₃-C₁₄ cycloalkyl, the C₃-C₁₄ cycloalkyl is for example C₃-C₁₄ monocyclic cycloalkyl, for example C₃-C₆ monocyclic cycloalkyl, for example cyclopropyl.

In a certain scheme:
when R²⁻⁹ is C₃-C₁₄ cycloalkyl, the C₃-C₁₄ cycloalkyl is for example C₃-C₁₄ saturated cycloalkyl.

In a certain scheme:
when R²⁻⁹⁻² and R²⁻⁹⁻³ are independently C₁-C₇ alkyl, the C₁-C₇ alkyl is for example C₁-C₃ alkyl, for example methyl, ethyl, *n*-propyl or isopropyl, for example methyl.

In a certain scheme:
when L is C₃-C₁₄ heterocycloalkyl substituted by one R⁴, R⁴ is C₁-C₇ alkyl optionally substituted by one R²⁻⁹, the "C₃-C₁₄ heterocycloalkyl substituted by one R⁴" is for example 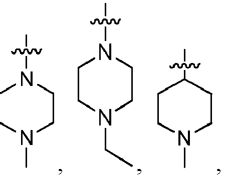 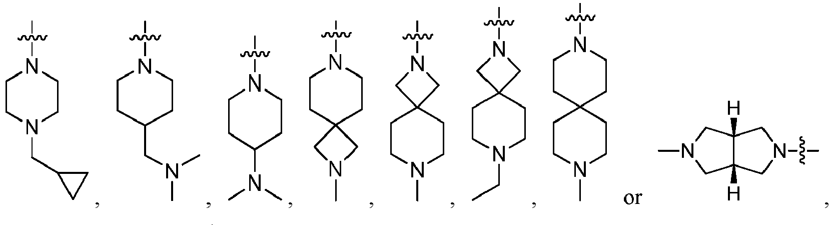 for example

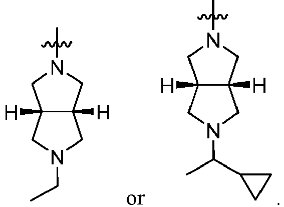

In a certain scheme:
when L is C₃-C₁₄ heterocycloalkyl substituted by one R⁴, R⁴ is C₃-C₁₄ cycloalkyl optionally substituted by one R²⁻⁹, the "C₃-C₁₄ heterocycloalkyl substituted by one R⁴" is

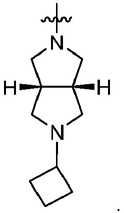

In a certain scheme:
when L is C₃-C₁₄ heterocycloalkyl substituted by one R⁴, R⁴ is C₃-C₁₄ heterocycloalkyl optionally substituted by one R²⁻⁹, the "C₃-C₁₄ heterocycloalkyl substituted by one R⁴" is 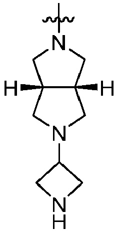 or

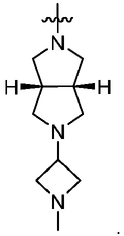

In a certain scheme:
when L is C₃-C₁₄ heterocycloalkyl substituted by one R⁴, R⁴ is -NR²⁻¹R²⁻²; the "C₃-C₁₄ heterocycloalkyl substituted by one R⁴" is

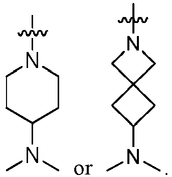

In a certain scheme:
when R²⁻³ is independently C₃-C₁₄ heterocycloalkyl, the C₃-C₁₄ heterocycloalkyl is for example C₃-C₁₄ monocyclic heterocycloalkyl.

The C₃-C₁₄ monocyclic heterocycloalkyl is, for example, "C₃-C₉ monocyclic heterocycloalkyl having one or two heteroatoms selected from one or two of N, O and S", for example, "C₃-C₅ monocyclic heterocycloalkyl having one or two heteroatoms selected from one or two of N, O and S", for example, "C₃-C₅ monocyclic heterocycloalkyl having one or two heteroatoms selected from one or two of N, O and S" and which is connected to a benzene ring by a nitrogen atom, for example, tetrahydropyrrolyl.

The tetrahydropyrrolyl is for example

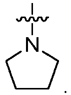

In a certain scheme:
when R²⁻³ is independently C₃-C₁₄ heterocycloalkyl, the C₃-C₁₄ heterocycloalkyl is for example C₃-C₁₄ saturated heterocycloalkyl.

In a certain scheme:
when R²⁻³ is independently C₃-C₁₄ heterocycloalkyl, the heteroatom of the C₃-C₁₄ heterocycloalkyl may not be substituted.

In a certain scheme:
when R²⁻³ is independently C₃-C₁₄ heterocycloalkyl, the methylene in the C₃-C₁₄ heterocycloalkyl may not be substituted or replaced.

In a certain scheme:
when R² is C₃-C₁₄ heterocycloalkyl optionally substituted by one R²⁻⁹, the C₃-C₁₄ heterocycloalkyl, for example, "C₃-C₉ monocyclic heterocycloalkyl having one or two heteroatoms selected from one or two of N, O and S", for example, "C₃-C₅ monocyclic heterocycloalkyl having one or two heteroatoms selected from one or two of N, O and S", for example, "C₃-C₅ monocyclic heterocycloalkyl having one or two heteroatoms selected from one or two of N, O and S" and which is connected to a benzene ring by a nitrogen atom, for example, piperazinyl.

The piperazinyl is for example

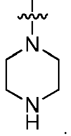

In a certain scheme:
when R² is C₃-C₁₄ heterocycloalkyl substituted by one R²⁻⁹, the C₃-C₁₄ heterocycloalkyl is for example C₃-C₁₄ saturated heterocycloalkyl.

In a certain scheme:
when R² is C₃-C₁₄ heterocycloalkyl optionally substituted by one R²⁻⁹, the heteroatom of the C₃-C₁₄ heterocycloalkyl may not be substituted except R²⁻⁹.

In a certain scheme:
when R² is C₃-C₁₄ heterocycloalkyl optionally substituted by one R²⁻⁹, the methylene in the C₃-C₁₄ heterocycloalkyl may not be substituted or replaced.

In a certain scheme:
when R²⁻⁹ is independently C₁-C₇ alkyl, the C₁-C₇ alkyl is for example C₁-C₃ alkyl, for example methyl, ethyl, *n*-propyl or isopropyl.

In a certain scheme:
the ratio of each isomer in the pyrazolone-fused pyrimidine compound represented by formula I may be equal, for example, racemate.

In a certain scheme:
the atoms in the pyrazolone-fused pyrimidine compound represented by formula I, the pharmaceutically acceptable salt thereof, the solvate thereof or the solvate of the pharmaceutically acceptable salt thereof may all exist in their natural abundance.

In a certain scheme:
R¹ is hydrogen or methyl.

In a certain scheme:
n is 1 or 2.

In a certain scheme:
L is -(CH₂)ₘ-R², "C₃-C₂₀ cycloalkyl substituted by one R³", C₃-C₁₄ heterocycloalkyl or "C₃-C₁₄ heterocycloalkyl substituted by one R⁴"; m is 1 or 2, and can also be 3.

In a certain scheme:
L is "C₃-C₂₀ cycloalkyl substituted by one R³", C₃-C₁₄ heterocycloalkyl or "C₃-C₁₄ heterocycloalkyl substituted by one R⁴".

In a certain scheme:
L is -(CH₂)ₘ-R², "C₃-C₁₄ fused heterocycloalkyl substituted by one or two R⁴" or "C₃-C₁₄ spiro heterocycloalkyl substituted by one or two R^{4"};
when L is C₃-C₁₄ fused heterocycloalkyl, the C₃-C₁₄ fused heterocycloalkyl is C₅-C₁₄ fused heterocycloalkyl;
when L is C₃-C₁₄ spiro heterocycloalkyl, the C₃-C₁₄ spiro heterocycloalkyl is C₅-C₁₄ spiro heterocycloalkyl.

In a certain scheme:
R² is -NR²⁻¹R²⁻².

In a certain scheme:
R⁴ is -NR²⁻¹R²⁻² or "C₁-C₇ alkyl, C₃-C₁₄ cycloalkyl or C₃-C₁₄ heterocycloalkyl optionally substituted by one, two or three R^{2-9"}.

In a certain scheme:
R²⁻¹ and R²⁻² are independently C₁-C₇ alkyl.

In a certain scheme:
R²⁻⁹ is independently C₁-C₇ alkyl or C₃-C₁₄ heterocycloalkyl.

In a certain scheme:
L is

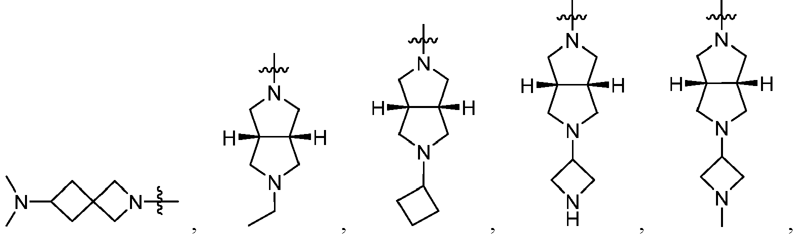

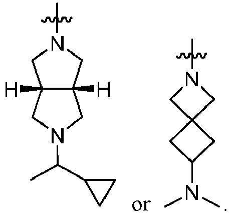

In a certain scheme:
R², R³ and R⁴ are independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻¹, C₁-C₇ alkyl or C₃-C₁₄ cycloalkyl;
R²⁻⁹⁻² and R²⁻⁹⁻³ are independently C₁-C₇ alkyl.

In a certain scheme:
R³ and R⁴ are independently -NR²⁻¹R²⁻² or "C₁-C₇ alkyl optionally substituted by one R^{2-9"};
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl.

In a certain scheme:
R², R³ and R⁴ are independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻¹¹ is independently -NR²⁻⁹⁻⁸⁻¹R²⁻⁹⁻⁸⁻²;
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl.

In a certain scheme:
L is -(CH₂)ₘ-R², "C₃-C₂₀ cycloalkyl substituted by one R^{3"}, C₃-C₁₄ heterocycloalkyl or "C₃-C₁₄ heterocycloalkyl substituted by one R^{4"}; m is 1 or 2, and can also be 3;
R², R³ and R⁴ are independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl.

In a certain scheme:
L is "C₃-C₂₀ cycloalkyl substituted by one R³", C₃-C₁₄ heterocycloalkyl or "C₃-C₁₄ heterocycloalkyl substituted by one R⁴";
R³ and R⁴ are independently -NR²⁻¹R²⁻² or "C₁-C₇ alkyl optionally substituted by one R^{2-9"};
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl.

In a certain scheme: R², R³ and R⁴ are independently -NR²⁻¹R²⁻², R²⁻¹ and R²⁻² are independently hydrogen, C₁-C₇ alkyl or C₃-C₁₄ cycloalkyl.

In a certain scheme: R², R³ and R⁴ are independently -NR²⁻¹R²⁻², R²⁻¹ and R²⁻² are independently C₁-C₇ alkyl.

In a certain scheme: L is C₃-C₂₀ cycloalkyl or "C₃-C₂₀ cycloalkyl substituted by one or two R³".

In a certain scheme: L is C₃-C₂₀ cycloalkyl substituted by one or two R³; R³ is independently -NR²⁻¹R²⁻² or "C₁-C₇ alkyl optionally substituted by one R^{2-9"}; R²⁻¹ and R²⁻² are independently C₁-C₇ alkyl; R²⁻⁹ is independently -NR²⁻⁹⁻²R²⁻⁹⁻³ or C₃-C₁₄ cycloalkyl; R²⁻⁹⁻² and R²⁻⁹⁻³ are independently C₁-C₇ alkyl".

In a certain scheme, L is C₃-C₁₄ heterocycloalkyl or "C₃-C₁₄ heterocycloalkyl substituted by one or two R⁴"; the C₃-C₁₄ heterocycloalkyl is C₃-C₁₄ spiro heterocycloalkyl.

In a certain scheme, L is C₃-C₁₄ heterocycloalkyl or "C₃-C₁₄ heterocycloalkyl substituted by one R⁴"; the C₃-C₁₄ heterocycloalkyl is C₃-C₁₄ spiro heterocycloalkyl; R⁴ is independently -NR²⁻¹R²⁻², or "C₁-C₇ alkyl optionally substituted by one R^{2-9"}; R²⁻¹ and R²⁻² are independently C₁-C₇ alkyl; R²⁻⁹ is independently -NR²⁻⁹⁻²R²⁻⁹⁻³ or C₃-C₁₄ cycloalkyl; R²⁻⁹⁻² and R²⁻⁹⁻³ are independently C₁-C₇ alkyl.

In a certain scheme, L is C₃-C₁₄ heterocycloalkyl or "C₃-C₁₄ heterocycloalkyl substituted by one or two R^{4"}; the C₃-C₁₄ heterocycloalkyl is C₃-C₁₄ fused heterocycloalkyl.

In a certain scheme, L is C₃-C₁₄ heterocycloalkyl or "C₃-C₁₄ heterocycloalkyl substituted by one R⁴"; the C₃-C₁₄ heterocycloalkyl is C₃-C₁₄ fused heterocycloalkyl; R⁴ is independently -NR²⁻¹R²⁻² or "C₁-C₇ alkyl optionally substituted by one R^{2-9"}; R²⁻¹ and R²⁻² are independently C₁-C₇ alkyl; R²⁻⁹ is independently -NR²⁻⁹⁻²R²⁻⁹⁻³ or C₃-C₁₄ cycloalkyl; R²⁻⁹⁻² and R²⁻⁹⁻³ are independently C₁-C₇ alkyl.

In a certain scheme, L is C₃-C₁₄ heterocycloalkyl or "C₃-C₁₄ heterocycloalkyl substituted by one or two R⁴"; the C₃-C₁₄ heterocycloalkyl is C₃-C₁₄ bridged heterocycloalkyl.

In a certain scheme, L is C₃-C₁₄ heterocycloalkyl, or "C₃-C₁₄ heterocycloalkyl substituted by one R⁴"; the C₃-C₁₄ heterocycloalkyl is C₃-C₁₄ bridged heterocycloalkyl; R⁴ is independently -NR²⁻¹R²⁻², or "C₁-C₇ alkyl optionally substituted by one R^{2-9"}; R²⁻¹ and R²⁻² are independently C₁-C₇ alkyl; R²⁻⁹ is independently -NR²⁻⁹⁻²R²⁻⁹⁻³ or C₃-C₁₄ cycloalkyl; R²⁻⁹⁻² and R²⁻⁹⁻³ are independently C₁-C₇ alkyl.

In a certain scheme, L is (CH₂)ₘ-R².

In a certain scheme:
R¹ is hydrogen or methyl;
n is 1 or 2;
L is -(CH₂)ₘ-R², C₃-C₂₀ cycloalkyl, "C₃-C₂₀ cycloalkyl substituted by one or two R³", C₃-C₁₄ heterocycloalkyl or "C₃-C₁₄ heterocycloalkyl substituted by one or two R⁴";
m is 1, 2, 3 or 4;
R², R³ and R⁴ are independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻²
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl.

In a certain scheme:
R¹ is hydrogen or methyl;
n is 1 or 2;
L is -(CH₂)ₘ-R², "C₃-C₂₀ cycloalkyl substituted by one R³", C₃-C₁₄ heterocycloalkyl or "C₃-C₁₄ heterocycloalkyl substituted by one R⁴"; m is 1 or 2, and can also be 3;
R², R³ and R⁴ are independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl.

In a certain scheme:
R¹ is hydrogen or methyl;
n is 1 or 2;
L is "C₃-C₂₀ cycloalkyl substituted by one R³", C₃-C₁₄ heterocycloalkyl or "C₃-C₁₄ heterocycloalkyl substituted by one R⁴;
R³ and R⁴ are independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl.

In a certain scheme:
R¹ is hydrogen or methyl;
n is 1 or 2;
L is -(CH₂)ₘ-R², "C₃-C₂₀ cycloalkyl substituted by one or two R³" or "C₃-C₁₄ heterocycloalkyl substituted by one or two R⁴";
m is 1, 2, 3 or 4;
R², R³ and R⁴ are independently -NR²⁻¹R²⁻²;
R²⁻¹ and R²⁻² are independently hydrogen, C₁-C₇ alkyl, or C₃-C₁₄ cycloalkyl.

In a certain scheme:
R¹ is hydrogen or methyl;
n is 1 or 2;
L is -(CH₂)ₘ-R², "C₃-C₂₀ cycloalkyl substituted by one R³", "C₃-C₁₄ heterocycloalkyl substituted by one R⁴"; m is 1 or 2, and can also be 3;
R², R³ and R⁴ are independently -NR²⁻¹R²⁻²;
R²⁻¹ and R²⁻² are independently C₁-C₇ alkyl.

In a certain scheme:
R¹ is hydrogen or methyl;
n is 1 or 2;
L is "C₃-C₂₀ cycloalkyl substituted by one R³" or "C₃-C₁₄ heterocycloalkyl substituted by one R⁴;
R³ and R⁴ are independently -NR²⁻¹R²⁻²;
R²⁻¹ and R²⁻² are independently C₁-C₇ alkyl.

In a certain scheme:
R¹ is hydrogen or methyl;
n is 1 or 2;
L is C₃-C₂₀ cycloalkyl or "C₃-C₂₀ cycloalkyl substituted by one or two R³";
R³ is independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻²
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl.

In a certain scheme:
R¹ is hydrogen or methyl;
n is 1 or 2;
L is C₃-C₂₀ cycloalkyl substituted by one R³;
R³ is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl.

In a certain scheme:
R¹ is hydrogen or methyl;
n is 1 or 2;
L is C₃-C₂₀ cycloalkyl substituted by one R³;
R³ is independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl.

In a certain scheme:
R¹ is hydrogen or methyl;
n is 1 or 2;
L is C₃-C₁₄ heterocycloalkyl or "C₃-C₁₄ heterocycloalkyl substituted by one or two R⁴"; the C₃-C₁₄ heterocycloalkyl is C₃-C₁₄ spiro heterocycloalkyl;
R⁴ is independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻²
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl.

In a certain scheme:
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ spiro heterocycloalkyl;
R⁴ is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl.

In a certain scheme:
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ spiro heterocycloalkyl;
R⁴ is independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl.

In a certain scheme:
R¹ is hydrogen or methyl;
n is 1 or 2;
L is C₃-C₁₄ heterocycloalkyl or "C₃-C₁₄ heterocycloalkyl substituted by one or two R⁴"; the C₃-C₁₄ heterocycloalkyl is C₃-C₁₄ fused heterocycloalkyl;
R⁴ is independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻²;
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl.

In a certain scheme:
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ fused heterocycloalkyl;
R⁴ is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl.

In a certain scheme:
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ fused heterocycloalkyl;
R⁴ is independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl.

In a certain scheme:
R¹ is hydrogen or methyl;
n is 1 or 2;
L is C₃-C₁₄ heterocycloalkyl or "C₃-C₁₄ heterocycloalkyl substituted by one or two R⁴"; the C₃-C₁₄ heterocycloalkyl is C₃-C₁₄ bridged heterocycloalkyl;
R⁴ is independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻¹⁻¹ is independently -NR²⁻⁹⁻⁸⁻¹R²⁻⁹⁻⁸⁻²;
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl.

In a certain scheme:
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ bridged heterocycloalkyl;
R⁴ is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl.
In a certain scheme:
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ bridged heterocycloalkyl;
R⁴ is independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl.
In a certain scheme:
R¹ is hydrogen or methyl;
n is 1 or 2;
L is (CH₂)ₘ-R²;
m is 1, 2, 3 or 4;
R², R³ and R⁴ are independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻²
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl.

In a certain scheme:
R¹ is hydrogen or methyl;
n is 1 or 2;
L is (CH₂)ₘ-R²; m is 1 or 2 and can also be 3.
R² is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl.
In a certain scheme,
R¹ is hydrogen or methyl;
n is 1 or 2;
L is -(CH₂)ₘ-R², "C₃-C₁₄ fused heterocycloalkyl substituted by one or two R⁴" or "C₃-C₁₄ spiro heterocycloalkyl substituted by one or two R⁴";
when L is C₃-C₁₄ fused heterocycloalkyl, the C₃-C₁₄ fused heterocycloalkyl is C₅-C₁₄ fused heterocycloalkyl;
when L is C₃-C₁₄ spiro heterocycloalkyl, the C₃-C₁₄ spiro heterocycloalkyl is C₅-C₁₄ spiro heterocycloalkyl;
R² is -NR²⁻¹R²⁻²;
R⁴ is -NR²⁻¹R²⁻² or "C₁-C₇ alkyl, C₃-C₁₄ cycloalkyl or C₃-C₁₄ heterocycloalkyl" optionally substituted by one, two or three R²⁻⁹.

In a certain scheme,
R¹ is hydrogen;
n is 1;
L is -(CH₂)ₘ-R², "C₃-C₁₄ fused heterocycloalkyl substituted by one or two R⁴" or "C₃-C₁₄ spiro heterocycloalkyl substituted by one or two R⁴";
when L is C₃-C₁₄ fused heterocycloalkyl, the C₃-C₁₄ fused heterocycloalkyl is C₅-C₁₄ fused heterocycloalkyl;
when L is C₃-C₁₄ spiro heterocycloalkyl, the C₃-C₁₄ spiro heterocycloalkyl is C₅-C₁₄ spiro heterocycloalkyl;
R² is -NR²⁻¹R²⁻²;
R⁴ is -NR²⁻¹R²⁻² or "C₁-C₇ alkyl, C₃-C₁₄ cycloalkyl or C₃-C₁₄ heterocycloalkyl" optionally substituted by one, two or three R²⁻⁹;
R²⁻¹ and R²⁻² are independently C₁-C₇ alkyl;
R²⁻⁹ is independently C₁-C₇ alkyl or C₃-C₁₄ heterocycloalkyl.

In a certain scheme, the pyrazolone-fused pyrimidine compounds represented by formula I are not

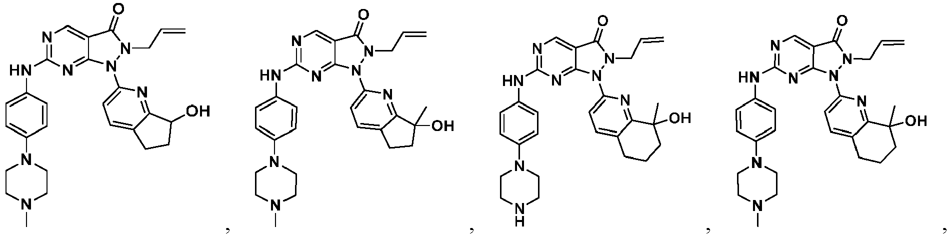

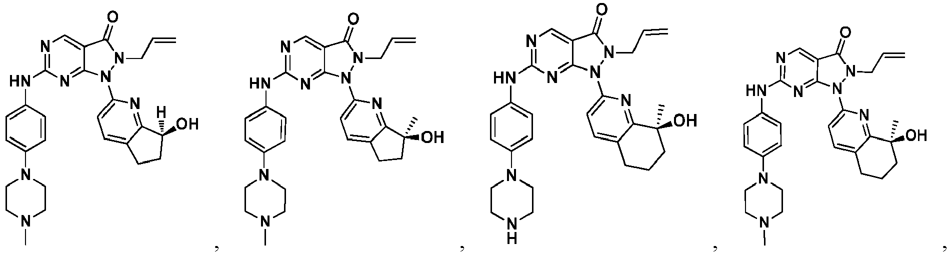

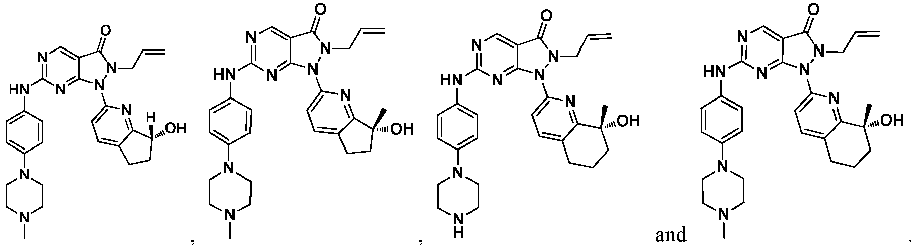

In a certain scheme, in the pyrazolone-fused pyrimidine compound represented by formula I, the pharmaceutically acceptable salt thereof, the solvate thereof or the solvate of the pharmaceutically acceptable salt thereof, the pyrazolone-fused pyrimidine compound represented by formula I is any of the following structures: 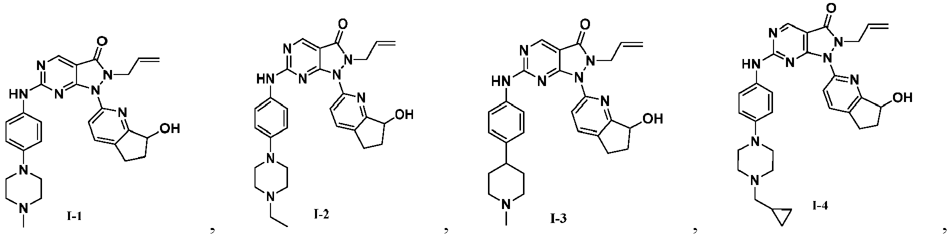 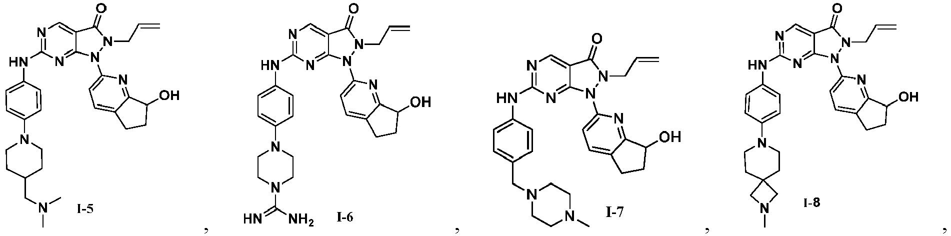 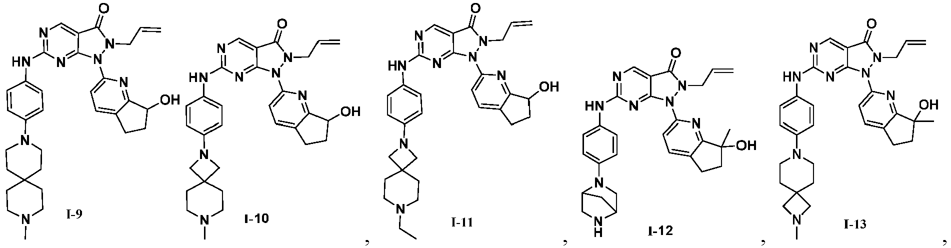 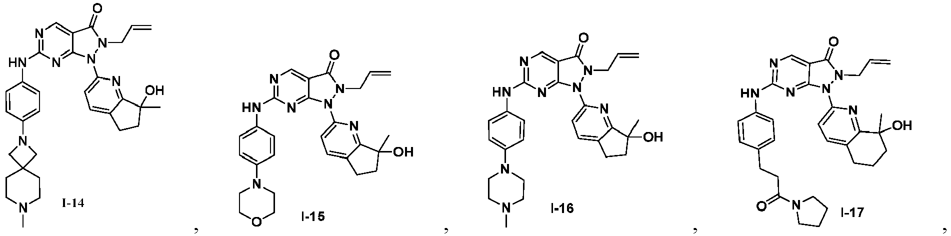 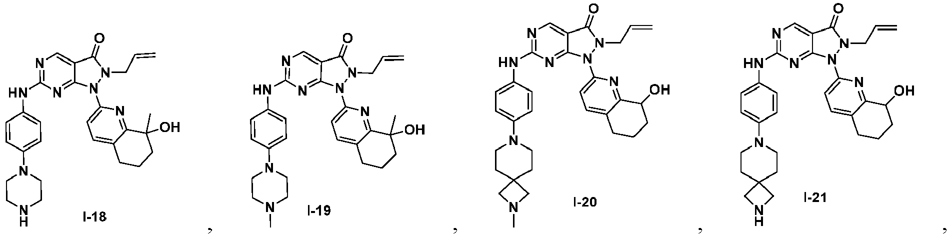 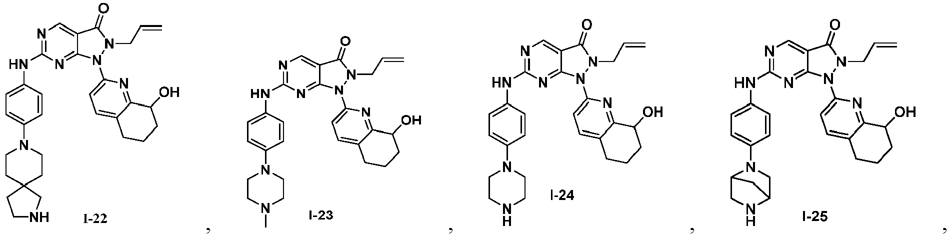 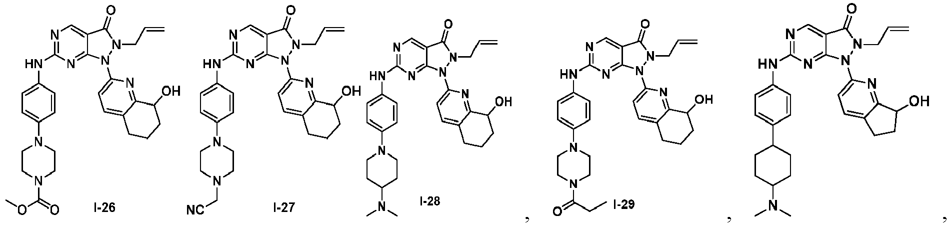 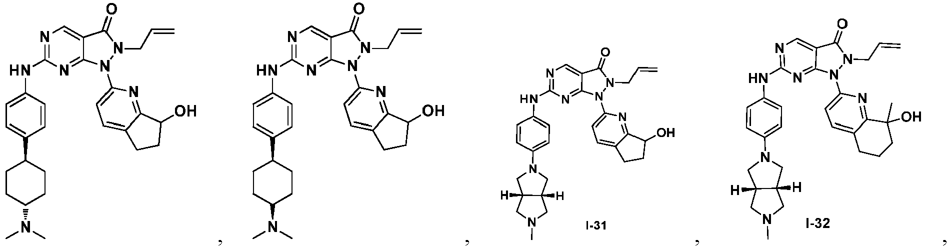 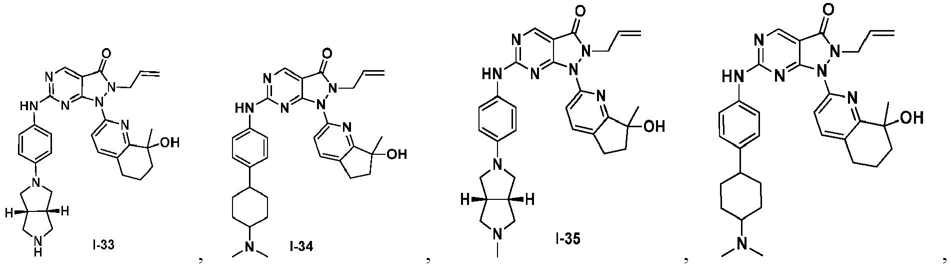 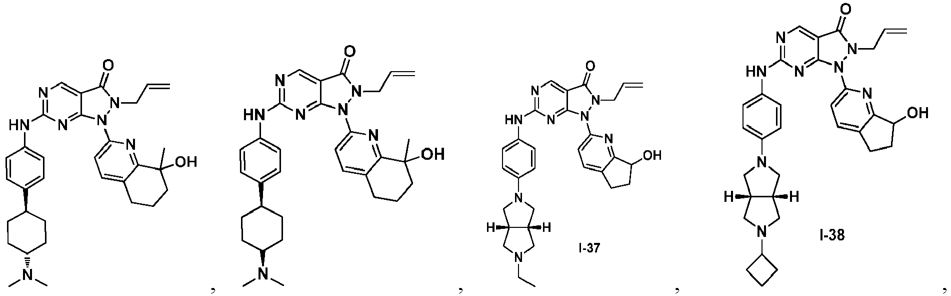 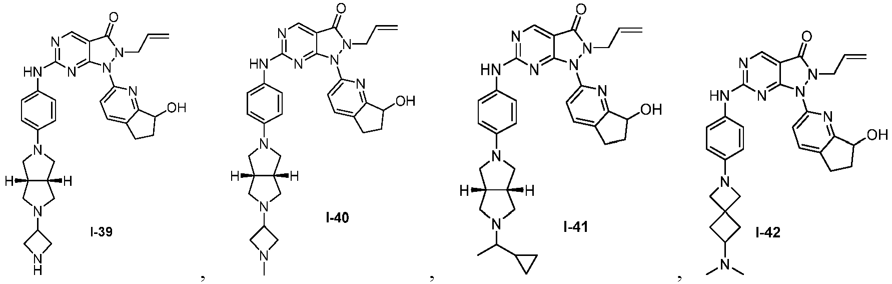 and

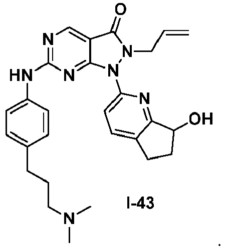

In a certain scheme, in the pyrazolone-fused pyrimidine compound represented by formula I, the pharmaceutically acceptable salt thereof, the solvate thereof or the solvate of the pharmaceutically acceptable salt thereof, the pyrazolone-fused pyrimidine compound represented by formula I is any of the following compounds: 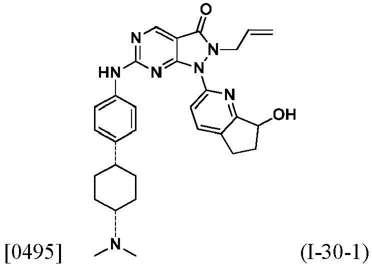 with a retention time of 9.66 min under the following conditions: Agilent 1260 high performance liquid chromatograph; mobile phase A: water (0.1 % formic acid), mobile phase B: acetonitrile; column time: 15 min; column type: Waters' Xselect, 5 µm, 4.6×250 mm; gradient elution, 5 % mobile phase B→50 % mobile phase B; 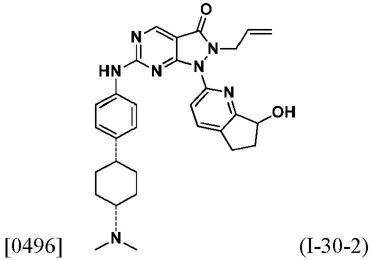 with a retention time of 10.04 min under the following conditions: Agilent 1260 high performance liquid chromatograph; mobile phase A: water (0.1 % formic acid), mobile phase B: acetonitrile; column time: 15 min; column type: Waters' Xselect, 5 µm, 4.6×250 mm; gradient elution, 5 % mobile phase B→50 % mobile phase B; 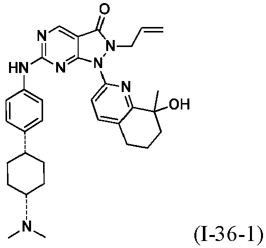 with a retention time of 7.80 min under the following conditions: Agilent 1260 high performance liquid chromatograph; mobile phase A: water (0.1 % formic acid), mobile phase B: acetonitrile; column time: 15 min; column type: Waters' Xselect, 5 µm, 4.6×250 mm; gradient elution, 5 % mobile phase B→95 % mobile phase B; 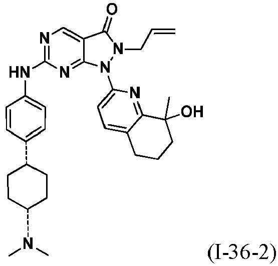 with a retention time of 7.97 min under the following conditions: Agilent 1260 high performance liquid chromatograph; mobile phase A: water (0.1 % formic acid), mobile phase B: acetonitrile; column time: 15 min; column type: Waters' Xselect, 5 µm, 4.6×250 mm; gradient elution, 5 % mobile phase B→95 % mobile phase B;
wherein, 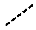 means that the *cis-trans* conformation is uncertain.

The present disclosure also provides a preparation method of the compound **I,** comprising the following steps: step I, a C-N coupling reaction is carried out between compound **1A** and compound **1B** to obtain compound **1C;** step II, an intermediate sulfoxide with higher activity is formed by compound **1C** under the action of an oxidant, and then a substitution reaction is carried out between the intermediate sulfoxide with compound **ID** to obtain compound **I;**

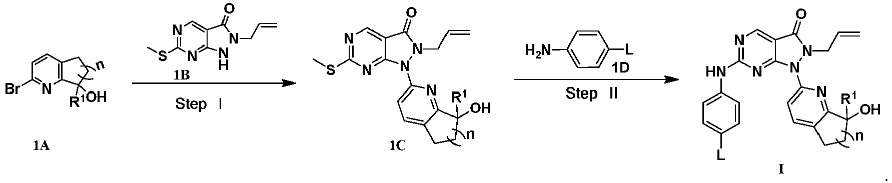

The conditions and steps of the reaction in the preparation method of compound I can be conventional conditions and steps in the art, and the following conditions and steps are particularly preferred in the present disclosure:
in step I, the C-N coupling reaction is a coupling method commonly used in the art for constructing C-N bonds, such as Ullmann reaction, Buchwald reaction, preferably Ullmann reaction, more preferably cuprous iodide/potassium carbonate/*N*, *N*-diisopropylethylamine/dioxane reaction conditions.

In step II, the oxidant is preferably *m*-chloroperoxybenzoic acid (*m*-CPBA); the conditions of the substitution reaction are the reaction conditions commonly used in the art for substitution, such as alkaline conditions or acidic conditions, wherein diisopropylethylamine is preferred for alkaline conditions and trifluoroacetic acid is preferred for acidic conditions.

In the above preparation method of compound **I,** the compound **1A** can also be obtained by the method shown in the following reaction formula 1: step I, a reaction is carried out between compound **1A1** and a brominating agent in an organic solvent to obtain compound **1A2**; step II, oxidizing pyridine of compound **1A2** with an oxidant to obtain compound **1A3** with higher reactivity; step III, a reaction is carried out between compound **1A3** and a nucleophile to obtain compound **1A4**; step IV, compound **1A4** is deacetylated under alkaline conditions to obtain compound **1A;** 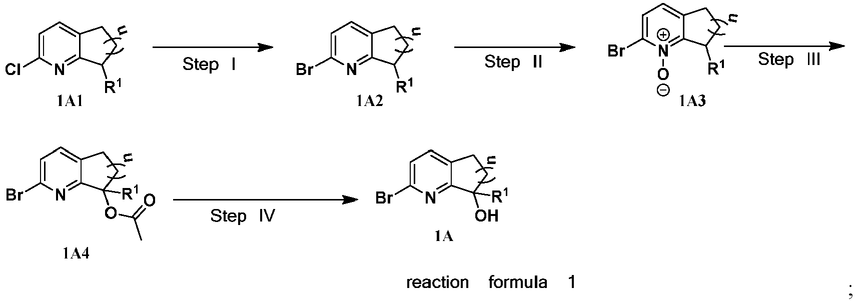 wherein R¹ is hydrogen; n is 1 or 2.

The conditions and steps of the reaction formula 1 can be conventional conditions and steps in the art, and the following conditions are particularly preferred in the present disclosure:
in step I, the organic solvent is preferably a high boiling point solvent such as toluene, acetonitrile; the brominating agent is preferably bromotrimethylsilane, phosphorus oxybromide; the reaction temperature is preferably 120 °C-150 °C, conventional heating reaction or microwave heating reaction.

In step II, the oxidant may be an oxidant commonly used in the art for the oxidation of nitrogen to nitrogen oxides, preferably *m*-chloroperoxybenzoic acid (m-CPBA).

In step III, the nucleophile is preferably anhydride, such as acetic anhydride.

In step IV, the alkaline conditions are those commonly used in the art for the removal of acetyl, preferably with potassium hydroxide in an ethanol solvent to remove acetyl.

In the above preparation method of compound **I,** the compound **1A** may further be obtained by the method shown in reaction equation 2 as follows: step I, oxidizing compound **1A'** under the action of an oxidant to form compound **1A5**; step II, compound **1A5** is attacked by a Grignard reagent to obtain compound **1A**. 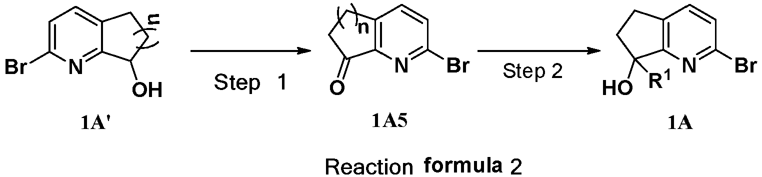 wherein, R¹ is methyl; n is 1 or 2.

The conditions and steps of the reaction formula 2 can be conventional conditions and steps in the art, and the following conditions are particularly preferred in the present disclosure:
in step I, the oxidant is a condition commonly used in the art for the oxidation of hydroxyl to form aldehydes or ketones, preferably a Dess-Martin reagent. Compound **1A'** is prepared by the method of reaction formula 1 in method 1.

In step II, the Grignard reagent is a Grignard reagent commonly used in the art, preferably methyl Grignard reagent.

The present disclosure also provides a compound represented by formula 1C: 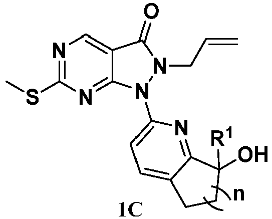 wherein, R¹ is hydrogen or methyl; n is 1 or 2.

In a certain scheme, the compound represented by formula 1C may be any of the following structures:

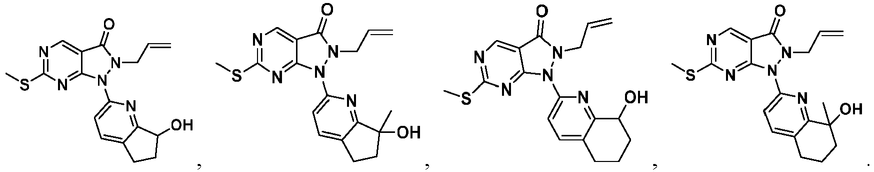

The present disclosure also provides an application of a substance X in the preparation of kinase inhibitors (such as WEE1 kinase);
the substance **X** is the pyrazolone-fused pyrimidine compound represented by formula **I,** the pharmaceutically acceptable salt thereof, the solvate thereof or the solvate of the pharmaceutically acceptable salt thereof.

In the application, the kinase inhibitor can be a kinase inhibitor used *in vitro.*

The present disclosure also provides an application of the substance **X** in the manufacture of a medicament;
the substance **X** is the pyrazolone-fused pyrimidine compound represented by formula **I,** the pharmaceutically acceptable salt thereof, the solvate thereof or the solvate of the pharmaceutically acceptable salt thereof.

The present disclosure also provides an application of the substance **X** in the manufacture of a medicament; the medicament is used for treating and/or preventing diseases related to WEE1 kinase;
the substance **X** is the pyrazolone-fused pyrimidine compound represented by formula **I,** the pharmaceutically acceptable salt thereof, the solvate thereof or the solvate of the pharmaceutically acceptable salt thereof.

The diseases related to WEE1 kinase such as cancer. The cancers are for example brain cancer, head and neck cancer, esophageal cancer, thyroid cancer, small cell cancer, non-small cell cancer, breast cancer, lung cancer, stomach cancer, gallbladder-cholangiocarcinoma, liver cancer, pancreatic cancer, colon cancer, rectal cancer, ovarian cancer, choriocarcinoma, endometrial carcinoma, cervical cancer, renal pelvis-ureteral cancer, bladder cancer, prostate cancer, penile cancer, testicular cancer, embryonal carcinoma, nephroblastoma, skin cancer, malignant melanoma, neuroblastoma, osteosarcoma, ewing tumor, soft tissue tumor, acute leukemia, chronic lymphatic leukemia, chronic myeloid leukemia, or, Hodgkin's lymphoma, for example, breast cancer, lung cancer, pancreatic cancer, colon cancer, ovarian cancer, acute leukemia, chronic lymphatic leukemia, chronic myeloid leukemia, Hodgkin's lymphoma, for example, colon cancer or ovarian cancer, for example, colon cancer or cervical cancer.

The present disclosure also provides an application of the substance **X** in the manufacture of a medicament; the medicament is used for treating and/or preventing cancer;
the substance **X** is the pyrazolone-fused pyrimidine compound represented by formula **I,** the pharmaceutically acceptable salt thereof, the solvate thereof or the solvate of the pharmaceutically acceptable salt thereof.

The cancers are for example brain cancer, head and neck cancer, esophageal cancer, thyroid cancer, small cell cancer, non-small cell cancer, breast cancer, lung cancer, stomach cancer, gallbladder-cholangiocarcinoma, liver cancer, pancreatic cancer, colon cancer, rectal cancer, ovarian cancer, choriocarcinoma, endometrial carcinoma, cervical cancer, renal pelvis-ureteral cancer, bladder cancer, prostate cancer, penile cancer, testicular cancer, embryonal carcinoma, nephroblastoma, skin cancer, malignant melanoma, neuroblastoma, osteosarcoma, ewing tumor, soft tissue tumor, acute leukemia, chronic lymphatic leukemia, chronic myeloid leukemia, or, Hodgkin's lymphoma, for example, breast cancer, lung cancer, pancreatic cancer, colon cancer, ovarian cancer, acute leukemia, chronic lymphatic leukemia, chronic myeloid leukemia, Hodgkin's lymphoma, for example, colon cancer or ovarian cancer, for example, colon cancer or cervical cancer.

The present disclosure also provides a method for treating and/or preventing diseases related to WEE1 kinase comprising administering a therapeutically effective amount of the substance **X** to a patient;
the substance **X** is the pyrazolone-fused pyrimidine compound represented by formula **I,** the pharmaceutically acceptable salt thereof, the solvate thereof or the solvate of the pharmaceutically acceptable salt thereof.

The diseases related to WEE1 kinase such as cancer. The cancers are for example brain cancer, head and neck cancer, esophageal cancer, thyroid cancer, small cell cancer, non-small cell cancer, breast cancer, lung cancer, stomach cancer, gallbladder-cholangiocarcinoma, liver cancer, pancreatic cancer, colon cancer, rectal cancer, ovarian cancer, choriocarcinoma, endometrial carcinoma, cervical cancer, renal pelvis-ureteral cancer, bladder cancer, prostate cancer, penile cancer, testicular cancer, embryonal carcinoma, nephroblastoma, skin cancer, malignant melanoma, neuroblastoma, osteosarcoma, ewing tumor, soft tissue tumor, acute leukemia, chronic lymphatic leukemia, chronic myeloid leukemia, or, Hodgkin's lymphoma, for example, breast cancer, lung cancer, pancreatic cancer, colon cancer, ovarian cancer, acute leukemia, chronic lymphatic leukemia, chronic myeloid leukemia, Hodgkin's lymphoma, for example, colon cancer or ovarian cancer, for example, colon cancer or cervical cancer.

The present disclosure also provides a method for treating and/or preventing cancer comprising administering a therapeutically effective amount of the substance **X** to a patient;
the substance **X** is the pyrazolone-fused pyrimidine compound represented by formula **I,** the pharmaceutically acceptable salt thereof, the solvate thereof or the solvate of the pharmaceutically acceptable salt thereof.

The cancers are for example brain cancer, head and neck cancer, esophageal cancer, thyroid cancer, small cell cancer, non-small cell cancer, breast cancer, lung cancer, stomach cancer, gallbladder-cholangiocarcinoma, liver cancer, pancreatic cancer, colon cancer, rectal cancer, ovarian cancer, choriocarcinoma, endometrial carcinoma, cervical cancer, renal pelvis-ureteral cancer, bladder cancer, prostate cancer, penile cancer, testicular cancer, embryonal carcinoma, nephroblastoma, skin cancer, malignant melanoma, neuroblastoma, osteosarcoma, ewing tumor, soft tissue tumor, acute leukemia, chronic lymphatic leukemia, chronic myeloid leukemia, or, Hodgkin's lymphoma, for example, breast cancer, lung cancer, pancreatic cancer, colon cancer, ovarian cancer, acute leukemia, chronic lymphatic leukemia, chronic myeloid leukemia, Hodgkin's lymphoma, for example, colon cancer or ovarian cancer, for example, colon cancer or cervical cancer.

The present disclosure also provides a pharmaceutical composition comprising substance **X** and (one or more) pharmaceutical excipients;
the substance **X** is the pyrazolone-fused pyrimidine compound represented by formula **I,** the pharmaceutically acceptable salt thereof, the solvate thereof or the solvate of the pharmaceutically acceptable salt thereof.

The present disclosure also provides a combination comprising substance **X** and an anticancer drug,
the substance **X** is the pyrazolone-fused pyrimidine compound represented by formula **I,** the pharmaceutically acceptable salt thereof, the solvate thereof or the solvate of the pharmaceutically acceptable salt thereof.

The anticancer drugs may be a conventional anticancer drug in the art (but not substance **X** as described above), such as one or more of anticancer alkylating agents, anticancer metabolic antagonists, anticancer antibiotics, anticancer drugs derived from plant, anticancer platinum ligand compounds, anticancer camptothecin derivatives, anticancer tyrosine kinase inhibitors, monoclonal antibodies, interferons, biological response modifiers, mitoxantrone, L-asparaginase, procarbazine, dacarbazine, hydroxyurea, pentostatin, retinoic acid, alefacept, darbepoetin alfa, anastrozole, exemestane, bicalutamide, leuprolide, flutamide, fulvestrant, pegaptanib sodium, denileukin diftitox 2, aldesleukin, thyrotropinα, arsenic trioxide, bortezomib, capecitabine and goserelin, for example, anticancer metabolic antagonists.

The anticancer alkylating agent may be a conventional anticancer alkylating agent in the art, such as one or more of mechlorethanmine *N*-oxide, cyclophosphamide, ifosfamide, melphalan, busulfan, dibromomannitol, carboquone, thiotepa, ranimustine, nimustine, temozolomide and carmustine.

The anticancer metabolic antagonist may be a conventional anticancer metabolic antagonist in the art, such as one or more of methotrexate, 6-mercaptopurine nucleoside, mercaptopurine, 5-fluorouracil, tegafur, doxifluridine, carmofur, cytarabine, cytarabine octadecyl sodium phosphate, enocitabine, S-1, gemcitabine, fludarabine and pemetrexed disodium, such as 5-fluorouracil.

The anticancer antibiotic may be a conventional anticancer antibiotic in the art, such as one or more of actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus, and valrubicin.

The anticancer drug derived from plants may be a conventional anticancer drug derived from plants in the art, such as one or more of vincristine, vinblastine, vindesine, etoposide, sobuzoxane, docetaxel, paclitaxel and vinorelbine.

The anticancer platinum coordination compound may be a conventional anticancer platinum coordination compound in the art, such as one or more of cisplatin, carboplatin, nedaplatin and oxaliplatin.

The anticancer camptothecin derivative may be a conventional anticancer camptothecin derivative in the art, such as one or more of irinotecan, topotecan and camptothecin.

The anticancer tyrosine kinase inhibitor may be a conventional anticancer tyrosine kinase inhibitor in the art, such as one or more of gefitinib, imatinib and erlotinib.

The monoclonal antibody may be a conventional monoclonal antibody in the art, such as one or more of cetuximab, bevacizumab, rituximab, alemtuzumab and trastuzumab.

The interferon may be a conventional interferon in the art, such as one or more of interferon α, interferon α-2a, interferon α-2b, interferon β, interferon γ-1a and interferon γ-n1.

The biological response regulator may be a conventional biological response regulator in the art, such as one or more of coriolus versicolor polysaccharide, lentinan, sizofiran, sapylin and ubenimex.

The components in the combination can be used simultaneously or separately (for example, sequentially); when the components in the combination are used simultaneously, the components in the combination can be uniformly mixed (e.g., the mixture of the components).

The components of the combination may be prepared as a single pharmaceutical composition for simultaneous use, or the components may be individually prepared as a single independent pharmaceutical composition (e.g., in kit form), which may be used simultaneously or separately (e.g., sequentially).

The present disclosure also provides an application of the above combination in the preparation of a medicament for preventing and/or treating cancer.

The cancers are for example brain cancer, head and neck cancer, esophageal cancer, thyroid cancer, small cell cancer, non-small cell cancer, breast cancer, lung cancer, stomach cancer, gallbladder-cholangiocarcinoma, liver cancer, pancreatic cancer, colon cancer, rectal cancer, ovarian cancer, choriocarcinoma, endometrial carcinoma, cervical cancer, renal pelvis-ureteral cancer, bladder cancer, prostate cancer, penile cancer, testicular cancer, embryonal carcinoma, nephroblastoma, skin cancer, malignant melanoma, neuroblastoma, osteosarcoma, ewing tumor, soft tissue tumor, acute leukemia, chronic lymphatic leukemia, chronic myeloid leukemia, or, Hodgkin's lymphoma, for example, breast cancer, lung cancer, pancreatic cancer, colon cancer, ovarian cancer, acute leukemia, chronic lymphatic leukemia, chronic myeloid leukemia, Hodgkin's lymphoma, for example, colon cancer or ovarian cancer, for example, colon cancer or cervical cancer.

In the application of the present disclosure, the above substance X and the above anticancer drugs can be administered simultaneously or separately (for example, sequentially).

The present disclosure also provides a method for treating and/or preventing cancer comprising administering a therapeutically effective amount of the above combination to a patient.

The cancers are for example brain cancer, head and neck cancer, esophageal cancer, thyroid cancer, small cell cancer, non-small cell cancer, breast cancer, lung cancer, stomach cancer, gallbladder-cholangiocarcinoma, liver cancer, pancreatic cancer, colon cancer, rectal cancer, ovarian cancer, choriocarcinoma, endometrial carcinoma, cervical cancer, renal pelvis-ureteral cancer, bladder cancer, prostate cancer, penile cancer, testicular cancer, embryonal carcinoma, nephroblastoma, skin cancer, malignant melanoma, neuroblastoma, osteosarcoma, ewing tumor, soft tissue tumor, acute leukemia, chronic lymphatic leukemia, chronic myeloid leukemia, or, Hodgkin's lymphoma, for example, breast cancer, lung cancer, pancreatic cancer, colon cancer, ovarian cancer, acute leukemia, chronic lymphatic leukemia, chronic myeloid leukemia, Hodgkin's lymphoma, for example, colon cancer or ovarian cancer, for example, colon cancer or cervical cancer.

The present disclosure also provides an application of the above substance X in the preparation of a medicament, the medicament in combination with "the above anticancer drug" used for preventing "and/or treating cancer.

The cancers are for example brain cancer, head and neck cancer, esophageal cancer, thyroid cancer, small cell cancer, non-small cell cancer, breast cancer, lung cancer, stomach cancer, gallbladder-cholangiocarcinoma, liver cancer, pancreatic cancer, colon cancer, rectal cancer, ovarian cancer, choriocarcinoma, endometrial carcinoma, cervical cancer, renal pelvis-ureteral cancer, bladder cancer, prostate cancer, penile cancer, testicular cancer, embryonal carcinoma, nephroblastoma, skin cancer, malignant melanoma, neuroblastoma, osteosarcoma, ewing tumor, soft tissue tumor, acute leukemia, chronic lymphatic leukemia, chronic myeloid leukemia, or, Hodgkin's lymphoma, for example, breast cancer, lung cancer, pancreatic cancer, colon cancer, ovarian cancer, acute leukemia, chronic lymphatic leukemia, chronic myeloid leukemia, Hodgkin's lymphoma, for example, colon cancer or ovarian cancer, for example, colon cancer or cervical cancer.

The anticancer drugs may be a conventional anticancer drug in the art (but not substance X as described above), such as one or more of anticancer alkylating agents, anticancer metabolic antagonists, anticancer antibiotics, anticancer drugs derived from plant, anticancer platinum ligand compounds, anticancer camptothecin derivatives, anticancer tyrosine kinase inhibitors, monoclonal antibodies, interferons, biological response modifiers, mitoxantrone, L-asparaginase, procarbazine, dacarbazine, hydroxyurea, pentostatin, retinoic acid, alefacept, darbepoetin alfa, anastrozole, exemestane, bicalutamide, leuprolide, flutamide, fulvestrant, pegaptanib sodium, denileukin diftitox 2, aldesleukin, thyrotropinα, arsenic trioxide, bortezomib, capecitabine and goserelin, for example, anticancer metabolic antagonists.

The anticancer alkylating agent may be a conventional anticancer alkylating agent in the art, such as one or more of mechlorethanmine *N*-oxide, cyclophosphamide, ifosfamide, melphalan, busulfan, dibromomannitol, carboquone, thiotepa, ranimustine, nimustine, temozolomide and carmustine.

The anticancer metabolic antagonist may be a conventional anticancer metabolic antagonist in the art, such as one or more of methotrexate, 6-mercaptopurine nucleoside, mercaptopurine, 5-fluorouracil, tegafur, doxifluridine, carmofur, cytarabine, cytarabine octadecyl sodium phosphate, enocitabine, S-1, gemcitabine, fludarabine and pemetrexed disodium, such as 5-fluorouracil.

The anticancer antibiotic may be a conventional anticancer antibiotic in the art, such as one or more of actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus, and valrubicin.

The anticancer drug derived from plants may be a conventional anticancer drug derived from plants in the art, such as one or more of vincristine, vinblastine, vindesine, etoposide, sobuzoxane, docetaxel, paclitaxel and vinorelbine.

The anticancer platinum coordination compound may be a conventional anticancer platinum coordination compound in the art, such as one or more of cisplatin, carboplatin, nedaplatin and oxaliplatin.

The anticancer camptothecin derivative may be a conventional anticancer camptothecin derivative in the art, such as one or more of irinotecan, topotecan and camptothecin.

The anticancer tyrosine kinase inhibitor may be a conventional anticancer tyrosine kinase inhibitor in the art, such as one or more of gefitinib, imatinib and erlotinib.

The monoclonal antibody may be a conventional monoclonal antibody in the art, such as one or more of cetuximab, bevacizumab, rituximab, alemtuzumab and trastuzumab.

The interferon may be a conventional interferon in the art, such as one or more of interferon α, interferon α-2a, interferon α-2b, interferon β, interferon γ-1a and interferon γ-n1.

The biological response regulator may be a conventional biological response regulator in the art, such as one or more of coriolus versicolor polysaccharide, lentinan, sizofiran, sapylin and ubenimex.

In the application of the present disclosure, the above substance X and the above anticancer drugs can be administered simultaneously or separately (for example, sequentially).

The present disclosure also provides an application of the above anticancer drug in the preparation of a medicament, the medicament "in combination with the substance X" used for preventing \and/or treating cancer.

The cancers are for example brain cancer, head and neck cancer, esophageal cancer, thyroid cancer, small cell cancer, non-small cell cancer, breast cancer, lung cancer, stomach cancer, gallbladder-cholangiocarcinoma, liver cancer, pancreatic cancer, colon cancer, rectal cancer, ovarian cancer, choriocarcinoma, endometrial carcinoma, cervical cancer, renal pelvis-ureteral cancer, bladder cancer, prostate cancer, penile cancer, testicular cancer, embryonal carcinoma, nephroblastoma, skin cancer, malignant melanoma, neuroblastoma, osteosarcoma, ewing tumor, soft tissue tumor, acute leukemia, chronic lymphatic leukemia, chronic myeloid leukemia, or, Hodgkin's lymphoma, for example, breast cancer, lung cancer, pancreatic cancer, colon cancer, ovarian cancer, acute leukemia, chronic lymphatic leukemia, chronic myeloid leukemia, Hodgkin's lymphoma, for example, colon cancer or ovarian cancer, for example, colon cancer or cervical cancer.

The anticancer drugs may be a conventional anticancer drug in the art (but not substance X as described above), such as one or more of anticancer alkylating agents, anticancer metabolic antagonists, anticancer antibiotics, anticancer drugs derived from plant, anticancer platinum ligand compounds, anticancer camptothecin derivatives, anticancer tyrosine kinase inhibitors, monoclonal antibodies, interferons, biological response modifiers, mitoxantrone, L-asparaginase, procarbazine, dacarbazine, hydroxyurea, pentostatin, retinoic acid, alefacept, darbepoetin alfa, anastrozole, exemestane, bicalutamide, leuprolide, flutamide, fulvestrant, pegaptanib sodium, denileukin diftitox 2, aldesleukin, thyrotropinα, arsenic trioxide, bortezomib, capecitabine and goserelin, for example, anticancer metabolic antagonists.

The anticancer alkylating agent may be a conventional anticancer alkylating agent in the art, such as one or more of mechlorethanmine *N*-oxide, cyclophosphamide, ifosfamide, melphalan, busulfan, dibromomannitol, carboquone, thiotepa, ranimustine, nimustine, temozolomide and carmustine.

The anticancer metabolic antagonist may be a conventional anticancer metabolic antagonist in the art, such as one or more of methotrexate, 6-mercaptopurine nucleoside, mercaptopurine, 5-fluorouracil, tegafur, doxifluridine, carmofur, cytarabine, cytarabine octadecyl sodium phosphate, enocitabine, S-1, gemcitabine, fludarabine and pemetrexed disodium, such as 5-fluorouracil.

The anticancer antibiotic may be a conventional anticancer antibiotic in the art, such as one or more of actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus, and valrubicin.

The anticancer drug derived from plants may be a conventional anticancer drug derived from plants in the art, such as one or more of vincristine, vinblastine, vindesine, etoposide, sobuzoxane, docetaxel, paclitaxel and vinorelbine.

The anticancer platinum coordination compound may be a conventional anticancer platinum coordination compound in the art, such as one or more of cisplatin, carboplatin, nedaplatin and oxaliplatin.

The anticancer camptothecin derivative may be a conventional anticancer camptothecin derivative in the art, such as one or more of irinotecan, topotecan and camptothecin.

The anticancer tyrosine kinase inhibitor may be a conventional anticancer tyrosine kinase inhibitor in the art, such as one or more of gefitinib, imatinib and erlotinib.

The monoclonal antibody may be a conventional monoclonal antibody in the art, such as one or more of cetuximab, bevacizumab, rituximab, alemtuzumab and trastuzumab.

The interferon may be a conventional interferon in the art, such as one or more of interferon α, interferon α-2a, interferon α-2b, interferon β, interferon γ-1a and interferon γ-n1.

The biological response regulator may be a conventional biological response regulator in the art, such as one or more of coriolus versicolor polysaccharide, lentinan, sizofiran, sapylin and ubenimex.

In the application of the present disclosure, the above substance X and the above anticancer drugs can be administered simultaneously or separately (for example, sequentially).

The present disclosure also provides a pharmaceutical composition comprising the above combination and (one or more) pharmaceutical excipients.

The pharmaceutical composition can be composed of the combination and the pharmaceutical excipients.

The present disclosure also provides a combination drug kit comprising a pharmaceutical composition A and a pharmaceutical composition B;
The pharmaceutical composition A comprises the above substance X, and (one or more) pharmaceutical excipients;
The pharmaceutical composition B comprises the anticancer drugs and one or more pharmaceutical excipients.

The combination drug kit can be composed of the pharmaceutical composition A and the pharmaceutical composition B.

The pharmaceutical composition A can be composed of the substance X, and (one or more) pharmaceutical excipients;
The pharmaceutical composition B can be composed of the anticancer drugs and one or more pharmaceutical excipients.

The anticancer drugs may be a conventional anticancer drug in the art (but not substance X as described above), such as one or more of anticancer alkylating agents, anticancer metabolic antagonists, anticancer antibiotics, anticancer drugs derived from plant, anticancer platinum ligand compounds, anticancer camptothecin derivatives, anticancer tyrosine kinase inhibitors, monoclonal antibodies, interferons, biological response modifiers, mitoxantrone, L-asparaginase, procarbazine, dacarbazine, hydroxyurea, pentostatin, retinoic acid, alefacept, darbepoetin alfa, anastrozole, exemestane, bicalutamide, leuprolide, flutamide, fulvestrant, pegaptanib sodium, denileukin diftitox 2, aldesleukin, thyrotropinα, arsenic trioxide, bortezomib, capecitabine and goserelin, for example, anticancer metabolic antagonists.

The anticancer alkylating agent may be a conventional anticancer alkylating agent in the art, such as one or more of mechlorethanmine *N*-oxide, cyclophosphamide, ifosfamide, melphalan, busulfan, dibromomannitol, carboquone, thiotepa, ranimustine, nimustine, temozolomide and carmustine.

The anticancer metabolic antagonist may be a conventional anticancer metabolic antagonist in the art, such as one or more of methotrexate, 6-mercaptopurine nucleoside, mercaptopurine, 5-fluorouracil, tegafur, doxifluridine, carmofur, cytarabine, cytarabine octadecyl sodium phosphate, enocitabine, S-1, gemcitabine, fludarabine and pemetrexed disodium, such as 5-fluorouracil.

The anticancer antibiotic may be a conventional anticancer antibiotic in the art, such as one or more of actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus, and valrubicin.

The anticancer drug derived from plants may be a conventional anticancer drug derived from plants in the art, such as one or more of vincristine, vinblastine, vindesine, etoposide, sobuzoxane, docetaxel, paclitaxel and vinorelbine.

The anticancer platinum coordination compound may be a conventional anticancer platinum coordination compound in the art, such as one or more of cisplatin, carboplatin, nedaplatin and oxaliplatin.

The anticancer camptothecin derivative may be a conventional anticancer camptothecin derivative in the art, such as one or more of irinotecan, topotecan and camptothecin.

The anticancer tyrosine kinase inhibitor may be a conventional anticancer tyrosine kinase inhibitor in the art, such as one or more of gefitinib, imatinib and erlotinib.

The monoclonal antibody may be a conventional monoclonal antibody in the art, such as one or more of cetuximab, bevacizumab, rituximab, alemtuzumab and trastuzumab.

The interferon may be a conventional interferon in the art, such as one or more of interferon α, interferon α-2a, interferon α-2b, interferon β, interferon γ-1a and interferon γ-n1.

The biological response regulator may be a conventional biological response regulator in the art, such as one or more of coriolus versicolor polysaccharide, lentinan, sizofiran, sapylin and ubenimex.

Each pharmaceutical composition in the combination drug kit can be used simultaneously or separately (for example, sequentially).

Unless otherwise specified, the following terms appearing in the present specification and claims have the following meanings:
The term "pharmaceutically acceptable" means that salts, solvents, excipients, etc. are generally nontoxic, safe and suitable for patient use. The "patient" is preferably a mammal, more preferably a human.

The term "pharmaceutically acceptable salt" refers to the salt prepared by the compound of the present disclosure and a relatively nontoxic and pharmaceutically acceptable acid or base. When the compound of the present disclosure contains a relatively acidic functional group, a base addition salt can be obtained by bringing the neutral form of the compound into contact with a sufficient amount of a pharmaceutically acceptable base in a pure solution or a suitable inert solvent. The pharmaceutically acceptable base addition salts include, but are not limited to, lithium salts, sodium salts, potassium salts, calcium salts, aluminum salts, magnesium salts, zinc salts, bismuth salts, ammonium salts, and diethanolamine salts. When the compound of the present disclosure contains a relatively basic functional group, an acid addition salt can be obtained by bringing the neutral form of the compound into contact with a sufficient amount of a pharmaceutically acceptable acid in a pure solution or a suitable inert solvent. The pharmaceutically acceptable acids include inorganic acids, the inorganic acids include but are not limited to hydrochloric acid, hydrobromic acid, hydroiodic acid, nitric acid, carbonic acid, phosphoric acid, phosphorous acid, sulfuric acid,etc. The pharmaceutically acceptable acids include organic acids, the organic acids including but not limited to: acetic acid, propionic acid, oxalic acid, isobutyric acid, maleic acid, malonic acid, benzoic acid, succinic acid, octanedioic acid, *trans*-butenedioic acid, lactic acid, mandelic acid, phthalic acid, benzenesulfonic acid, *p*-toluenesulfonic acid, citric acid, salicylic acid, tartaric acid, methanesulfonic acid, isonicotinic acid, acid citric acid, oleic acid, tannic acid, pantothenic acid, hydrogen tartrate, ascorbic acid, gentianic acid, fumaric acid, gluconic acid, saccharic acid, formic acid, ethanesulfonic acid, pamoic acid (i.e., 4, 4'-methylene-bis(3-hydroxy-2-naphthoic acid)), amino acid (e.g., glutamic acid, arginine), etc. When the compounds of the present disclosure contain relatively acidic and basic functional groups, they can be converted into base addition salts or acid addition salts. See Berge et al., "Pharmaceutical Salts", Journal of Pharmaceutical Science 66: 1-19 (1977), or, Handbook of Pharmaceutical Salts: Properties, Selection, and Use (P. Heinrich Stahl and Camille G. Wermuth, ed., Wiley-VCH, 2002).

The term "solvate" refers to a substance formed by combining a compound of the present disclosure with a stoichiometric or non-stoichiometric solvent. Solvent molecules in solvates can exist in the form of ordered or unordered arrangement. The solvents include but are not limited to: water, methanol, ethanol, etc.

The terms "pharmaceutically acceptable salts" and "solvates" in the terms "solvates of pharmaceutically acceptable salts", as described above, refer to compound of the present disclosure, 1. prepared with a relatively nontoxic, pharmaceutically acceptable acid or base, and 2. formed in combination with a stoichiometric or non-stoichiometric solvent. The "solvates of pharmaceutically acceptable salts" include but are not limited to hydrochloric acid monohydrate of the compound of the present disclosure.

The terms "compound", "pharmaceutically acceptable salt", "solvate" and "solvate of pharmaceutically acceptable salt" can exist in crystalline or amorphous form. The term "crystalline" means that the ions or molecules in it are arranged in a defined way in a three-dimensional space in a strictly periodic manner, and have a regular pattern of periodic recurrence at a certain distance apart; because of the above periodic arrangement, there can be a variety of crystalline forms, that is, the phenomenon of polycrystalline forms. The term "amorphous" refers to the disordered distribution of ions or molecules, that is, there is no periodic arrangement between ions and molecules.

The terms "compound", "pharmaceutically acceptable salt", "solvate" and "solvate of pharmaceutically acceptable salt" may exist in the form of a single stereoisomer or a mixture thereof (e.g. racemate), if stereoisomers exist. The term "stereoisomer" refers to *cis-trans* isomer or optical isomer. These stereoisomers can be separated, purified and enriched by asymmetric synthesis or chiral separation methods (including but not limited to thin layer chromatography, rotary chromatography, column chromatography, gas chromatography, high pressure liquid chromatography, etc.), and can also be obtained by chiral resolution by bonding (chemical bonding, etc.) or salting (physical bonding, etc.) with other chiral compounds. The term "single stereoisomer" means that one stereoisomer of a compound of the present disclosure is not less than 95% by mass relative to all stereoisomers of the compound.

The terms "compound", "pharmaceutically acceptable salt", "solvate" and "solvate of pharmaceutically acceptable salt" may exist in the form of single tautomer or a mixture thereof, preferably in the form in which the more stable tautomer is predominant.

The terms "compound", "pharmaceutically acceptable salt", "solvate" and "solvate of pharmaceutically acceptable salt" can exist in their natural abundance or unnatural abundance. Taking hydrogen atom as an example, its natural abundance form means that about 99.985% is protium and about 0.015% is deuterium; in the form of unnatural abundance, for example, about 95% of which is deuterium. That is, one or more atoms in the terms "compound", "pharmaceutically acceptable salt", "solvate" and "solvate of pharmaceutically acceptable salt" may be atoms that exist in unnatural abundance.

When an arbitrary variable (e.g. R¹⁻¹⁻¹) occurs many times in the definition of a compound, the definition of each position of the variable is independent of the definition of the rest, and their meanings are independent of each other and do not affect each other. Therefore, if a certain group is replaced by one, two or three R¹⁻¹⁻¹ groups, that is to say, the group may be replaced by up to three R¹⁻¹⁻¹ groups, the definition of R¹⁻¹⁻¹ at this position is independent from that of the R¹⁻¹⁻¹. In addition, the combination of substituents and/or variables is allowed only when the combination produces a stable compound.

The term "optionally substituted" means that it may or may not be substituted.

The term "halogen" refers to fluorine, chlorine, bromine or iodine.

The term "alkyl" refers to a saturated linear or branched monovalent hydrocarbon group with one to twelve carbon atoms (e.g. C₁-C₆ alkyl, e.g. C₁-C₄ alkyl). Examples of alkyl include, but are not limited to, methyl, ethyl, 1-propyl, 2-propyl, 1-butyl, 2-methyl-1-butyl, 2-butyl, 2-methyl-2-propyl, 1-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, and 1-octyl.

The term "alkenyl" refers to a linear or branched monovalent hydrocarbon group having two to twelve carbon atoms with at least one unsaturated position, that is, a carbon-carbon sp² double bond (e.g. C₂-C₆ alkenyl group, e.g. C₂-C₄ alkenyl group), and includes groups with *"cis"* and *"trans"* orientations or "E" and "Z" orientations. Examples include, but are not limited to, vinyl, allyl.

The term "alkynyl" refers to a linear or branched monovalent hydrocarbon group having two to twelve carbon atoms with at least one unsaturated position, i.e., a carbon-carbon sp triple bond (e.g. C₂-C₆ alkynyl, e.g. C₂-C₄ alkynyl). Examples include, but are not limited to, ethynyl and propynyl.

The term "cycloalkyl" refers to a saturated or partially unsaturated (containing one or two double bonds) non-aromatic cyclic hydrocarbon group (e.g. C₃-C₆ cycloalkyl) having three to twenty carbon atoms, including monocyclic cycloalkyl and polycyclic cycloalkyl. The cycloalkyl group contains 3 to 20 carbon atoms, preferably 3 to 12 carbon atoms, more preferably 3 to 6 carbon atoms.

Examples of monocyclic cycloalkyl groups include, but are not limited to, cyclopropyl, cyclobutyl, cyclopentyl, cyclohexyl, cycloheptyl, cyclooctyl, cyclononyl, cyclodecyl, cycloundecyl, cyclododecyl, 1-cyclopent-1-enyl, 1-cyclopent-2-enyl, 1-cyclopent-3-enyl, 5-hexenyl, 1-cyclohex-1-enyl, 1-cyclohex-2-enyl, and 1-cyclohex-3-enyl.

Polycyclic alkyl groups are polycyclic (e.g., bicyclic and tricyclic) cycloalkyl structures, including spirocyclic, fused cyclic and bridged cyclic cycloalkyl. Wherein, "spirocycloalkyl" refers to a polycyclic group sharing one carbon atom (called spiro atom) between the single rings of 5 to 20 membered, which may contain one or more double bonds, but none of the rings has a fully conjugated π electron system. Preferably 6 to 14 membered, more preferably 7 to 10 membered. According to the number of spirocycloalkyl shared between rings, the spirocycloalkyl is divided into monospirocycloalkyl, bisspirocycloalkyl or polyspirocycloalkyl, preferably monospirocycloalkyl and bisspirocycloalkyl. More preferably 4-membered/4-membered, 4-membered/5-membered, 4-membered/6-membered, 5-membered/5-membered or 5-membered/6-membered monospirocycloalkyl. Examples of spirocycloalkyl include, but are not limited to: 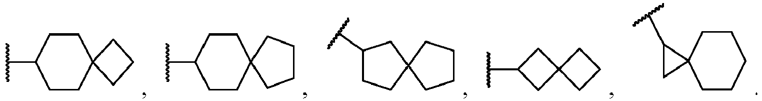 Wherein, "fused cycloalkyl" refers to all-carbon polycyclic groups of 5 to 20 membered, each ring in the system sharing an adjacent pair of carbon atoms with other rings in the system, which may contain one or more double bonds, but none of the rings has a fully conjugated π-electron system. Preferably 6 to 14 membered, more preferably 7 to 10 membered. According to the number of constituent rings, it can be divided into bicyclic, tricyclic, tetracyclic or polycyclic fused cycloalkyl, preferably bicyclic or tricyclic, more preferably 5-membered/5-membered or 5-membered/6-membered bicyclic cycloalkyl. Examples of fused cycloalkyl include, but are not limited to: 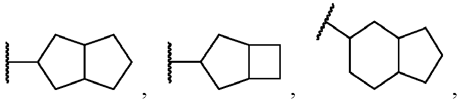 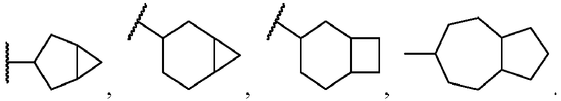 "Bridged cycloalkyl" refers to all-carbon polycyclic group of 5 to 20 members, any two rings share two non-directly connected carbon atoms, which may contain one or more double bonds, but none of which has a completely conjugated π-electron system. Preferably 6 to 14 membered, more preferably 7 to 10 membered. According to the number of constituent rings, it can be divided into bicyclic, tricyclic, tetracyclic or polycyclic bridged cycloalkyl, preferably bicyclic, tricyclic or tetracyclic, more preferably bicyclic or tricyclic. Examples of bridged cycloalkyl include, but are not limited to:

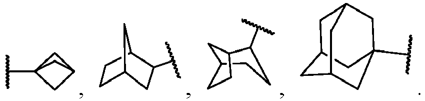

The term "heterocycloalkyl" refers to a saturated or partially unsaturated monocyclic or polycyclic cyclic hydrocarbon substituent having 3 to 20 ring atoms, wherein at least one ring atom is a heteroatom independently selected from boron, silicon, oxygen, sulfur, selenium, nitrogen and phosphorus, and the remaining ring atoms are C. The group may be a carbon group or a heteroatom group (i.e. it may be C-linked or N-linked, as long as it is possible). Examples of heterocyclic groups include, but are not limited to, pyrrolidinyl, tetrahydrofuran, tetrahydrothiophene, tetrahydropyranyl, tetrahydrothiopyranyl, piperidinyl, morpholinyl, 4- thiomorpholinyl, thioalkyl and piperazinyl. For example, the group derived from tetrahydropyrrole can be tetrahydropyrrol-1-yl (N-linked) or tetrahydropyrrol-3-yl (C-linked). For example a 3-7 membered monocyclic ring (1-6 carbon atoms and 1-3 heteroatoms selected from N, O, P, B, Si, S and Se, where N, B, P or Se is optionally substituted by one or more oxygen atoms to obtain groups like NO, BOH, PO, PO₂, SeO; N can be optionally quaternized; S atoms can be optionally substituted by one or more oxygen or nitrogen atoms) to obtain a group like SO, SO₂, S(=O)(=NR^{a}), S(=NR^{b}) or S(=NR^{c})₂, while R^{a}, R^{b} and R^{c} are independently cyano, C₁-C₇ alkyl, C₃-C₁₄ cycloalkyl, "C₃-C₁₄ heterocycloalkyl having 1-4 heteroatoms and one or more heteroatoms of boron, silicon, oxygen, sulfur, selenium, nitrogen and phosphorus", "C₁-C₇ heteroaryl having 1-4 heteroatoms and one or more heteroatoms of boron, silicon, oxygen, sulfur, selenium, nitrogen and phosphorus", C₆-C₁₀ aryl or C₁-C₇ alkoxy; meanwhile, the-CH₂-group may be optionally substituted by -C(=O)-, -C(=S)- or -C(=NR^{d})-, R^{d} is independently cyano, C₁-C₇ alkyl, C₃-C₁₄ cycloalkyl, "C₃-C₁₄ heterocycloalkyl having 1-4 heteroatoms and one or more heteroatoms of boron, silicon, oxygen, sulfur, selenium, nitrogen and phosphorus", "C₁-C₇ heteroaryl having 1-4 heteroatoms and one or more heteroatoms of boron, silicon, oxygen, sulfur, selenium, nitrogen and phosphorus", C₆-C₁₀ aryl or C₁-C₇ alkoxy; when the ring is a 3-membered ring, wherein only one heteroatom is present, or, a bicyclic ring consisting of 7 to 10 atoms (4 to 9 carbon atoms and 1-3 heteroatoms selected from N, O, P, B, Si, S, Se, wherein N, S, B, Se or P is optionally substituted by one or more oxygen atoms to obtain groups like NO, BOH, SO, SO₂, PO, PO₂, SeO, while -CH₂- group may be optionally substituted by -C(=O)-). Depending on the structure, the heterocyclic group can be a monovalent group or a divalent group, i.e., a subheterocyclic group. Spiro heterocyclic group, fused heterocyclic group and bridged heterocyclic group are also included in this definition.

Spiro heterocyclic group (spiro heterocycloalkyl) refers to a 5-20 membered polycyclic heterocyclic group sharing one atom (called spiro atom) between the single rings, wherein one or more ring atoms are heteroatoms selected from nitrogen, oxygen or S(O)ₘ (wherein m is an integer from 0 to 2), and the rest ring atoms are carbon. The spiro heterocyclic group may contain one or more double bonds, but none of the rings has a fully conjugated π electron system. Preferably 6 to 14 membered, more preferably 7 to 12 membered. According to the number of spiro atoms shared between rings, spiro heterocyclic groups are divided into monospirol heterocyclic groups, bisspiro heterocyclic groups or polyspiro heterocyclic groups, preferably monospiro heterocyclic groups and bisspiro heterocyclic groups. More preferably 4-membered/4-membered, 4-membered/5-membered, 4-membered/6-membered, 5-membered/5-membered, 5-membered/6-membered, 6-membered/6-membered monospiro cycloalkyl. Spiro heterocyclic groups include, but are not limited to:

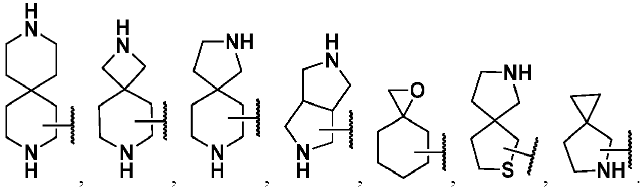

Fused heterocyclic group (fused heterocycloalkyl) refers to a 5-20 membered polycyclic heterocyclic group, each ring in the system shares an adjacent pair of atoms with other rings in the system, one or more rings may contain one or more double bonds, but none of the rings has a completely conjugated π electron system, wherein one or more ring atoms are heteroatoms selected from nitrogen, oxygen or S(O)ₘ (wherein m is an integer 0 to 2), and the remaining ring atoms are carbon. Preferably 6 to 14 membered, more preferably 7 to 12 membered. According to the number of constituent rings, it can be divided into bicyclic, tricyclic, tetracyclic or polycyclic fused heterocyclic groups, preferably bicyclic or tricyclic, more preferably 5-membered/5-membered or 5-membered/6-membered fused heterocyclic groups. Fused heterocyclic groups include, but are not limited to:

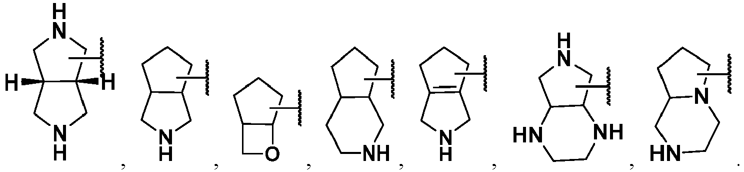

Bridged heterocyclic group (bridged heterocycloalkyl) refers to a 5-20 membered polycyclic heterocyclic group, each ring in the system shares two non-directly connected atoms, which may contain one or more double bonds, but none of the rings has a completely conjugated π electron system, wherein one or more ring atoms are heteroatoms selected from nitrogen, oxygen or S(O)ₘ (wherein m is an integer 0 to 2), and the remaining ring atoms are carbon. Preferably 6 to 14 membered, more preferably 7 to 12 membered. According to the number of constituent rings, it can be divided into bicyclic, tricyclic, tetracyclic or polycyclic heterocyclic groups, preferably bicyclic, tricyclic or tetracyclic, more preferably bicyclic or tricyclic. Bridged heterocyclic groups include, but are not limited to:

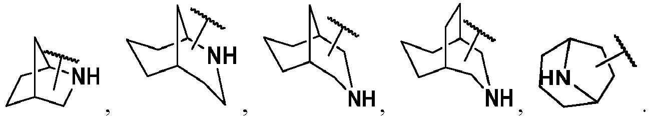

The term "aryl" refers to any stable monocyclic or bicyclic carbon ring having up to 10 atoms in each ring, wherein at least one of which is an aromatic ring. Examples of the above aryl units include phenyl, naphthyl, tetrahydronaphthyl, 2, 3-dihydroindenyl, biphenyl, phenanthrenyl, anthryl or acenaphthyl. It will be understood that in the case where the aryl substituent is a bicyclic substituent and one of the rings is a non-aromatic ring the connection is made through the aromatic ring.

The term "heteroaryl" refers to a stable monocyclic or bicyclic ring having up to 7 atoms in each ring, wherein at least one ring is an aromatic ring and contains 1-4 heteroatoms selected from boron, silicon, oxygen, sulfur, selenium, nitrogen and phosphorus. Heteroaryl within this definition include, but are not limited to: acridinyl, carbazolyl, cinnolinyl, quinoxalinyl, pyrazolyl, indolyl, benzotriazolyl, furanyl, thienyl, benzothienyl, benzofuranyl, quinolyl, isoquinolyl, oxazolyl, isoxazolyl, indolyl, pyrazinyl, pyridazinyl, pyridyl, pyrimidinyl, pyrimidinyl, pyrrolyl, tetrahydroquinolyl. "Heteroaryl" should also be understood to include any N-oxide derivative of a nitrogen-containing heteroaryl. In the case where the heteroaryl substituent is a bicyclic substituent and one ring is a non-aromatic ring or does not contain heteroatoms, it can be understood that the connections are made through aromatic rings respectively. Heteroaromatic ring-fused aromatic ring and bicyclic heteroaromatic ring systems can be fused to form rings. Wherein, N, S, B, P or Se is optionally substituted by one or more oxygen atoms to obtain groups like NO, SO, SO₂, BOH, PO, PO₂, SeO, and the N atom can be quaternized. Heteroaryl can be attached to the main structure at any heteroatom or carbon atom to form stable compounds. Depending on the structure, heteroaryl can be monovalent groups or divalent groups, i.e., heteroarylene.

The term "alkoxy" refers to an alkyl linked by an oxygen bridge; the alkyl is defined as above.

The term "alkylthiol" refers to an alkyl linked by a sulfur bridge; the alkyl is defined as above.

The term "component" refers to each component of the combination of the present disclosure, i.e., the pyrazolone-fused pyrimidine compound represented by formula **I,** the pharmaceutically acceptable salt thereof, the solvate thereof, the solvate of the pharmaceutically acceptable salt thereof, the metabolite thereof or the prodrug thereof, or the anticancer drug.

The term "pharmaceutical excipients" refers to excipients and additives used in drug production and prescription formulation, and refers to all substances contained in pharmaceutical preparations except active ingredients. See the Pharmacopoeia of the People's Republic of China (2015 Edition) Part IV, or, Handbook of Pharmaceutical Excipients (Raymond C Rowe, 2009 Sixth Edition).

The term "treatment" refers to therapeutic therapy. When referring to a specific condition, treatment means (1) alleviating one or more biological manifestations of the disease or condition, (2) interfering with (a) one or more points in the biological cascade causing or contributing to the condition or (b) one or more biological manifestations of the condition, (3) ameliorating one or more symptoms, effects, or side effects associated with the condition or its treatment, or one or more symptoms, effects, or side effects, or (4) slowing the development of the condition or one or more biological manifestations of the condition.

The term "prevention" refers to a reduction in the risk of acquiring or developing diseases or disorders.

The term "therapeutically effective amount" refers to the amount of a compound that is sufficient to effectively treat the diseases or disorders described herein when administered to a patient. The "therapeutically effective amount" will vary according to the compound, the condition and its severity, and the age of the patient to be treated, but it can be adjusted by those skilled in the art as needed.

The term "patient" refers to any animal, preferably a mammal, preferably a human, to which the compound or composition is to be administered or has been administered according to an embodiment of the present disclosure. The term "mammal" includes any mammal. Examples of mammals include, but are not limited to, cattle, horses, sheep, pigs, cats, dogs, mice, rats, rabbits, guinea pigs, monkeys, humans, etc., with humans being the most preferred.

The term "active ingredient" refers to the active ingredient in the pharmaceutical composition or combination kit of the present disclosure, i.e., the ompound represented by formula I, the pharmaceutically acceptable salt thereof, the solvate thereof, the solvate of the pharmaceutically acceptable salt thereof, the metabolite thereof or the prodrug thereof, the anticancer drug, or a combination thereof.

The absolute configuration of a stereocenter is represented by a wedge-shaped bond and a dashed line bond ( 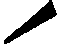 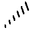 ).

On the basis of not violating the common sense in the field, the above preferred conditions can be arbitrarily combined to obtain the preferred embodiments of the present disclosure.

The reagents and raw materials used in the present disclosure are commercially available.

The positive progressive effect of the present disclosure is that: the compounds of the present disclosure have better inhibitory activity against WEE1 kinase and have better bioavailability.

### Detailed description of the embodiment

The present disclosure will be further illustrated by way of embodiments below, but it is not limited to the scope of the embodiments. The experimental methods not specified in the specific conditions in the following embodiments are selected according to the conventional methods and conditions, or according to the commodity specifications.

The structures of all compounds of the present disclosure can be identified by nuclear magnetic resonance (¹HNMR) and/or mass spectrometry (MS). ¹H NMR chemical shift (δ) was recorded in PPM (10⁻⁶). NMR was performed by Bruker AVANCE-400 spectrometer.

LC-MS was determined by Agilent 1200HPLC/6120 mass spectrometer.

HPLC was determined by Agilent 1260 high performance liquid chromatograph. Specific conditions of HPLC: mobile phase A: water (0.1 % formic acid), mobile phase B: acetonitrile; column time: 15min; column type: Waters' Xselect, 5 µm, 4.6 × 250 mm.

The thin layer silica gel plate was Liangchen silicon source HSGF254 or Qingdao GF254 silica gel plate. Column chromatography generally uses Yantai Huanghai 200-300 mesh silica gel as carrier.

### Embodiment 1

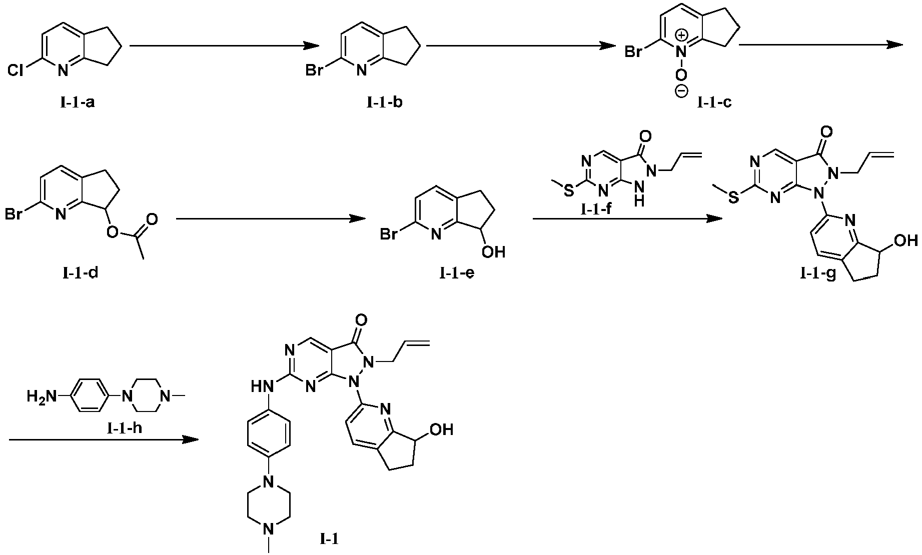

### Step 1:

1, 2-Chloro-6,7-dihydro-5*H*-cyclopenta[*b*]pyridine (1 g, 6.51 mmol) (**I-1-a**) was added to 30 mL of toluene, then phosphorus oxybromide (4 g, 13.95 mmol) was added thereto, and the mixture was stirred at 130 °C for three days. After the reaction mixture was cooled and concentrated, saturated sodium bicarbonate solution was slowly added until the pH value was about 10; then the mixture was extracted three times with dichloromethane (3^{∗}30 mL), dried over anhydrous sodium sulfate and concentrated to obtain a crude product, the crude product was purified through a column (ethyl acetate: petroleum ether=0-15%) to obtain 0.84 g of 2-bromo-6,7-dihydro-5*H*-cyclopenta[*b*]pyridine (**I-1-b**) as a brown solid, yield: 65 %. LC-MS: m / z: (M+H)⁺=198.

### Step 2:

2-Bromo-6,7-dihydro-5*H*-cyclopenta[*b*]pyridine (**I-1-b**) (0.8 g, 4 mmol) was added to 25 mL of dichloromethane, then 77 % *m*-chloroperoxybenzoic acid (1.34 g, 6 mmol) was also added to 25 mL of dichloromethane, and the mixture was added to a solution of the compound represented by formula **I-1-b**, and stirred at room temperature overnight. The reaction mixture was washed with saturated sodium bicarbonate solution, and the aqueous phase was extracted with dichloromethane for three times (3^{∗}30 mL), the combined organic layer was dried over anhydrous sodium sulfate and concentrated to obtain a crude product, the crude product was purified by a column (ethyl acetate: petroleum ether =0-60 %) to obtain 0.74 g of 2-bromo-6,7-dihydro-5*H*-cyclopenta[*b*]pyridine 1-oxide (represented by formula **I-1-c**) as a white solid, yield: 90%. LC-MS: m / z: (M+H)⁺=214.

### Step 3:

2-Bromo-6,7-dihydro-5*H*-cyclopenta[*b*]pyridine 1-oxide (represented by formula **I-1-c**) (0.74 g, 3.5 mmol) was dissolved in 20 mL of acetic anhydride, and the mixture was stirred overnight at 120 °C. The reaction mixture was concentrated, and then 30 mL of dichloromethane was added thereto, the mixture was washed twice with saturated sodium bicarbonate solution (2^{∗}10 mL), then washed once with 10 mL of saturated saline, dried over anhydrous sodium sulfate and concentrated, then the residue was passed through a column (ethyl acetate: petroleum ether=0-30%) to obtain 0.74 g of 2-bromo-6,7-dihydro-5*H-*cyclopenta[*b*]pyridin-7-yl acetate (**I-1-d**) as a colorless oil, yield: 84 %. LC-MS: m / z: (M+H)⁺=256.

### Step 4:

2-Bromo-6,7-dihydro-5*H*-cyclopenta[*b*]pyridin-7-yl acetate (**I-1-d**) (0.74 g, 2.9 mmol) was added to 6 mL of ethanol, then 15 mL of a solution of potassium hydroxide (172 mg, 3.07 mmol) in ethanol was added thereto. The mixture was stirred at room temperature for 3 hours. 25 mL of water was added to the reaction mixture, most of the ethanol was evaporated; then the mixture was extracted with dichloromethane (3^{∗}25 mL), washed with 20 mL of saturated saline, dried over anhydrous sodium sulfate and concentrated, then the residue was passed through a column (ethyl acetate: petroleum ether=0-30%) to obtain 0.57 g of 2-bromo-6,7-dihydro-5*H*-cyclopenta[*b*]pyridin-7-ol (**I-1-e**) as a colorless oil, yield: 92%. ¹H NMR (400 MHz, CDCl₃) δ 7.45 (d, *J* = 8.0 Hz, 1H), 7.35 (d, *J* = 8.0 Hz, 1H), 5.22 (td, *J* = 7.5, 3.3 Hz, 1H), 3.26 (d, *J* = 3.4 Hz, 1H), 3.01 (m, 1H), 2.80 (m, 1H), 2.63 - 2.50 (m, 1H), 2.09 (m, 1H). LC- MS: m / z: (M+H)⁺=214.

### Step 5:

2-Bromo-6,7-dihydro-5*H*-cyclopenta[b]pyridin-7-ol (0.54 g, 2.52 mmol) (represented by formula I-1-e), 2-allyl-6-(methylthio)-1,2-dihydro-3*H*-pyrazolo[3,4-*d*]pyrimidin-3-one (0.56 g, 2.52 mmol) (**I-1-f**), cuprous iodide (0.482 g, 2.52 mmol), potassium carbonate (0.7 g, 5.07 mmol) and *N*¹,*N*²-dimethylethylenediamine (0.46 g, 5.2 mmol) were added to 20 mL of 1,4-dioxane, and the mixture was stirred overnight at 105 °C under the protection of argon. The reaction product was cooled and concentrated to obtain a crude product, then the crude product was purified by passing through a column (methanol: dichloromethane=0-10%) to obtain 0.42 g of 2-allyl-1-(7-hydroxy-6,7-dihydro-5*H-*cyclopenta[*b*]pyridin-2-yl)-6-(methylthio)-1,2-dihydro-3*H*-pyrazolo[3,4-d]pyrimidin-3-one (**I-1-g**) as a white solid, yield: 46 %. LC-MS: m / z: (M+H)⁺=356.

### Step 6:

*m*-Chloroperoxybenzoic acid (108 mg, 0.48 mmol) was added to a solution of 2-allyl-1-(7-hydroxy-6,7-dihydro-5*H*-cyclopenta[*b*]pyridin-2-yl)-6-(methylthio)-1,2-dihydro-3*H*-pyrazolo[3,4-*d*]pyrimidin-3-one (142mg, 0.4 mmol) (**I-1-g**) in 30 mL of toluene, and the obtained mixture was stirred at room temperature for 2 hours. 4-(4-Methylpiperazin-1-yl)aniline (100mg, 0.52 mmol) (I-1-h) and 2 mL of *N*, *N*-diisopropylethylamine were added to the above reaction mixture, and the mixture was stirred overnight at room temperature. The reaction mixture was concentrated to obtain a crude product, and the crude product was purified by passing through a column (methanol: dichloromethane = 0-20%), and then prepared by high performance liquid chromatography to obtain 95 mg of 2-allyl-1-(7-hydroxy-6,7-dihydro-5*H-*cyclopenta[*b*]pyridin-2-yl)-6-((4-(4-methylpiperazin-1-yl)phenyl)amino)-1,2-dihydro-3*H*-pyrazolo[3, 4-*d*]pyrimidin-3-one (**I-1**) as a yellow solid, yield: 47 %. ¹H NMR (400 MHz, CDCl₃) δ 8.73 (s, 1H), 8.39 (s, 1H), 7.76 (d, *J* = 8.2 Hz, 1H), 7.59 (d, *J* = 8.1 Hz, 1H), 7.44 (d, J= 8.0 Hz, 2H), 6.81 (d, *J* = 8.8 Hz, 2H), 5.68 (ddd, *J* = 16.5, 12.1, 6.1 Hz, 1H), 5.25 (t, *J* = 6.7 Hz, 1H), 5.03 (d, J= 10.1 Hz, 1H), 4.94 (d, *J* = 17.2 Hz, 1H), 4.77 - 4.60 (m, 2H), 3.34 (s, 4H), 3.11 (m, 5H), 2.96 - 2.85 (m, 1H), 2.64 (m, 4H), 2.12 (m,1H). LC- MS: m / z: (M+H)⁺ =499.

### Embodiment 2

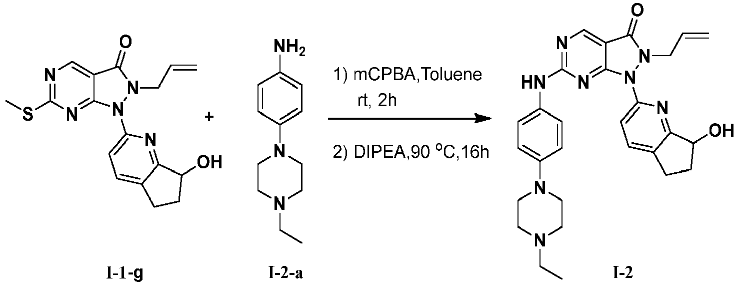

2-Allyl-1-(7-hydroxy-6,7-dihydro-5*H*-cyclopenta[*b*]pyridin-2-yl)-6-(methylthio)-1,2-dihydro-3*H*-pyrazolo[3,4-*d*]pyrimidin-3-one (40 mg, 0.11 mmol) (**I-1-g**) and 3-chloroperoxybenzoic acid (30.2 mg, 0.135 mmol) were dissolved in 15 mL of toluene, the obtained solution was stirred at room temperature for 2 hours. 4-(4-Ethylpiperazin-1-yl)aniline (30 mg, 0.14mmol) and *N*,*N*-diisopropylethylamine (29.0 mg, 0.22 mmol) were added to the above solution, and the reaction mixture was stirred for 16 hours at 90 °C under the protection of nitrogen. The reaction mixture was concentrated, and the obtained solid was separated by thin layer chromatography plate (dichloromethane: methanol=10:1) to obtain a crude product, the crude product was then separated by preparative HPLC to obtain 38.6 mg of 2-allyl-6-((4-(4-(4-ethylpiperazin-1-yl)phenyl)amino)-1-(7-hydroxy-6,7-dihydro-5H-cyclopenta[*b*]pyridin-2-yl)-1,2-dihydro-3*H*-pyrazolo[3,4-*d*]pyrimidin-3-one (1-2) as a white solid, yield: 66.9 %. ¹H NMR (400 MHz, CDCl₃) δ8.68 (d, J = 34.2 Hz, 1H), 8.49 (s, 1H), 7.87 (d, J = 8.1 Hz, 1H), 7.66 (d, J= 8.1 Hz, 1H), 7.52 (d, J = 8.8 Hz, 2H), 6.89 (d, J = 8.9 Hz, 2H), 5.73 (ddd, J = 12.8, 8.2, 3.9 Hz, 1H),5.14 (dd, J = 7.1, 5.7 Hz, 1H), 5.04 (dd, J = 10.2, 1.0 Hz, 1H), 4.97 (d, J = 1.2 Hz, 1H), 4.80 - 4.66 (m,2H), 3.49 - 3.27 (m, 8H), 3.19 (dd, J = 14.0, 6.8 Hz, 2H), 3.10 (ddd, J = 16.1, 8.6, 4.6 Hz, 1H), 2.98 - 2.80(m, 1H), 2.56 (dtd, J = 13.0, 8.1, 4.7 Hz, 1H), 2.15 - 1.96 (m, 1H), 1.38 (t, J = 7.0 Hz, 3H). LC- MS: m / z: (M+H)⁺ =513.

### Embodiment 5

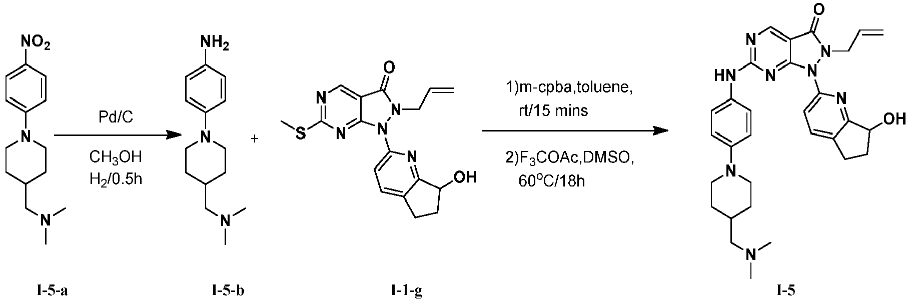

### Step 1:

67 mg (0.25 mmol) of *N*, *N*-dimethyl-1-(1-(4-nitrophenyl)piperidin-4-yl) methanamine (**I-5-a**) was dissolved in 5 mL of methanol, and the reaction was carried out for half an hour under hydrogen and catalyzed by palladium carbon. The reaction mixture was filtered and concentrated to obtain 56 mg of 4-(4-((dimethylamino)methyl)piperidin-1-yl)aniline (**I-5-b**) as a yellow solid, yield: 97 %. LC-MS: m / z: (M+H)⁺ =234.

### Step 2:

2-Allyl-1-(7-hydroxy-6,7-5*H*-cyclopenta[*b*]pyridin-2-yl)-6-(methylthio)-1,2-dihydro-3H-pyrazolo[3,4-*d*]pyrimidin-3-one (**I-1-g**) was dissolved in 10 mL of toluene, and 30 mg (0.17 mol, 77 %) of 3-chloroperoxybenzoic acid was added thereto, the mixture was stirred at room temperature for 15 min, and the toluene was evaporated to dryness, then a solution of 4-(4-(dimethylamino)methyl)piperidin-1-yl)aniline (I-5-b) (40 mg, 0.11 mmol) in 5 mL of dimethyl sulfoxide was added thereto, and 0.4 mL of trifluoroacetic acid was added thereto, then the temperature was raised to 60 °C, and the mixture was stirred for about 18 hours. The reaction mixture was cooled and extracted with water and ethyl acetate. The mixture was concentrated, then the residue was purified by passing through a thin layer chromatography (DCM/CH₃OH/NH₃.CH₃OH = 100/10/1.5) three times to obtain 29 mg of 2-allyl-6-((4-((dimethylamino)methylpiperidin-1-yl)phenylamino)-1-(7-hydroxy-6,7-dihydro-5*H*-cyclopenta[*b*]pyridin-2-yl)-1,2-dihydro-3*H*-pyrazolo[3,4-*d*]pyrimidin-3-1 (**I-5**) as a yellow solid, yield: 32 %. ¹H NMR (400 MHz, Chloroform-d) δ 8.79 (s, 1H), 7.73 (d, J = 8.2 Hz, 1H), 7.63 (d, J = 8.4 Hz, 1H), 7.41 (d, J = 8.4Hz, 2H), 6.93 - 6.79 (m, 2H), 5.68 (ddt, J = 16.6, 10.2, 6.2 Hz, 1H), 5.22 (t, J = 6.8 Hz, 1H), 5.02 (dd, J = 10.2, 1.3 Hz,1H), 4.94 (dt, J = 17.1, 1.4 Hz, 1H), 4.68 (s, 2H), 3.64 (d, J = 12.2 Hz, 2H), 3.09 (ddd, J = 16.7, 9.1, 3.8 Hz, 1H), 2.88(dd, J = 16.2, 7.9 Hz, 1H), 2.69 (td, J = 12.4, 2.7 Hz, 2H), 2.63 - 2.56 (m, 1H), 2.40 (s, 6H), 2.24 - 2.18 (m, 1H), 2.09(ddt, J = 13.3, 8.7, 6.7 Hz, 3H), 2.00 (d, J = 6.6 Hz, 1H), 1.94 (d, J = 13.1 Hz, 3H), 1.41 (tt, J = 12.1, 6.2 Hz, 2H). LC-MS: m / z: (M+H)⁺ =541.

### Embodiment 9

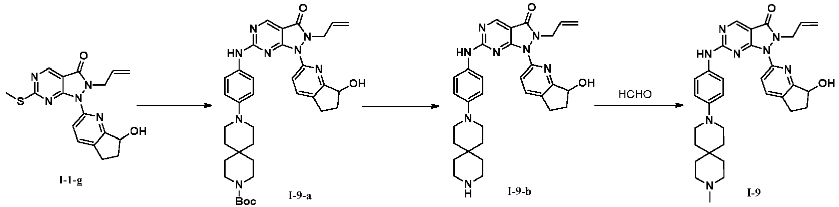

### Step 1:

2-Allyl-1-(7-hydroxy-6,7-dihydro-5*H*-cyclopenta[*b*]pyridin-2-yl)-6-(methylthio)-1,2-dihydro-3*H*-pyrazolo[3,4-*d*]pyrimidin-3-one (**I-1-g**) (40 mg, 0.11 mmol) was dissolved in 10 mL of toluene, and 3-chloroperoxybenzoic acid (30 mg, 0.13 mmol ) was added thereto, and the reaction mixture was stirred at 20 °C for 2 hours. Diisopropylethylamine (30 mg, 0.23 mmol) and *tert-butyl* 3-(4-aminophenyl)-3,9-diazaspiro[5.5]undecane-9-carboxylate (40 mg, 0.12 mmol) were added, and the reaction mixture was stirred at 20 °C for 24 hours. The reaction mixture was concentrated, and prepared by TLC (dichloromethane/methanol = 10/1) to obtain methyl 9-(4-((2-allyl-1-(7-hydroxy-6,7-dihydro-5*H-*cyclopenta[*b*]pyridin-2-yl)-3-oxo-2,3-dihydro-*tert*-butyl-1*H*-pyrazolo[3,4-*d*]pyrimidin-6-yl)amino)phenyl)-3,9-diazaspiro[5.5]undecane-3-carboxylate (I-9-a) (30 mg, 40.83 %) as a yellow solid. LC-MS: m / z: (M+H)⁺ =653.

### Step 2:

Methyl 9-(4-((2-allyl-1-(7-hydroxy-6,7-dihydro-5*H*-cyclopenta[*b*]pyridin-2-yl)-3-oxo-2,3-dihydro-*tert*-butyl-1*H*-pyrazolo[3,4-*d*]pyrimidin-6-yl)amino)phenyl)-3,9-diazaspiro[5.5]undecane-3-carboxylate (I-9-a) (30 mg, 0.046 mmol) was dissolved in 4 mL of dichloromethane, and 1 mL of trifluoroacetic acid was added thereto, then the reaction mixture was stirred at 20 °C for 1 hour. The reaction mixture was concentrated to obtain 6-((4-(3, 9-diazaspiro[5.5]undecan-3-yl)phenyl) amino)-2-allyl-1-(7-hydroxy-6, 7-dihydro-5*H*-cyclopenta[*b*]pyridin-2-yl)-1, 2-dihydro-3*H*-pyrazolo [3,4-*d*]pyrimidin-3-one (I-9-b) (30 mg, 118.1 %) as a yellow-brown solid. LC-MS: m / z: (M+H)⁺ =553.

### Step 3:

6-((4-(3,9-Diazaspiro[5.5]undecan-3-yl)phenyl)amino)-2-allyl-1-(7-hydroxy-6,7-dihydro-5*H-*cyclopenta[*b*]pyridin-2-yl)-1,2-dihydro-3*H*-pyrazolo[3,4-*d*]pyrimidin-3-one (I-9-b) (30 mg, 0.054 mmol) was dissolved in 2 mL of methanol, and 37 % formaldehyde aqueous solution (88 mg, 1.08 mmol) was added thereto, and the reaction mixture was stirred at 20 °C for 2 hours. Sodium triacetoxyborohydride (17 mg, 0.081 mmol) was added, and the reaction mixture was stirred at 20 °C for 24 hours. The reaction mixture was quenched with saturated sodium bicarbonate aqueous solution, extracted with dichloromethane, the organic phase was dried over anhydrous sodium sulfate, filtered, concentrated and prepared by TLC (dichloromethane/methanol = 10/1) to obtain 2-allyl-1-(7-hydroxy-6,7-dihydro-5*H*-cyclopenta[*b*]pyridin-2-yl)-6-((4-(9-methyl-3,9-diazaspiro[5.5]undecan-3-yl)phenyl)amino)-1,2-dihydro-3*H*-pyrazolo[3,4-*d*]pyrimidin-3-one (1-9) as a yellow solid (10.77 mg, 35.01 %). ¹H NMR (400 MHz, CDCl₃) δ1H NMR (400 MHz, MeOD) δ8.79 (d, *J* = 3.1 Hz, 1H), 7.89 (d, *J* = 8.2 Hz, 1H), 7.71 (d, *J* = 8.1 Hz, 1H),7.50 (dd, *J* = 22.7, 8.8 Hz, 2H), 6.96 (t, *J* = 6.2 Hz, 2H), 5.74 (ddt, J= 16.4, 10.3, 6.1 Hz, 1H), 5.16 - 5.10(m, 1H), 5.06 (dd, *J* = 10.3, 1.2 Hz, 1H), 4.99 (d, *J* = 1.3 Hz, 1H), 4.76 (d, *J* = 6.0 Hz, 2H), 3.38 - 3.28 (m,4H), 3.07 (d, *J* = 4.8 Hz, 1H), 2.96 - 2.79 (m, 1H), 2.64 - 2.45 (m, 5H), 2.28 (s,3H), 2.12 - 1.97 (m, 1H), 1.75 - 1.54(m, 8H). LC- MS: m / z: (M+H)⁺ =567.

### Embodiment 12

### Step 1:

*Tert*-butyl 5-(4-nitrophenyl)-2,5-diazabicyclo[2.2.1]heptane-2-carboxylate (64 mg, 0.2 mmol) (I-12-a) and 10% palladium carbon (30 mg) were added to 25 mL of methanol, and the reaction flask was ventilated three times with a hydrogen balloon, and the reaction mixture was stirred for 3 hours at room temperature in a hydrogen atmosphere. The reaction mixture was filtered and evaporated to dryness to obtain 55 mg of *tert*-butyl 5-(4-aminophenyl)-2,5-diazabicyclo[2.2.1]heptane-2-carboxylate (I-12-b) as a brown solid, which was directly used in the next step. The yield was 95 %. LC- MS: m / z: (M+H)⁺ =290.

### Step 2:

*m*-Chloroperoxybenzoic acid (25mg, 0.112 mmol) was added to a solution of 2-allyl-1-(7-hydroxy-7-methyl-6,7-dihydro-5*H*-cyclopenta[*b*]pyridin-2-yl)-6-(methylthio)-1,2-dihydro-3*H-*pyrazolo[3,4-*d*]pyrimidin-3-one (35mg, 0.95 mmol) (I-16-h) in 20 mL of toluene, and the obtained mixture was stirred at room temperature for 15 min. *Tert*-butyl 5-(4-aminophenyl)-2,5-diazabicyclo[2.2.1]heptane-2-carboxylate (55mg, 0.19mmol) (I-12-b) and 0.2 mL of *N*,*N*-diisopropylethylamine were added to the above reaction mixture, and the mixture was stirred overnight at room temperature. The reaction mixture was concentrated to obtain a crude product, the crude product was purified by thin layer chromatography {7 M ammonia methanol: (dichloromethane: ethyl acetate = 8: 2)} = 1:18 to obtain 40 mg of *tert*-butyl 5-(4-((2-allyl-1-(7-hydroxy-7-methyl-6,7-dihydro-5*H*-cyclopenta[*b*]pyridin-2-yl)-3-oxo-2,3-dihydro-1H-pyrazolo[3,4-*d*]pyrimidin-6-yl)amino)phenyl)-2,5-diazabicyclo[2.2.1]heptane-2-carboxylate (**I-12-c**) as a yellow solid, yield: 69%. LC-MS: m / z: (M+H)⁺ =611.

### Step 3:

*Tert*-butyl 5-(4-((2-allyl-1-(7-hydroxy-7-methyl-6,7-dihydro-5*H*-cyclopenta[*b*]pyridin-2-yl)-3-oxo-2,3-dihydro-1*H*-pyrazolo[3,4-*d*]pyrimidin-6-yl)amino)phenyl)-2,5-diazabicyclo[2.2.1]heptane-2-carboxylate (30mg, 0.049 mmol) (I-12-c) was added to 10mL of dichloromethane, then zinc bromide (40 mg, 0.178 mmol) was added thereto, and the mixture was stirred at room temperature for 0.5 hours. After the reaction mixture was concentrated, 30mL of dichloromethane, 1 mL of methanol and saturated sodium bicarbonate solution (3 mL) were added thereto; after the mixture was stirred for 10 minutes, the organic layer was dried over anhydrous sodium sulfate and purified by thin layer chromatography {7 M ammonia methanol: (dichloromethane: ethyl acetate = 9: 1)} = 1: 8 to obtain 14 mg of 6-((4-(2,5-diazabicyclo[2.2.1]heptan-2-yl)phenyl)amino)-2-allyl-1-(7-hydroxy-7-methyl-6,7-dihydro-5*H-*cyclopenta[*b*]pyridin-2-yl)-1,2-dihydro-3*H*-pyrazolo[3,4-*d*]pyrimidin-3-one (**I-12**) as a yellow solid, yield: 55%. ¹H NMR (400 MHz, CDCl₃) δ 8.71 (d, 1H), 7.70 (m, 2H), 7.34 (d, J = 6.4 Hz, 2H), 6.47 (d, J = 8.6 Hz, 2H), 5.79 - 5.59 (m, 1H), 5.01 (d, J = 10.2 Hz, 1H), 4.92 (d, J = 17.1 Hz, 1H), 4.75 (m, 2H), 4.30 (s, 1H), 3.88 (d, J = 6.5 Hz, 1H), 3.63 (d, J = 7.4 Hz, 1H), 3.21 - 2.98 (m, 4H), 2.93 - 2.82 (m, 2H), 2.42 - 2.25 (m, 2H), 2.02 - 1.84 (m, 2H), 1.63 (d, 3H). LC-MS: m / z: (M+H)⁺ =511.

### Embodiment 13

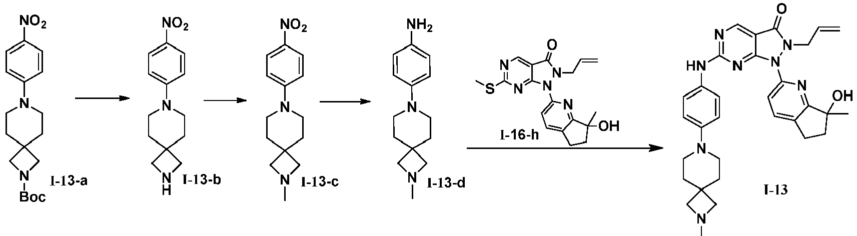

### Step 1:

*Tert*-butyl 7-(4-nitrophenyl)-2,7-diazaspiro[3-4]nonane-2-carboxylate (80 mg, 0.23 mmol) (1-13-a) was added to a mixed solvent consisting of 5 mL of dichloromethane and 5 mL of trifluoroacetic acid, and the mixture was stirred at room temperature for 2 hours. The reaction mixture was concentrated to obtain a crude product as a yellow solid (**I-13-b**), which was directly used in the next step. LC-MS: m / z: (M+H)⁺ =248.

### Step 2:

7-(4-Nitrophenyl)-2,7-diazaspiro[3.5]nonane (55 mg, 0.22 mmol) (**I-13-b**), 0.5 mL of *N,N-*diisopropylethylamine, 0.4 mL of formaldehyde aqueous solution and sodium borohydride acetate (240 mg, 1.13 mol) were added to 15 mL of methanol, and the mixture was stirred at room temperature overnight. After the reaction mixture was concentrated, 20mL of dichloromethane and 1mL of methanol were added, the mixed solution was washed with saturated sodium carbonate solution (5mL), the organic layer was dried over anhydrous sodium sulfate and concentrated to obtain 50 mg of 2-methyl-7-(4-nitrophenyl)-2, 7-diazaphenyl[3.5]nonane (I-13-c) as a yellow solid with a yield of 86 %. LC-MS: m / z: (M+H)⁺ =262.

### Step 3:

2-Methyl-7-(4-nitrophenyl)-2,7-diazaspiro[3.5]nonane (60 mg, 0.23 mmol) (I-13-c) and 10 % palladium carbon (30 mg) were added to 20 mL of methanol, and the reaction flask was ventilated three times with a hydrogen balloon, and the reaction mixture was stirred for 2 hours at room temperature in a hydrogen atmosphere. The reaction mixture was filtered and evaporated to dryness to obtain 50 mg of 4-(2-methyl-2,7-diazaspiro[3.5]nonan-7-yl)aniline (I-13-d) as a brown solid, which was directly used in the next step. The yield was 94%. LC-MS: m / z: (M+H)⁺ =232.

### Step 4:

*m*-Chloroperoxybenzoic acid (22 mg, 0.1 mmol) with a content of 77 % was added to a solution of 2-allyl-1-(7-hydroxy-7-methyl-6,7-dihydro-5*H*-cyclopenta[*b*]pyridin-2-yl)-6-(methylthio)-1,2-dihydro-3*H*-pyrazolo[3,4-*d*]pyrimidin-3-one (35 mg, 0.09 mmol) (**I-16-h**) in 15 mL of toluene, and the obtained mixture was stirred at room temperature for 15 min. A mixture of 4-(2-methyl-2,7-diazaspiro[3.5]nonan-7-yl)aniline (50 mg, 0.22 mmol) (**I-13-d**) and 0.2 mL of *N*,*N*-diisopropylethylamine in 3 mL of dichloromethane in the above reaction mixture was stirred at room temperature overnight. The reaction mixture was concentrated to obtain a crude product, the crude product was purified by thin layer chromatography {7 M ammonia methanol:(dichloromethane: ethyl acetate=8:2)}=1:20 to obtain 7 mg of 2-allyl-1-(7-hydroxy-7-methyl-6,7-dihydro-5*H*-cyclopenta[*b*]pyridin-2-yl)-6-((4-(2-methyl-2,7-diazaspiro[3.5]nonan-7-yl)phenyl)amino)-1,2-dihydro-3*H*-pyrazolo[3,4-*d*]pyrimidin-3-one (**I-13**) as a yellow solid with a yield of 13%. ¹H NMR (400 MHz, CDCl₃) δ 8.73 (s, 1H), 7.69 (m,2H), 7.43 (d, *J* = 8.8 Hz, 2H), 6.88 (d, *J* = 9.0 Hz, 2H), 5.69 (ddt, *J* = 16.8, 10.2, 6.3 Hz, 1H), 5.01 (dd, *J* = 10.2, 1.0 Hz, 1H), 4.92 (dd, *J* = 17.1, 1.2 Hz, 1H), 4.84 (dd, *J* = 15.6, 5.5 Hz, 1H), 4.72 (dd, *J* = 15.7, 6.6 Hz, 1H), 3.22 (d, *J* = 13.5 Hz, 4H), 3.11-3.02 (m, 5H), 2.92-2.83 (m, 1H), 2.50-2.42 (s, 3H), 2.42-2.26 (m, 2H), 1.96-1.87 (m, 4H), 1.63 (s, 3H). LC- MS: m / z: (M+H)⁺ =553.

### Embodiment 14

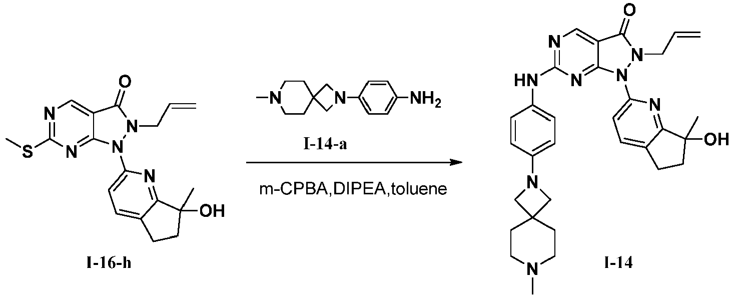

2-Allyl-1-(7-hydroxy-7-methyl-5,6-dihydrocyclopenta[*b*]pyridin-2-yl)-6-methylthio-pyrazolo [3,4-*d*]pyrimidin-3-one (**I-16-h**) (40 mg, 0.11 mmol) was dissolved in 10 mL of toluene, and 3-chloroperoxybenzoic acid (30 mg, 0.13 mmol ) was added thereto, and the reaction mixture was stirred at 20 °C for 2 hours. Diisopropylethylamine (30 mg, 0.23 mmol) and 4-(7-methyl-2,7-diazaspiro[3.5]nonan-2-yl)aniline (**I-14-a**) (30 mg, 0.13 mmol) were added, and the reaction mixture was stirred at 20 °C for 24 hours. The reaction mixture was filtered, concentrated and prepared by TLC (dichloromethane/methanol = 10/1) to obtain 2-allyl-1-(7-hydroxy-7-methyl-6,7-dihydro-5*H*-cyclopenta[*b*]pyridin-2-yl)-6-((4-(7-methyl-2,7-diazaspiro[3.5]nonan-2-yl)phenyl)amino)-1,2-dihydro-3*H*-pyrazolo[3,4-*d*]pyrimidin-3-one (represented by formula **I-14**) as a yellow solid (28 mg, 46.78 %). ¹H NMR (400 MHz, CDCl₃) δ1H NMR (400 MHz, MeOD) δ 8.71 (d, *J* = 40.7 Hz, 1H), 7.86 (dd, *J* = 15.2, 7.9 Hz, 1H), 7.71 (d, *J* = 8.1Hz, 1H), 7.41 (ddd, *J* = 29.8, 17.3, 7.9 Hz, 2H), 6.46 (d, *J* = 8.9 Hz, 2H), 5.73 (ddt, *J* = 16.4, 10.3,6.1 Hz,1H), 5.04 (dd, *J* = 10.2, 1.1 Hz, 1H), 4.98 - 4.91 (m, 2H), 4.76 (dt, *J* = 23.8, 14.4 Hz, 2H), 3.62 (s, 4H),3.06 (dt, *J* = 16.0, 6.9 Hz, 1H), 2.95 - 2.67 (m, 5H), 2.55 (s, 3H), 2.25 (dt, *J* = 15.2, 7.4 Hz, 2H), 1.98 (t, *J*= 5.3 Hz, 4H), 1.59 (s, 3H). LC- MS: m / z: (M+H)⁺ =553.

### Embodiment 16

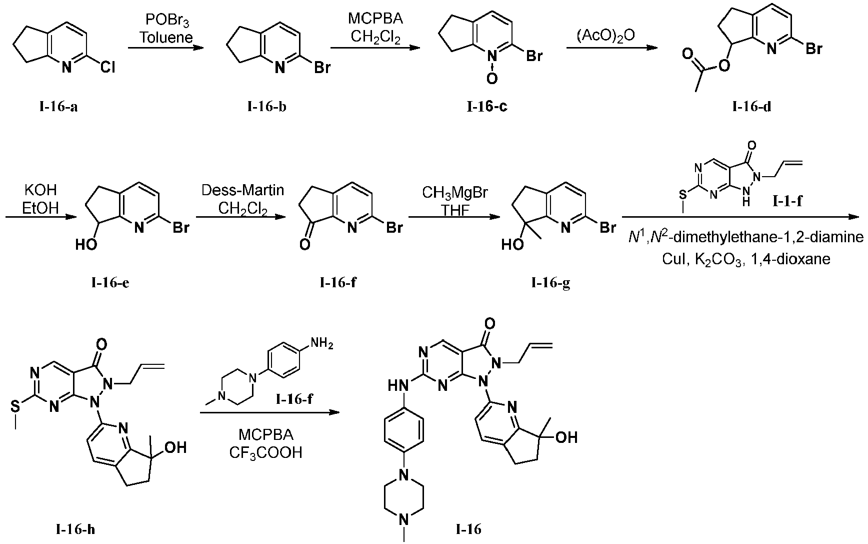

### Step 1:

2-Chloro-6,7-dihydro-5*H*-cyclopenta[*b*]pyridine (**I-16-a**) (23.0 mmol) was dissolved in toluene (30 mL), phosphorus oxybromide (46 mmol) was added to the reaction mixture, the reaction mixture was heated to reflux and stirred for 16 hours. The pH value of the reaction mixture was adjusted to 9 by adding saturated sodium bicarbonate aqueous solution, extracted with ethyl acetate, the organic phase was washed with saturated saline, dried over anhydrous sodium sulfate, suction-filtered, the crude product was evaporated to dryness, and the crude product was purified by column chromatography (petroleum ether/ethyl acetate=100/0-50/50) to obtain the target compound 2-bromo-6,7-dihydro-5*H*-cyclopenta[*b*]pyridine (**I-16-b**) (4.0 g, 89 %) as a gray solid. LC-MS: m / z: (M+H)⁺ =198.

### Step 2:

2-Bromo-6,7-dihydro-5*H*-cyclopenta[*b*]pyridine (**I-16-b**) (18.0 mmol) was dissolved in dichloromethane (20 mL), 3-chloroperoxybenzoic acid (27.0 mmol, 70 %) was added to the reaction mixture, and the reaction mixture was stirred at room temperature for 16 hours. After the reaction was completed, the pH value of the reaction mixture was adjusted to 9 by adding saturated sodium bicarbonate aqueous solution, then the mixture was extracted with dichloromethane; the organic phase was washed with saturated saline, dried over anhydrous sodium sulfate, suction-filtered, evaporated to dryness, and the crude product was purified by column chromatography (dichloromethane/methanol=100/0-97/3) to obtain the target compound 2-bromo-6,7-dihydro-5*H*-cyclopenta[*b*]pyridine 1-oxide (**I-16-c**) (3.2 g, 84 %) as a white solid. LC-MS: m / z: (M+H)⁺ =216.

### Step 3:

2-Bromo-6,7-dihydro-5*H*-cyclopenta[*b*]pyridine 1-oxide (**I-16-c**) (15.0 mmol) was dissolved in acetic anhydride (30 mL), and the reaction mixture was stirred at 110 °C for 16 hours, and the reaction mixture was evaporated to dryness. The pH value of the reaction mixture was adjusted to 9 by adding saturated sodium bicarbonate aqueous solution, extracted with ethyl acetate, the organic phase was washed with saturated saline, dried over anhydrous sodium sulfate, suction-filtered, the crude product was evaporated to dryness, and the crude product was purified by column chromatography (petroleum ether/ethyl acetate=100/0-50/50) to obtain the target compound 2-bromo-6,7-dihydro-5*H*-cyclopenta[*b*]pyridin-7-yl acetate (**I-16-d**) (2.7 g, 71 %) as a yellow solid. LC-MS: m / z: (M+H)⁺ =256.

### Step 4:

2-Bromo-6,7-dihydro-5*H*-cyclopenta[*b*]pyridin-7-yl acetate (**I-16-d**) (11.0 mmol) was dissolved in ethanol (15 mL) and water (10 mL), potassium hydroxide (12.0 mmol) was added thereto, and the reaction mixture was stirred for 3 hours at room temperature. The reaction mixture was extracted with ethyl acetate, the organic phase was washed with saturated saline, dried over anhydrous sodium sulfate, suction-filtered, the crude product was evaporated to dryness, and the crude product was purified by column chromatography (petroleum ether/ethyl acetate=100/0-50/50) to obtain the target compound 2-bromo-6,7-dihydro-5*H-*cyclopenta[*b*]pyridin-7-ol (**I-16-b**) (1.50 g, 66 %) as a white solid. LC-MS: m / z: (M+H)⁺ =214.

### Step 5:

2-Bromo-6,7-dihydro-5*H*-cyclopenta[*b*]pyridin-7-ol (**I-16-e**) (7.0 mmol) was dissolved in dichloromethane (50 mL), and 3-oxo-115-benzo[*d*][1,2]iodoazol-1,1,1(3*H*)-triacetate (14.0 mmol) was added thereto, and the reaction mixture was stirred for 3 hours at room temperature. The reaction mixture was filtered, and the filtrate was evaporated to dryness to obtain a crude product, the crude product was purified by column chromatography (petroleum ether/ethyl acetate = 100/0-50/50) to obtain the target compound 2-bromo-5,6-dihydro-7*H*-cyclopenta[*b*]pyridin-7-one (**I-16-f**) (1.20 g, 81 %) as a white solid. LC-MS: m / z: (M+H)⁺ = 212.

### Step 6:

2-Bromo-5,6-dihydro-7*H*-cyclopenta[*b*]pyridin-7-one **(I-16-f)** (3.54 mmol) was dissolved in anhydrous tetrahydrofuran (10 mL), the reaction mixture was cooled to 0 °C, methylmagnesium bromide (7.07 mmol) was added thereto, and the reaction mixture was stirred for 1 hour at room temperature. The reaction mixture was quenched by adding water, extracted with ethyl acetate, the organic phase was washed with saturated saline, dried over anhydrous sodium sulfate, filtered, the filtrate was evaporated to dryness to obtain a crude product, and the crude product was purified by column chromatography (petroleum ether/ethyl acetate=100/0-50/50) to obtain the target compound 2-bromo-7-methyl-6,7-dihydro-5*H-*cyclopenta[*b*]pyridin-7-ol (**I-16-g**) (320 mg, 39.7 %) as a white solid. LC-MS: m / z: (M+H)⁺ =229.

### Step 7:

2-Allyl-6-(methylthio)-1,2-dihydro-3*H*-pyrazolo[3,4-*d*]pyrimidin-3-one (**I-1-f**) (1.54 mmol) was dissolved in anhydrous 1,4-dioxane (20 mL), and *N*¹,*N*²-dimethylethyl-1,2-diamine (2.81 mmol), cuprous iodide (1.40 mmol), potassium carbonate (1.96 mmol) and 2-bromo-7-methyl-6,7-dihydro-5*H-*cyclopenta[*b*]pyridin-7-ol (**I-16-g**) (1.40 mmol) were added to the reaction mixture. The reaction mixture was heated to 110 °C under the protection of nitrogen, and stirred for 16 hours. The reaction mixture was filtered, and the filtrate was evaporated to obtain a crude product, the crude product was purified by column chromatography (petroleum ether/ethyl acetate = 100/0-50/50) to obtain the target compound 2-allyl-1-(7-hydroxy-7-methyl-6,7-dihydro-5*H*-cyclopenta[*b*]pyridin-2-yl)-6-(methylthio)-1,2-dihydro-3*H-*pyrazolo[3,4-*d*]pyrimidin-3-one (**I-16-h**) (170 mg, 32.8 %) as a white solid. LC-MS: m / z: (M+H)⁺ = 370.

### Step 8:

2-Allyl-1-(7-hydroxy-7-methyl-6,7-dihydro-5*H*-cyclopenta[*b*]pyridin-2-yl)-6-(methylthio)-1,2-dihydro-3*H*-pyrazolo[3,4-*d*]pyrimidin-3-one (**I-16-h**) (0.23 mmol) was dissolved in toluene (15 mL), 3-chlorophenoxyformic acid (0.25 mmol) was added thereto, and the reaction mixture was stirred at room temperature for 0.5 hours, then the reaction mixture was evaporated to dryness, the obtained sulfoxide intermediate was dissolved in dimethyl sulfoxide (10 mL); 4-(4-methylpiperazin-1-yl)aniline (**I-16-f**) (0.25 mmol) and trifluoroacetic acid (0.3 mL) were added thereto, and the reaction mixture was heated to 60 °C and stirred for 16 hours. The pH value of the reaction mixture was adjusted to 9 with saturated sodium carbonate solution, water (50 mL) and ethyl acetate (50 mL) were added thereto, and the phases were separated, the organic phase was washed with saturated saline, dried over anhydrous sodium sulfate, filtered, evaporated to dryness, and prepared and purified by liquid phase to obtain 2-allyl-1-(7-hydroxy-7-methyl-6,7-dihydro-5*H*-cyclopenta[*b*]pyridin-2-yl)-6-((4-(4-methylpiperazin-1-yl)phenyl)amino)-1,2-dihydro-3*H-*pyrazolo[3,4-*d*]pyrimidin-3-one (1-16) as a white solid (25 mg, 21.2 %). ¹H NMR (400 MHz, DMSO) δ 10.11 (s, 1H), 8.83 (s, 1H), 8.27 (s, 2H), 7.94 (d, J = 7.3 Hz, 1H), 7.77-7.53 (m, 3H), 6.92 (d, J = 8.9 Hz, 2H), 5.76-5.63 (m, 1H), 5.00 (d, J = 10.2 Hz, 1H), 4.87 (d, J = 17.1 Hz,1H), 4.81-4.54 (m, 3H), 3.11 (s, 4H), 3.04-2.92 (m, 1H), 2.81 (dd, J = 15.1, 8.0 Hz, 1H), 2.48 (s, 4H),2.24 (s, 3H), 2.14 (t, J = 6.9 Hz, 2H), 2.01 (dd, J = 15.3, 7.7 Hz, 1H), 1.46 (s, 3H). LC- MS: m / z: (M+H)⁺ =513.

### Embodiment 17

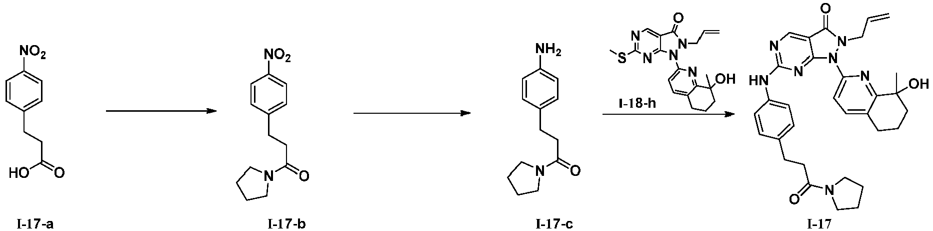

### Step 1:

3-(4-Nitrophenyl)propionic acid (300 mg, 1.54 mmol) and pyrrolidine (I-17-a) (131 mg, 1.84 mmol) were dissolved in 5 mL of dichloromethane, and a solution of propylphosphonic anhydride (1.47 g, 50%, 2.31 mmol) was added thereto, and the reaction was carried out at 15 °C for 1 hour. The reaction mixture was concentrated, and purified by passing through a column (methanol/dichloromethane = 0-10%) to obtain a white solid (I-17-b) (300 mg, 78.6 %). LC-MS: m / z: (M+H)⁺ = 249.

### Step 2:

3-(4-Nitrophenyl)-1-pyrrolidin-1-yl-propan-1-one (I-17-b) (50 mg, 0.20 mmol) was dissolved in 10 mL of methanol, 10% Pd/C (30 mg) was added thereto, and the reaction mixture was stirred at 15 °C with hydrogen (15 psi) for 2 hours. The residue was filtered and concentrated to obtain a white solid (I-17-c) (40 mg, 91%). LC-MS: m / z: (M+H)⁺=219.

### Step 3:

2-Allyl-1-(8 -hydroxy-8-methyl-6,7-dihydro-5*H*-quinolin-2-yl)-6-methylthio-pyrazolo[3,4-*d*]pyrimidin-3-one (**I-18-h**) (40, 0.10 mmol) was dissolved in 10 mL of toluene, and 3-chloroperoxybenzoic acid (32 mg, 0.14 mmol ) was added thereto, and the reaction mixture was stirred at 20 °C for 2 hours. Diisopropylethylamine (30 mg, 0.23 mmol) and 3-(4-aminophenyl)-1-pyrrolidin-1-ylpropan-1-one (I-17-c) (27 mg, 0.13 mmol) were added, and the reaction mixture was stirred at 20 °C for 24 hours. The reaction mixture was diluted with dichloromethane (50 mL), washed with saturated saline, dried over anhydrous sodium sulfate, filtered and concentrated, and prepared by TLC (dichloromethane/methanol=10/1) to obtain 2-allyl-1-(8-hydroxy-8-methyl-5,6,7,8-tetrahydroquinolin-2-yl)-6-((4-(3-oxo-3-(pyrrolidin-1-yl)propyl)phenyl)amino)-1,2-dihydro-3*H*-pyrazolo[3,4-*d*]pyrimidin-3-one (**I-17**) (28.55 mg, 49 %) as a yellow solid. ¹H NMR(400 MHz, CDCl₃) δ1H NMR (400 MHz, MeOD) δ 8.83 (s, 1H), 7.74 (dd, *J* = 18.0, 8.3 Hz, 2H), 7.61 (d, *J* = 8.5 Hz, 2H), 7.20(d, *J* = 8.6 Hz, 2H), 5.81 - 5.66 (m, 1H), 5.05 (dd, *J* = 10.2, 1.1 Hz, 1H), 4.94 (ddd, *J* = 14.9, 10.8, 3.6 Hz,2H), 4.81 - 4.68 (m, 1H), 3.39 (dd, *J* = 11.5, 4.8 Hz, 2H), 3.30 (s, 3H), 2.91 (dt, *J* = 10.7, 7.3 Hz, 4H), 2.63(t, *J* = 7.5 Hz, 2H), 2.14 - 1.93 (m, 3H), 1.92 - 1.75 (m, 5H), 1.62 (d, *J* = 8.4 Hz, 3H). LC- MS: m / z: (M+H)⁺=554.

### Embodiment 18

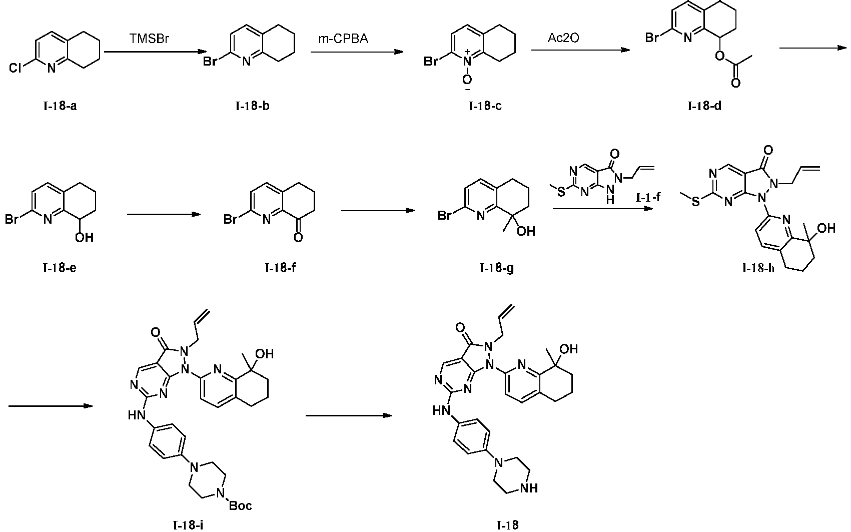

### Step 1:

2-Chloro-5,6,7,8-tetrahydroquinoline (2 g, 11.93 mmol) (**I-18-a**) was dissolved in 40 mL of acetonitrile, and bromotrimethylsilane (7.3 g, 43.7 mmol) was added thereto, and the reaction mixture was stirred under microwave at 150 °C for 2 hours. The reaction mixture was concentrated, and the residue was diluted with dichloromethane (30 mL) and washed with saturated sodium bicarbonate aqueous solution (20 mL×3). The organic layer was dried over anhydrous sodium sulfate, filtered and concentrated to obtain a crude product, the crude product was purified by passing through a column (ethyl acetate / petroleum ether = 0-100%) to obtain the product 2-bromo-5,6,7,8-tetrahydroquinoline (**I-18-b**) (2.4 g, 95 %) as a colorless oil. LC-MS: m / z: (M+H)⁺=554.

### Step 2:

2-Bromo-5,6,7,8-tetrahydroquinoline (I-18-b) (2.4 g, 11 mmol) was dissolved in 20 mL of dichloromethane, and 3-chloroperoxybenzoic acid (3.3 g, 70%, 14 mmol) was added thereto, and the reaction mixture was stirred at 15 °C for 16 hours. The reaction mixture was quenched with saturated sodium bicarbonate aqueous solution and extracted with dichloromethane (30 mL×2). The organic layer was dried over anhydrous sodium sulfate, filtered and concentrated to obtain a crude product, the crude product was purified by passing through a column (ethyl acetate / petroleum ether = 0-50%) to obtain the product 2-bromo-5,6,7,8-tetrahydroquinoline 1-oxide (**I-18-c**) (2.36 g, 91 %) as a white solid. LC-MS: m / z: (M+H)⁺=228.

### Step 3:

2-Bromo-5,6,7,8-tetrahydroquinoline 1-oxide (**I-18-c**) (2.36 g, 10.3 mmol) was dissolved in 50 mL of acetic anhydride, and the reaction mixture was stirred at 110 °C for 16 hours. The reaction mixture was concentrated and purified by passing through a column (ethyl acetate / petroleum ether = 0-50%) to obtain the product 2-bromo-5,6,7,8-tetrahydroquinolin-8-yl acetate (**I-18-d**) (2.2 g, 79 %) as a colorless oil. LC-MS: m / z: (M+H)⁺=270.

### Step 4:

2-Bromo-5,6,7,8-tetrahydroquinolin-8-yl acetate (**I-18-d**) (2.2 g, 8.17 mmol) was dissolved in 20 mL of ethanol, and a solution of KOH (500 mg, 8.93 mmol) dissolved in 50 mL of ethanol was added, and the reaction mixture was stirred at 15 °C for 3 hours. The reaction mixture was concentrated, 50 mL of water was added to the residue, and the mixture was extracted with dichloromethane (50 mL×2), the organic layer was dried over anhydrous sodium sulfate, filtered and concentrated to obtain colorless oily product 2-bromo-5,6,7,8-tetrahydroquinolin-8-ol (**I-18-e**) (1.6 g, 86 %). LC-MS: m / z: (M+H)⁺=228.

### Step 5:

2-Bromo-5,6,7,8-tetrahydroquinolin-8-ol (I-18-e)(1.6 g, 7.0 mmol) was dissolved in 50 mL of dichloromethane, and Dess-Martin reagent (8 g, 18.86 mmol) was added, and the reaction mixture was stirred at 15 °C for 3 hours. The reaction mixture was washed with saturated sodium bicarbonate aqueous solution and sodium thiosulfate aqueous solution, extracted with dichloromethane (30 mL × 2), the organic layer was dried over anhydrous sodium sulfate, filtered and concentrated to obtain a crude product, the crude product was purified by passing through a column (ethyl acetate / petroleum ether = 0-50%) to obtain 2-bromo-6,7-dihydroquinolin-8(5*H*)-one (**I-18-f**) (760 mg, 59 %) as a white solid. LC-MS: m / z: (M+H)⁺=242.

### Step 6:

2-Bromo-6,7-dihydroquinolin-8(5*H*)-one (**I-18-f**) (1.2 g, 5.3 mmol) was dissolved in 50 mL of tetrahydrofuran, and 3M methylmagnesium bromide (2.6 mL, 7.8 mmol) was added at 0 °C, and after addition the mixture was stirred for 10 min. The reaction mixture was quenched with saturated ammonium chloride aqueous solution, the organic phase was washed with saturated saline, dried over anhydrous sodium sulfate, filtered and concentrated to obtain a crude product, the crude product was purified by passing through a column (ethyl acetate / petroleum ether = 0-50%) to obtain 2-bromo-8-methyl-5,6,7,8-tetrahydroquinolin-8-ol (**I-18-g**) (1.3 g, 82 %) as a white solid. LC-MS: m / z: (M+H)⁺= 226.

### Step 7:

A mixture of 2-bromo-8-methyl-5,6,7,8-tetrahydroquinolin-8-ol **(I-18-g)** (750 mg, 3.10 mmol), mixture of 2-allyl-6-methylthio-1H-pyrazolo[1][3,4-d]pyrimidine-3-one (**I-1-f**) (688 mg, 3.10 mmol), cuprous iodide (592 mg, 3.0978 mmol), potassium carbonate (855 mg, 6.1955 mmol) and *N*¹,*N*²-dimethylethane-1,2-diamine (410 mg, 4.65 mmol) were suspended in 20 mL of dioxane, and the mixture was stirred at 95 °C for 16 hours. The reaction mixture was filtered and concentrated to obtain a crude product, the crude product was purified by passing through a column (ethyl acetate / petroleum ether = 0-60%) to obtain 2-allyl-1-(8-hydroxy-8-methyl-5,6,7,8-tetrahydroquinolin-2-yl)-6-(methylthio)-1,2-dihydro-3*H*-pyrazolo[3,4-*d*]pyrimidin-3-one (**I-18-h**) (430 mg, 36.2%) as a white solid. LC-MS: m / z: (M+H)⁺= 384.

### Step 8:

2-Allyl-1-(8-hydroxy-8-methyl-5,6,7,8-tetrahydroquinolin-2-yl)-6-(methylthio)-1,2-dihydro-3*H-*pyrazolo[3,4-*d*]pyrimidin-3-one (**I-18-h**)(40 mg, 0.10 mmol) was dissolved in 10 mL of toluene, and 3-chloroperoxybenzoic acid (30 mg, 0.14 mmol ) was added thereto, and the reaction mixture was stirred at 20 °C for 2 hours. Diisopropylethylamine (30 mg, 0.23 mmol) and *tert*-butyl 4-(4-aminophenyl)piperazine-1-carboxylate (33 mg, 0.12 mmol) were added, and the reaction mixture was stirred at 20 °C for 24 hours. The reaction mixture was concentrated, and prepared by TLC (dichloromethane/methanol = 10/1) to obtain *tert-butyl* 4-(4-((2-allyl-1-(8-hydroxy-8-methyl-5,6,7,8-tetrahydroquinolin-2-yl)-3-oxo-2,3-dihydro-*tert-*butyl-4-1*H*-pyrazolo[3,4-*d*]pyrimidin-6-yl)amino)phenyl)piperazine-1-carboxylate (**I-18-i**) (25 mg, 39.12 %) as a yellow solid. LC-MS: m / z: (M+H)⁺= 613.

### Step 9:

*Tert*-butyl 4-(4-((2-allyl-1-(8-hydroxy-8-methyl-5,6,7,8-tetrahydroquinolin-2-yl)-3-oxo-2,3-dihydro-*tert*-butyl-4-1*H*-pyrazolo[3,4-*d*]pyrimidin-6-yl)amino)phenyl)piperazine-1-carboxylate (**I-18-i**) (25 mg, 0.041 mmol) was dissolved in 3 mL of dichloromethane, and hydrochloric acid/dioxane solution (3 mL, 4 M) was added thereto, then the reaction mixture was stirred at 15 °C for 1 hour. The reaction mixture was concentrated and prepared by HPLC to obtain an off-white solid 2-allyl-1-(8-hydroxy-8-methyl-5,6,7,8-tetrahydroquinolin-2-yl)-6-((4-(piperazin-1-yl)phenyl)amino)-1,2-dihydro-3*H*-pyrazolo[3,4-*d*]pyrimidin-3-one (**I-18**) (19 mg, 94.14 %). ¹H NMR (400 MHz, CDCl₃) δ¹H NMR (400 MHz, MeOD) δ 8.81 (d, J = 4.6 Hz, 1H), 8.45 (s, 1H), 7.80 - 7.70 (m, 2H), 7.62 (d, J = 9.0Hz, 2H), 7.18 - 6.92 (m, 2H), 5.81 - 5.60 (m, 1H), 5.05 (dd, J = 10.2, 1.2 Hz, 1H), 4.99 - 4.91 (m, 2H),4.79 (dd, J = 16.1, 6.3 Hz, 1H), 3.38 (d, J = 10.4 Hz, 8H), 2.99 - 2.79 (m, 2H), 2.18 - 1.93 (m, 3H), 1.92 -1.77 (m, 1H), 1.63 (s, 3H). LC- MS: m / z: (M+H)⁺ =513.

### Embodiment 19

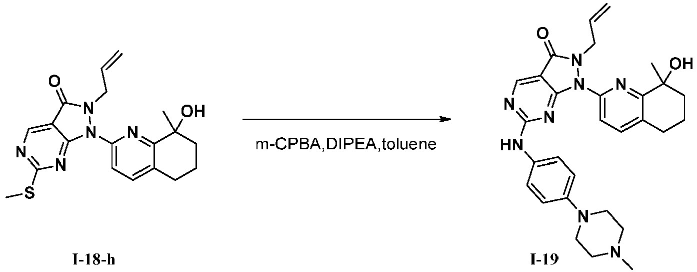

2-Allyl-1-(8 -hydroxy-8 -methyl-5,6,7,8-tetrahydroquinolin-2-yl)-6-(methylthio)-1,2-dihydro-3*H-*pyrazolo[3,4-*d*]pyrimidin-3-one (**I-18-h**) (40 mg, 0.11 mmol) was dissolved in 10 mL of toluene, and 3-chloroperoxybenzoic acid (30 mg, 0.13 mmol) was added thereto, and the reaction mixture was stirred at 20 °C for 2 hours. Diisopropylethylamine (30 mg, 0.23 mmol) and 4-(4-methylpiperazin-1-yl)aniline (30 mg, 0.16 mmol) were added, and the reaction mixture was stirred at 20 °C for 24 hours. The reaction mixture was filtered, concentrated and prepared by TLC (dichloromethane/methanol = 10/1) to obtain 2-allyl-1-(8-hydroxy-8-methyl-5,6,7,8-tetrahydroquinolin-2-yl)-6-((4-(4-methylpiperazin-1-yl)phenyl)amino)-1,2-dihydro-3*H*-pyrazolo[3,4-*d*]pyrimidin-3-one (**I-19**) as a yellow solid (26 mg, 47.34 %). ¹H NMR (400 MHz, CDCl₃) δ¹H NMR (400 MHz, MeOD) δ8.78 (s, 1H), 7.83 - 7.65 (m, 2H), 7.54 (d, *J =* 8.9 Hz, 2H), 6.95 (d, *J* = 9.1Hz, 2H), 5.90 - 5.61 (m, 1H), 5.05 (dd, *J* = 10.2,0.9 Hz, 1H), 4.95 (dd, *J* = 12.2, 6.2 Hz, 2H), 4.79 (dd, *J*= 16.1, 6.3 Hz, 1H), 3.26 - 3.11 (m, 4H), 2.97 - 2.76 (m, 2H), 2.76 - 2.58 (m, 4H), 2.39 (s, 3H), 2.15 -1.92 (m, 3H), 1.92 - 1.76 (m, 1H), 1.61 (d, J= 8.9 Hz, 3H). LC- MS: m / z: (M+H)⁺ =527.

### Embodiment 20 and embodiment 21

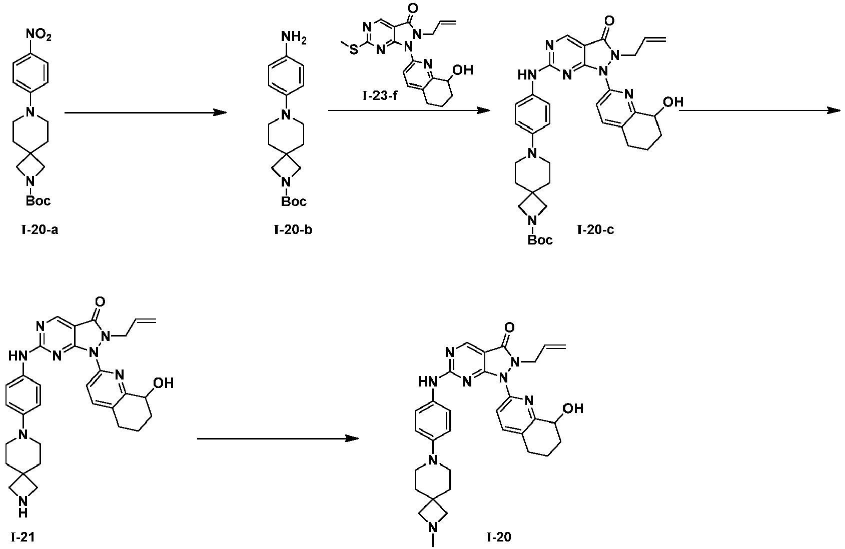

### Step 1:

*Tert*-butyl 7-(4-nitrophenyl)-2,7-diazaspiro[3-4]nonane-2-carboxylate (80 mg, 0.23 mmol) (represented by formula **I-20-a**) and 10% palladium carbon (30 mg) were added to 25 mL of methanol, and the reaction flask was ventilated three times with a hydrogen balloon, and the reaction mixture was stirred for 3 hours at room temperature in a hydrogen atmosphere. The reaction mixture was filtered and evaporated to dryness to obtain 70 mg of *tert*-butyl 7-(4-aminophenyl)-2,7-diazaspiro[3-4]nonane-2-carboxylate (**I-20-b**) as a brown solid, which was directly used in the next step. The yield was 95%. LC-MS: m / z: (M+H)⁺ =318.

### Step 2:

*m*-Chloroperoxybenzoic acid (38mg, 0.17 mmol) was added to a solution of 2-allyl-1-(8-hydroxy-5,6,7,8-tetrahydroquinolin-2-yl)-6-(methylthio)-1,2-dihydro-3*H*-pyrazolo[3,4-*d*]pyrimidin-3-one (53mg, 0.143mmol) (**I-23-f**) in 15 mL of toluene, and the obtained mixture was stirred at room temperature for 2 hours. A mixture of *tert*-butyl 7-(4-aminophenyl)-2,7-diazaspiro[3-4]nonane-2-carboxylate (60 mg, 0.189 mmol) (**I-20-b**) and 0.2 mL of *N*,*N*-diisopropylethylamine in 3 mL of dichloromethane in the above reaction mixture was stirred at room temperature overnight. The reaction mixture was concentrated to obtain a crude product, the crude product was purified by thin layer chromatography {7 M ammonia methanol: (dichloromethane: ethyl acetate = 8: 2)} = 1:13 to obtain 70 mg of methyl 7-(4-((2-allyl-1-(8-hydroxy-5,6,7,8-tetrahydroquinolin-2-yl)-3-oxo-2, 3-dihydro-1H-pyrazolo [3,4-d]pyrimidin-6-yl)amino)phenyl)-2,7-diazapiro[3.5]nonane-2-carboxylate (**I-20-c**) as a yellow solid, yield: 76%. LC-MS: m / z: (M+H)⁺ =639.

### Step 3:

methyl 7-(4-((2-allyl-1-(8-hydroxy-5,6,7,8-tetrahydroquinolin-2-yl)-3-oxo-2, 3-dihydro-1H-pyrazolo [3,4-d]pyrimidin-6-yl)amino)phenyl)-2,7-diazapiro[3.5]nonane-2-carboxylate (**I-20-c**) (70 mg, 0.11 mmol) was added to a mixed solution of 5 mL of dichloromethane and 5 mL of trifluoroacetic acid, and the mixture was stirred at room temperature for 2 hours. The reaction mixture was concentrated, and 30 mL of dichloromethane and 2 mL of methanol were added thereto, the obtained mixture was washed with saturated sodium carbonate solution (10 mL), the organic layer was dried over anhydrous sodium sulfate and purified by thin layer chromatography {7M ammonia methanol:(dichloromethane: ethyl acetate=8:1)}=1:9 to obtain 45 mg of 6-((4-(2,7-diazaspiro[3.5]nonan-7-yl)phenyl)amino)-2-allyl-1-(8-hydroxy-5,6,7,8-tetrahydroquinolin-2-yl)-1,2-dihydro-3*H*-pyrazolo[3,4-*d*]pyrimidin-3-one (**I-21**) as a yellow solid, yield: 76%. ¹H NMR (400 MHz, CDCl₃) δ 8.78 (s, 1H), 7.65 (d, *J* = 8.2 Hz, 1H), 7.58 (d, *J* = 8.3 Hz, 1H), 7.43 (d, *J* = 8.7 Hz, 2H), 6.90 (d, *J* = 9.1 Hz, 2H), 5.73 (ddt, *J* = 16.3, 10.3, 6.0 Hz, 1H), 5.07 (dd, *J* = 10.2, 1.1 Hz, 1H), 5.00 (dd, *J* = 17.1, 1.2 Hz, 1H), 4.73 (t, *J* = 6.5 Hz, 1H), 4.69 - 4.67 (d, *J* = 5.5 Hz, 2H), 3.46 (d, *J* = 8.2 Hz, 4H), 3.13 - 3.05 (m, 4H), 2.94 - 2.78 (m, 2H), 2.35 - 2.20 (m, 1H), 2.06 (m, 1H), 1.99 - 1.78 (m, 6H). LC- MS: m / z: (M+H)⁺ =539.

### Step 4:

6-((4-(2,7-Diazaspiro[3.5]nonan-7-yl)phenyl)amino)-2-allyl-1-(8-hydroxy-5,6,7,8-tetrahydroquinolin-2-yl)-1,2-dihydro-3*H*-pyrazolo[3,4-*d*]pyrimidin-3-one (25 mg, 0.046 mmol) (1-21), 0.1 mL of formaldehyde aqueous solution and sodium borohydride acetate (30 mg, 0.14 mmol) were added to 8 mL of methanol, and the mixture was stirred at room temperature overnight. The reaction mixture was concentrated, and 20 mL of dichloromethane and 1 mL of methanol were added thereto, the obtained mixture was washed with saturated sodium carbonate solution (5 mL), the organic layer was dried over anhydrous sodium sulfate and purified by thin layer chromatography {7M ammonia methanol: (dichloromethane: ethyl acetate=8:2)}=1:10 to obtain 45 mg of 2-allyl-1-(8-hydroxy-5,6,7,8-tetrahydroquinolin-2-yl)-6-((4-(2-methyl-2,7-diazaspiro[3 .5]nonan-7-yl)phenyl)amino)-1,2-dihydro-3*H*-pyrazolo[3,4-*d*]pyrimidin-3 -one (**I-20**) as a yellow solid with a yield of 58 %. ¹H NMR (400 MHz, CDCl₃) δ 8.79 (s, 1H), 7.66 (d, *J* = 8.2 Hz, 1H), 7.59 (d, J= 8.3 Hz, 1H), 7.43 (d, *J* = 8.8 Hz, 2H), 6.90 (t, *J* = 6.2 Hz, 2H), 5.74 (ddt, J= 16.3, 10.3, 6.0 Hz, 1H), 5.08 (dd, *J* = 10.2, 1.0 Hz, 1H), 5.00 (dd, *J* = 17.1, 1.2 Hz, 1H), 4.77 - 4.63 (m, 3H), 3.18 - 3.04 (m, 8H), 2.96 - 2.78 (m, 2H), 2.40 (s, 3H), 2.35 - 2.21 (m, 1H), 2.12 - 2.00 (m, 1H), 1.98 - 1.79 (m, 6H). LC-MS: m / z: (M+H)⁺ =553.

### Embodiment 23

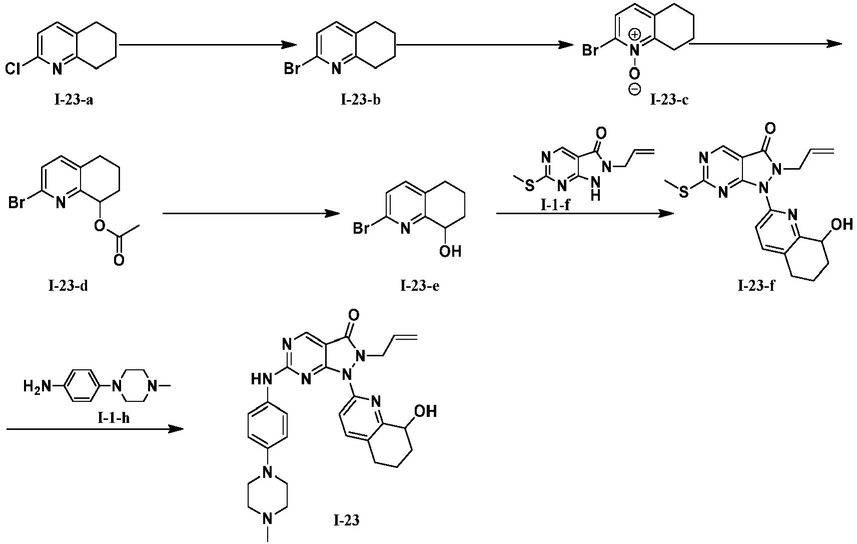

### Step 1:

2-Chloro-5,6,7,8-tetrahydroquinoline (**I-23-a**) (0.71 g, 4.23 mmol) and bromotrimethylsilane (3 g, 19.6 mmol) were added to 15 mL of acetonitrile, and the mixture was heated and stirred at 150 °C for 3 hours under microwave conditions. After the reaction mixture was cooled and concentrated, 10 mL of saturated sodium bicarbonate solution was slowly added until the pH value was about 10; then the mixture was extracted three times with dichloromethane (3^{∗}30 mL), dried over anhydrous sodium sulfate and concentrated to obtain a crude product, the crude product was purified through a column (ethyl acetate: petroleum ether=0-15%) to obtain 0.82 g of 2-bromo-5,6,7,8-tetrahydroquinoline (I-23-b) as a colorless oil, yield: 91 %. LC-MS: m / z: (M+H)⁺=212.

### Step 2:

2-Bromo-5,6,7,8-tetrahydroquinoline (I-23-b) (0.82 g, 3.9 mmol) was added to 25 mL of dichloromethane, then 77 % *m*-chloroperoxybenzoic acid (1.32 g, 5.9 mmol) was also added to 25 mL of dichloromethane, and the mixture was added to a solution of the compound 2, stirred at room temperature overnight. The reaction mixture was washed with saturated sodium bicarbonate solution, and the aqueous phase was extracted with dichloromethane for three times (3^{∗}30 mL), the combined organic layer was dried over anhydrous sodium sulfate and concentrated to obtain a crude product, the crude product was purified by a column (ethyl acetate: petroleum ether =0-80 %) to obtain 0.82 g of 2-bromo-5,6,7,8-tetrahydroquinoline 1-oxide (**I-23-c**) as a white solid, yield: 93%. LC-MS: m / z: (M+H)⁺=228.

### Step 3:

2-Bromo-5,6,7,8-tetrahydroquinoline 1-oxide (**I-23-c**) (0.82 g, 3.6 mmol) was dissolved in 20 mL of acetic anhydride, and the reaction mixture was stirred at 120 °C overnight. The reaction mixture was concentrated, and then 30 mL of dichloromethane was added thereto, the mixture was washed twice with saturated sodium bicarbonate solution (2^{∗}10 mL), then washed once with 10 mL of saturated saline, dried over anhydrous sodium sulfate and concentrated, then the residue was passed through a column (ethyl acetate: petroleum ether=0-30%) to obtain 0.74 g of 2-bromo-5,6,7,8-tetrahydroquinolin-8-yl acetate (**I-23-d**) as a colorless oil, yield: 76 %. LC-MS: m / z: (M+H)⁺=270.

### Step 4:

2-Bromo-5,6,7,8-tetrahydroquinolin-8-yl acetate (**I-23-d**) (0.74 g, 2.7 mmol) was added to 20 mL of ethanol, then 20 mL of a solution of potassium hydroxide (154 mg, 2.75 mmol) in ethanol was added thereto. The mixture was stirred at room temperature for 3 hours. 25 mL of water was added to the reaction mixture, most of the ethanol was evaporated; then the mixture was extracted with dichloromethane (3^{∗}25 mL), washed with 20 mL of saturated saline, dried over anhydrous sodium sulfate and concentrated, then the residue was passed through a column (ethyl acetate: petroleum ether=0-50%) to obtain 0.58 g of 2-bromo-5,6,7,8-tetrahydroquinolin-8-ol (**I-23-e**) as a colorless oil, yield: 93%. ¹H NMR (400 MHz, CDCl₃) δ 7.31 (t, *J* = 5.1 Hz, 1H), 4.71 (t, *J* = 6.6 Hz, 0H), 3.55 (s, 0H), 2.87 - 2.68 (m, 2H), 2.32 - 2.18 (m, 1H), 2.09 - 1.95 (m, 1H), 1.90 - 1.74 (m, 2H). LC- MS: m / z: (M+H)⁺ =227.

### Step 5:

2-Bromo-5,6,7,8-tetrahydroquinolin-8-ol (**I-23-e**) (0.58 g, 2.54 mmol), 2-allyl-6-(methylthio)-1,2-dihydro-3*H*-pyrazolo[3,4-*d*]pyrimidin-3-one (0.58 g, 2.61 mmol) (I-1-f), cuprous iodide (0.5 g, 2.6 mmol), potassium carbonate (0.72 g, 5.22 mmol) and *N*¹,*N*²-dimethylethylenediamine (0.46 g, 5.2 mmol) were added to 20 mL of 1,4-dioxane, and the mixture was stirred overnight at 100 °C under the protection of argon. The reaction product was cooled and concentrated to obtain a crude product, then the crude product was purified by passing through a column (methanol: dichloromethane=0-10%) to obtain 0.50 g of 2-allyl-1-(8-hydroxy-5,6,7,8-tetrahydroquinolin-2-yl)-6-(methylthio)-1,2-dihydro-3*H*-pyrazolo[3,4-*d*]pyrimidin-3-one (**I-23-f**) as a white solid, yield: 50 %. LC-MS: m / z: (M+H)⁺=370.

### Step 6:

*m*-Chloroperoxybenzoic acid (38mg, 0.17 mmol) was added to a solution of 2-allyl-1-(8-hydroxy-5,6,7,8-tetrahydroquinolin-2-yl)-6-(methylthio)-1,2-dihydro-3*H*-pyrazolo[3,4-*d*]pyrimidin-3-one (53mg, 0.14mmol (**I-23-f**) in 20 mL of toluene, and the obtained mixture was stirred at room temperature for 2 hours. 4-(4-Methylpiperazin-1-yl)aniline (35mg, 0.18 mmol) (**I-1-h**) and 0.2 mL of *N*, *N*-diisopropylethylamine were added to the above reaction mixture, and the mixture was stirred overnight at room temperature. The reaction mixture was concentrated to obtain a crude product, and the crude product was purified by passing through a column (methanol: dichloromethane = 0-20%), and then prepared by high performance liquid chromatography to obtain 34 mg of 2-allyl-1-(8-hydroxy-5,6,7,8-tetrahydroquinolin-2-yl)-6-((4-(4-methylpiperazin-1-yl(phenyl)amino)-1,2-dihydro-3*H*-pyrazolo[3,4-*d*]pyrimidin-3-one (**I-23**) as a yellow solid, yield: 34 %. ¹H NMR (400 MHz, CDCl₃) δ 8.78 (s, 1H), 8.04 (s, 1H), 7.64 (d, *J* = 8.2 Hz, 1H), 7.58 (t, *J* = 8.1 Hz, 1H), 7.43 (d, *J* = 8.8 Hz, 2H), 6.89 (d, *J* = 9.0 Hz, 2H), 5.72 (ddt, J= 16.4, 10.3, 6.0 Hz, 1H), 5.06 (dd, J= 10.2, 0.9 Hz, 1H), 4.99 (dd, *J* = 17.1, 1.2 Hz, 1H), 4.72 (t, *J* = 6.5 Hz, 1H), 4.67 (d, *J* = 5.9 Hz, 2H), 3.90 (s, 1H), 3.24 - 3.13 (m, 4H), 2.94 - 2.75 (m, 2H), 2.67 - 2.55 (m, 4H), 2.36 (s, 3H), 2.33 - 2.24 (m, 1H), 2.06 - 2.03 (m, 1H), 1.91 - 1.77 (m, 2H). LC- MS: m / z: (M+H)⁺ =513.

### Embodiment 28

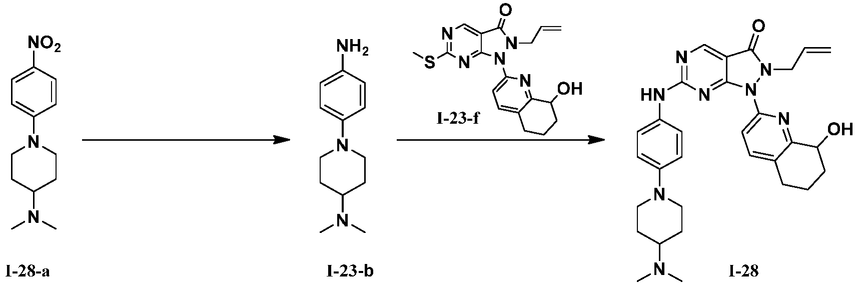

### Step 1:

*N*,*N*-dimethyl-1-(4-nitrophenyl)piperidin-4-amine (60 mg, 0.24 mmol) (I-28-a) and 10% palladium carbon (30 mg) were added to 25 mL of methanol, and the reaction flask was ventilated three times with a hydrogen balloon, and the reaction mixture was stirred for 3 hours at room temperature in a hydrogen atmosphere. The reaction mixture was filtered and evaporated to dryness to obtain 50 mg of 1-(4-aminophenyl)-*N*,*N*-dimethylpiperidin-4-amine (**I-28-b**) as a brown solid, which was directly used in the next step. The yield was 95%. LC-MS: m / z: (M+H)⁺ =220.

### Step 2:

*m*-Chloroperoxybenzoic acid (30mg, 0.134 mmol) was added to a solution of 2-allyl-1-(8-hydroxy-5,6,7,8-tetrahydroquinolin-2-yl)-6-(methylthio)-1,2-dihydro-3*H*-pyrazolo[3,4-*d*]pyrimidin-3-one (40mg, 0.108mmol) (**I-23-f**) in 15 mL of toluene, and the obtained mixture was stirred at room temperature for 2 hours. 1-(4-Aminophenyl)-*N*,*N*-dimethylpiperidin-4-amine (50 mg, 0.228 mmol) (I-28-b) and 0.2 mL of *N*,*N*-diisopropylethylamine were added to the above reaction mixture, and the mixture was stirred overnight at room temperature. The reaction mixture was concentrated to obtain a crude product, the crude product was prepared by high performance liquid chromatography to obtain 25 mg of 2-allyl-6-((4-(4-(dimethylamino)piperidin-1-yl)phenyl)amino)-1-(8-hydroxy-5,6,7,8-tetrahydroquinolin-2-yl)-1,2-dihydro-3*H*-pyrazolo[3,4-*d*]pyrimidin-3-one formate (1-28) as a yellow solid with a yield of 42 %. ¹H NMR (400 MHz, CDCl₃) δ 8.78 (s, 1H), 8.52 (s, 1H), 7.61 (q, *J* = 8.3 Hz, 2H), 7.46 (d, J= 8.3 Hz, 2H), 6.88 (d, *J* = 8.8 Hz, 2H), 5.78 - 5.65 (m, 1H), 5.07 (d, *J* = 10.2 Hz, 1H), 4.98 (d, *J* = 17.1 Hz, 1H), 4.72 (t, *J* = 7.3 Hz, 1H), 4.66 (d, *J* = 5.4 Hz, 2H), 3.72 (d, *J* = 12.0 Hz, 2H), 3.06 (t, *J* = 11.7 Hz, 1H), 2.95 - 2.81 (m, 2H), 2.75 (t, *J* = 11.9 Hz, 2H), 2.66 (s, 6H), 2.30 (m, 1H), 2.18 - 1.98 (m, 3H), 1.85 (m, 4H). LC- MS: m / z: (M+H)⁺ =541.

### Embodiment 32 and embodiment 33

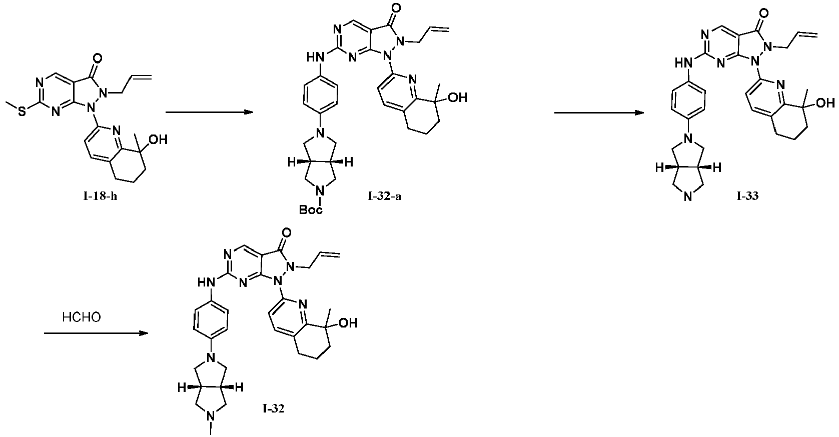

### Step 1:

2-Allyl-1-(8-hydroxy-8-methyl-6,7-dihydro-5*H*-quinolin-2-yl)-6-methylthio-pyrazolo[3,4-*d*]pyrimidin-3-one (40 mg, 0.104 mmol) (**I-18-h**) was dissolved in 10 mL of toluene, and 3-chloroperoxybenzoic acid (30 mg, 0.13 mmol ) was added thereto, and the reaction mixture was stirred at 20 °C for 2 hours. Diisopropylethylamine (30 mg, 0.23 mmol) and *tert-butyl* (3a*R*, 6a*S*)-2-(4-aminophenyl)-1,3,3a,4,6,6a-hexahydropyrrolo[3,4-*c*]pyrrole-5-carboxylate (30 mg, 0.10 mmol) were added, and the reaction mixture was stirred at 20 °C for 24 hours. The reaction mixture was filtered, concentrated and prepared by TLC (dichloromethane/methanol = 10/1) to obtain *tert*-butyl (3a*R*,6a*S*)-5-(4-((2-allyl-1-(8-hydroxy-8-methyl-5,6,7,8-tetrahydroquinolin-2-yl)-3-oxo-2,3-dihydro-1*H*-pyrazolo[3,4-*d*]pyrimidin-6-yl)amino)phenyl)hexahydropyrrolo[3,4-*c*]pyrrole-2(1*H*)-carboxylate (I-32-a) (35 mg, 52.53 %) as a yellow solid. LC-MS: m / z: (M+H)⁺ =639.

### Step 2:

*Tert*-butyl (3aR, 6a*S*)-5-(4-((2-allyl-1-(8-hydroxy-8-methyl-5,6,7,8-tetrahydroquinolin-2-yl)-3-oxo-2,3-dihydro-1*H*-pyrazolo[3,4-*d*]pyrimidin-6-yl)amino)phenyl)hexahydropyrrolo[3,4-*c*]pyrrole-2(1*H*)-carboxylate (I-32-a) (35 mg, 0.055 mmol) was dissolved in 3 mL of dichloromethane, and 6 mL of a 4 M solution of hydrochloric acid in dioxane was added thereto, and the reaction mixture was stirred at 20 °C for 1 hour. The reaction mixture was concentrated to obtain 2-allyl-6-((4-((3a*R*, 6a*S*)hexahydropyrrolo[3,4-*c*]pyrrolo-2(1*H*)-yl)phenyl)amino)-1-(8-hydroxy-8-methyl-5,6,7,8-tetrahydroquinolin-2-yl)-1,2-dihydro-3*H*-pyrazolo[3,4-*d*]pyrimidin-3-one (1-33) (30 mg, 95.21 %) as a yellow-brown solid. ¹H NMR (400 MHz, CDCl₃) δ¹H NMR (400 MHz, MeOD) δ 8.76 (s, 1H), 7.71 (s, 2H), 7.50 (d, J= 7.9 Hz, 2H), 6.75 (d, *J* = 8.7 Hz,2H), 5.89 - 5.56 (m, 1H), 5.05 (d, *J* = 10.1 Hz, 1H), 4.96 (s, 1H), 4.80 (d, *J* = 6.1 Hz, 2H), 3.61 (s, 2H),3.49 (d, *J* = 7.6 Hz, 2H), 3.26 (s, 5H), 3.24 (s, 1H), 2.89 (d, *J* = 6.3 Hz, 2H),2.16 - 1.95 (m, 4H), 1.86 (d, *J* = 6.3 Hz, 1H), 1.63 (s, 3H). LC- MS: m / z: (M+H)⁺ =539.

### Step 3:

2-Allyl-6-((4-((3a*R*,6a*S*)hexahydropyrrolo[3,4-c]pyrrolo-2(1*H*)-yl)phenyl)amino)-1-(8-hydroxy-8-methyl-5,6,7,8-tetrahydroquinolin-2-yl)-1,2-dihydro-3*H*-pyrazolo[3,4-*d*]pyrimidin-3-one (1-33) (30 mg, 0.052 mmol) was dissolved in 2 mL of methanol, and 37 % formaldehyde aqueous solution (21 mg, 0.26 mmol) was added thereto, and the reaction mixture was stirred at 20 °C for 2 hours. Sodium triacetoxyborohydride (22 mg, 0.10 mmol) was added, and the reaction mixture was stirred at 20 °C for 24 hours. The reaction mixture was concentrated and prepared by TLC (dichloromethane/methanol = 10/1) to obtain 20 mg of a yellow solid, the solid was then prepared in liquid phase to obtain 2-allyl-1-(8-hydroxy-8-methyl-5,6,7,8-tetrahydroquinolin-2-yl)-6-((4-((3a*R*,6a*S*)-5-methylhexahydropyrrolo[3,4-*c*]pyrrole-2(1*H*)-yl)phenyl)amino)-1,2-dihydro-3*H*-pyrazolo[3,4-*d*]pyrimidin-3-one (1-32) (8.5 mg, 29%) as a yellow solid. ¹H NMR (400 MHz, CDCl₃) δ¹H NMR (400 MHz, MeOD) δ8.78 (s, 1H), 7.72 (s, 2H), 7.52 (d, *J* = 8.2 Hz, 2H), 6.79 (d, *J* = 8.9 Hz,2H), 5.73 (ddd, *J* = 16.7, 10.2, 6.0 Hz, 1H), 5.05 (d, *J* = 9.3 Hz, 1H), 4.99 - 4.92 (m, 2H), 4.79 (dd, *J* = 16.1, 6.3 Hz, 1H), 3.53 - 3.38 (m, 4H), 3.18 (d, *J* = 7.1 Hz, 4H), 3.03 (d, *J* = 10.4 Hz, 2H), 2.96 - 2.83 (m,2H), 2.74 (s, 3H), 2.13 - 1.97 (m, 4H), 1.93 - 1.80 (m, 1H), 1.63 (s, 3H). LC- MS: m / z: (M+H)⁺ =553.

### Embodiment 34

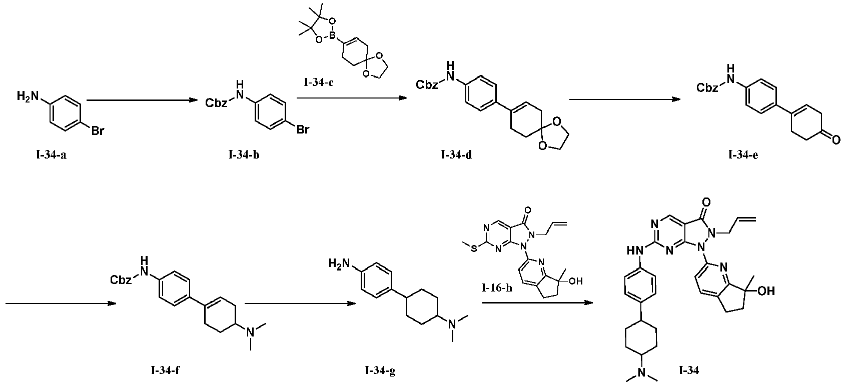

### Step 1:

4-Bromoaniline (I-34-a) (58.1 mmol) was dissolved in toluene (250 mL), potassium carbonate (87.2 mmol) and benzyl chloroformate (87.2 mmol) were added to the reaction mixture, and the reaction mixture was stirred for 16 hours under the protection of nitrogen. The reaction mixture was filtered, the filtrate was evaporated to dryness to obtain a crude product, and the crude product was washed with a small amount of ethyl acetate to obtain 15.2 g of the target compound benzyl (4-bromophenyl)carbamate (I-34-b) as a white solid with a yield of 85.4 %. LC-MS: m / z: (M+H)⁺ =306.

### Step 2:

Benzyl (4-bromophenyl)carbamate (I-34-b) (16.0 mmol) was dissolved in 1, 2-dimethoxyethane (50 mL), and 4,4,5,5-tetramethyl-2-(1,4-dioxaspiro[4.5]dec-7-en-8-yl)-1,3,2-dioxaborolane (represented by formula I-34-c) (16.0 mmol), sodium carbonate (42.0 mmol) and tetrakis(triphenylphosphine)palladium (1.6 mmol) were added to the reaction mixture, the reaction mixture was heated to 80 °C and stirred for 16 hours. The reaction mixture was filtered, the filtrate was evaporated to dryness to obtain a crude product, and the crude product was purified by column chromatography (dichloromethane/methanol=100/0-95/5) to obtain 5.6 g of the target compound benzyl (4-(1,4-dioxaspiro[4.5]dec-7-en-8-yl)phenyl)carbamate (**I-34-d**) as a white solid with a yield of 94 %. LC-MS: m / z: (M+H)⁺ =367.

### Step 3:

Benzyl (4-(1,4-dioxaspiro[4.5]dec-7-en-8-yl)phenyl)carbamate (I-34-d) (15.0 mmol) was dissolved in tetrahydrofuran (30 mL). Hydrochloric acid (20 mL, 4mmol/L) was added to the reaction mixture, and the reaction mixture was heated to 45 °C and stirred for 16 hours. The pH value of the reaction mixture was adjusted to 9 with potassium carbonate, extracted with dichloromethane, the organic phase was washed with saline, dried over anhydrous sodium sulfate, filtered and evaporated to dryness to obtain 4.0 g of the target compound benzyl (4'-oxo-2',3',4',5'-tetrahydro-[1,1'-biphenyl]-4-yl)carbamate (I-34-e) as a yellow solid with a yield of 81 %. LC-MS: m / z: (M+H)⁺ =322.

### Step 4:

Benzyl (4'-oxo-2',3',4',5'-tetrahydro-[1,1'-biphenyl]-4-yl)carbamate (**I-34-e**) (12.0 mmol) was dissolved in dichloromethane (25 mL), dimethylamine hydrochloride (25.0 mmol), diisopropylethylamine (25.0 mmol) and sodium triacetoxyborohydride (37.0 mmol) were added to the reaction mixture. The reaction mixture was stirred at room temperature for 16 hours, the reaction mixture was evaporated to dryness to obtain a crude product, the crude product was purified by column chromatography (dichloromethane/methanol=100/0-95/5) to obtain 3.5 g of the target compound benzyl (4'-(dimethylamino)-2',3',4',5'-tetrahydro-[1,1'-biphenyl]-4-yl)carbamate (I-34-f) as a yellow solid with a yield of 80 %. LC-MS: m / z: (M+H)⁺ =351.

### Step 5:

Benzyl (4'-(dimethylamino)-2',3',4',5'-tetrahydro-[1,1'-biphenyl]-4-yl)carbamate (I-34-f) (10.0 mmol) was dissolved in methanol (20 mL), palladium carbon (350 mg) was added thereto, and the reaction mixture was stirred under hydrogen at room temperature for 16 hours; the reaction mixture was filtered and the filtrate was evaporated to dryness to obtain a crude product, the crude product was washed with a small amount of ethyl acetate to obtain 1.2 g of the target compound 4-(4-(dimethylamino)cyclohexyl)aniline (**I-34-g**) as a white solid with a yield of 55 %. LC-MS: m / z: (M+H)⁺ =219.

### Step 6:

2-Allyl-1-(7-hydroxy-7-methyl-6,7-dihydro-5*H*-cyclopenta[*b*]pyridin-2-yl)-6-(methylthio)-1,2-dihydro-3*H*-pyrazolo[3,4-*d*]pyrimidin-3-one (**I-16-h**) (0.14 mmol) was dissolved in toluene (10 mL), 3-chlorophenoxyformic acid (0.15 mmol) was added thereto, and the reaction mixture was stirred at room temperature for 0.5 hours, then the reaction mixture was evaporated to dryness, the obtained sulfoxide intermediate was dissolved in dimethyl sulfoxide (10 mL); 4-(4-(dimethylamino)cyclohexyl) aniline (1-34-g) (0.15 mmol) and trifluoroacetic acid (0.3 mL) were added thereto, and the reaction mixture was heated to 60 °C and stirred for 16 hours. The pH of the reaction mixture was adjusted to 9 with saturated sodium carbonate solution, water (50 mL) and ethyl acetate (50 mL) were added thereto, and the phases were separated, the organic phase was washed with saturated saline, dried over anhydrous sodium sulfate, filtered, evaporated to dryness, and prepared and purified by liquid phase to obtain 25 mg of 2-allyl-6-((4-(4-(dimethylamino)cyclohexyl)phenyl)amino)-1-(7-hydroxy-7-methyl-6,7-dihydro-5*H*-cyclopenta[*b*]pyridin-2-yl)-1,2-dihydro-3*H*-pyrazolo[3,4-*d*]pyrimidin-3-one (**I-34**) as a white solid with a yield of 34.2 %. ¹H NMR (400 MHz, CDCl₃) δ 8.86 (s, 0H), 7.72 (dd, J = 24.0, 8.0 Hz, 1H), 7.54 (d, J = 7.8 Hz, 3H), 7.18(d, J = 8.2 Hz, 1H), 5.72 (d, J = 6.4 Hz, 0H), 5.37 (s, 0H), 4.99 (dd, J = 39.8, 13.5 Hz, 1H), 4.79 (d, J =23.4 Hz, 1H), 3.07 (s, 0H), 2.91 (d, J = 7.6 Hz, 0H), 2.54 (s, 2H), 2.36 (d, J = 5.9 Hz, 1H), 2.20 (s, 1H),2.04 (s, 1H), 1.64 (s, 1H), 1.55 (s, 1H). LC- MS: m / z: (M+H)⁺ =540.

### Embodiment 35

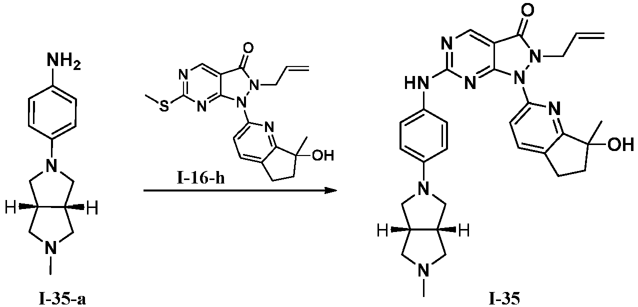

2-Allyl-1-(7-hydroxy-7-methyl-6,7-dihydro-5*H*-cyclopenta[*b*]pyridin-2-yl)-6-(methylthio)-1,2-dihydro-3*H*-pyrazolo[3,4-*d*]pyrimidin-3-one (I-16-h) (0.14 mmol) was dissolved in toluene (10 mL), 3-chlorophenoxyformic acid (0.15 mmol) was added thereto, and the reaction mixture was stirred at room temperature for 0.5 hours, then the reaction mixture was evaporated to dryness, the obtained sulfoxide intermediate was dissolved in dimethyl sulfoxide (10 mL); 4-(-5-methylhexahydropyrrolo[3,4-c]pyrrol-2(1*H*)-yl)aniline (I-35-a) (0.15 mmol) and trifluoroacetic acid (0.3 mL) were added thereto, and the reaction mixture was heated to 60 °C and stirred for 16 hours. The pH of the reaction mixture was adjusted to 9 with saturated sodium carbonate solution, water (50 mL) and ethyl acetate (50 mL) were added thereto, and the phases were separated, the organic phase was washed with saturated saline, dried over anhydrous sodium sulfate, filtered, evaporated to dryness, and prepared and purified by liquid phase to obtain 12.5 mg of 2-allyl-1-(7-hydroxy-7-methyl-6,7-dihydro-5*H*-cyclopenta[*b*]pyridin-2-yl)-6-((4-(-5-methylhexahydropyrrolo[3,4-*c*]pyrrol-2(1*H*)-yl)phenyl)amino)-1,2-dihydro-3*H*-pyrazolo[3,4-*d*]pyrimidin-3-one (1-35) as a yellow solid with a yield of 17.1 %. ¹H NMR (400 MHz, CDCl₃) δ 8.74 (s, 1H), 8.44 (s, 1H), 7.68 (dd, J = 23.7, 8.0 Hz, 2H), 7.41 (d, J = 7.8Hz, 2H), 6.64 (d, J = 8.1 Hz, 2H), 5.75-5.63 (m, 1H), 5.25 (s, 1H), 4.97 (dd, J = 39.0, 13.6 Hz, 2H), 4.85-4.63 (m, 2H), 3.67 (s, 2H), 3.39 (d, J = 9.3 Hz, 2H), 3.30-3.00 (m, 5H), 2.96-2.62 (m, 6H), 2.42-2.24 (m, 2H), 1.63 (s, 3H). LC-MS: m / z: (M+H)⁺ =539.

### Embodiment 38

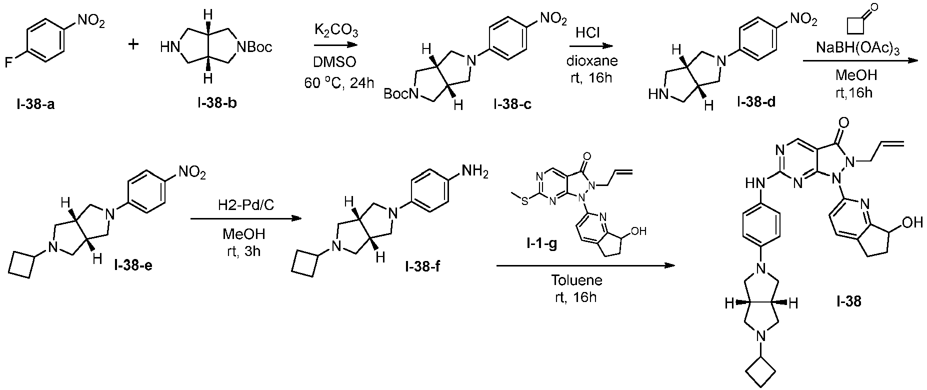

### Step 1:

*p*-Fluoronitrobenzene (represented by formula **I-38-a**) (1.0 g, 7.09 mmol) was dissolved in 20 mL of DMSO, and then *tert-butyl* (3aR, 6a*S*)-hexahydropyrrolo[3,4-*c*]pyrrole-2(1*H*)-carboxylate (**I-38-b**) (1.5 g, 7.09 mmol) and potassium carbonate (2.9 g, 21 mmol) were added thereto, and the mixture was stirred at 60 °C for 24 hours. After the reaction, 30 mL of water was added, and the mixture was extracted twice with 30 mL of ethyl acetate each time. The organic phases were combined, washed with 30 mL of saturated saline, dried over anhydrous sodium sulfate and concentrated, and the crude product was directly used for the next step. A yellow solid (I-38-c) (2.34 g, 7.02 mmol) was obtained with a yield of 99 %.

### Step 2:

*Tert*-butyl (3a*R*, 6a*S*)-5-(4-nitrophenyl)hexahydropyrrolo[3,4-c]pyrrole-2(1*H*)-carboxylate (1-38-c) (2.34 g, 7.02 mmol) was dissolved in 20 mL of dichloromethane, and 0.5 mL of a 4N solution of hydrogen chloride in dioxane was added, and the mixture was stirred for 16 hours at room temperature. Then the mixture was concentrated under reduced pressure, the obtained crude product was first dissolved with water and then extracted with dichloromethane. The pH value of the aqueous layer was adjusted to 10 with 2N NaOH solution and filtered to obtain a yellow solid (I-38-d) (1.6 g, 6.90 mmol) with a yield of 98 %.

### Step 3:

(3aR, 6a*S*)-2-(4-nitrophenyl)octahydropyrrolo[3,4-*c*]pyrrole (I-38-d) (300 mg, 1.29 mmol) was dissolved in methanol (10 mL), then cyclobutanone (270 mg, 3.86 mmol) and sodium triacetoxyborohydride (1.37 g, 6.45 mmol) were added thereto, and the mixture was stirred for 16 hours at room temperature. The reaction mixture was concentrated, dissolved with ethyl acetate, washed twice with saturated sodium bicarbonate, the organic layer was dried over anhydrous sodium sulfate, filtered and the crude product obtained by concentration was purified by thin layer chromatography to obtain a yellow solid (I-38-e) (240 mg, 0.835 mmol) with a yield of 65 %.

### Step 4:

(3a*R*,6a*S*)-2-cyclobutyl-5-(4-nitrophenyl)octahydropyrrolo[3,4-c]pyrrole (represented by formula I-38-e) (240 mg, 0.835 mmol) and palladium carbon (10 mg) were dissolved in 10 mL of methanol and the reaction was stirred for 3 hours at room temperature under a hydrogen balloon. After the reaction was completed, the mixture was filtered by diatomite to obtain a yellow solid (I-38-f) (176 mg, 0.684 mmol), which was directly used in the next step with a yield of 82 %. LC-MS: m / z: (M+H)⁺=258.1.

### Step 5:

*m*-Chloroperoxybenzoic acid (42 mg, 0.185 mmol) was added to a solution of 2-allyl-1-(7-hydroxy-6,7-dihydro-5*H*-cyclopenta[*b*]pyridin-2-yl)-6-(methylthio)-1,2-dihydro-3*H*-pyrazolo[3,4-*d*]pyrimidin-3-one (**I-1-g**) (60 mg, 0.168 mmol) in 15 mL of toluene, and the obtained solution was stirred at room temperature for 1 hour to obtain a solution of 2-allyl-1-(7-hydroxy-6,7-dihydro-5*H-*cyclopenta[*b*]pyridin-2-yl)-6-(methylsulfinyl)-1,2-dihydro-3*H*-pyrazolo[3,4-*d*]pyrimidin-3-one in toluene. 4-((3aR, 6aS)-5-cyclobutyl hexahydropyrrolo[3, 4-*c*]pyrrol-2(1*H*)-yl)aniline (**I-38-f**) (48 mg, 0.185 mmol) and 0.1 mL of *N*, *N*-diisopropylethylamine were added to the obtained solution, and the mixture was stirred at room temperature for 16 hours. The mixture was concentrated under reduced pressure, the obtained crude product was separated and purified by thin layer chromatography (7M ammonia methanol: dichloromethane = 0-10%) to obtain 21 mg of the target compound as a yellow solid (**I-38**) with a yield of 21 %. ¹H NMR (400 MHz, CDCl₃) δ 8.80 (s, 1H), 7.74 (d, J = 8.2 Hz, 1H), 7.66 (d, J = 7.9 Hz, 1H), 7.41 (d, J = 7.5 Hz, 2H), 6.65 (d, J = 8.9 Hz, 2H), 5.81 - 5.64 (m, 1H), 5.28 - 5.20 (m, 1H), 5.08 - 4.92 (m, 2H), 4.71 (s, 1H), 3.28 (s, 4H), 3.11 (ddd, J = 16.0, 8.8, 3.7 Hz, 3H), 2.91 (dt, J = 15.9, 7.8 Hz, 1H), 2.68 - 2.57 (m, 1H), 2.35 (s, 1H), 2.21 - 2.00 (m, 4H). LC-MS: m / z: (M+H)⁺=565.3.

### Embodiment 39

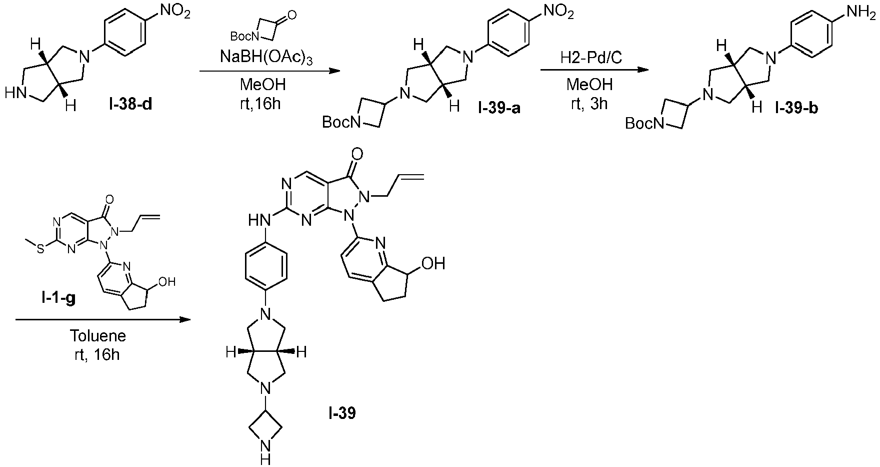

### Step 1:

(3aR, 6a*S*)-2-(4-nitrophenyl)octahydropyrrolo[3,4-c]pyrrole (**I-38-d**) (300 mg, 1.29 mmol) was dissolved in methanol (10 mL), then *tert*-butyl 3-oxazetidine-1-carboxylate (660 mg, 3.86 mmol) and sodium triacetoxyborohydride (1.37 g, 6.45 mmol) were added thereto, and the mixture was stirred for 16 hours at room temperature. The reaction mixture was concentrated, dissolved with ethyl acetate, washed twice with saturated sodium bicarbonate, the organic layer was dried over anhydrous sodium sulfate, filtered and the crude product obtained by concentration was purified by thin layer chromatography to obtain a yellow solid (I-39-a) (470 mg, 1.21 mmol) with a yield of 94 %.

### Step 2:

*Tert*-butyl 3-((3a*R*,6a*S*)-5-(4-nitrophenyl)hexahydropyrrolo[3,4-*c*]pyrrol-2(1*H*)-yl)azefidine-1-carboxylate (**I-39-a**) (470 mg, 1.21 mmol) and palladium carbon (10 mg) were dissolved in 10 mL of methanol, and the reaction was stirred for 3 hours at room temperature under a hydrogen balloon. After the reaction was completed, the mixture was filtered by diatomite to obtain a yellow solid (**I-39-b**) (320 mg, 0.892 mmol), which was directly used in the next step with a yield of 74 %. LC-MS: m / z: (M+H)⁺=359.2.

### Step 3:

*m*-Chloroperoxybenzoic acid (55 mg, 0.24 mmol) was added to a solution of 2-allyl-1-(7-hydroxy-6,7-dihydro-5*H*-cyclopenta[*b*]pyridin-2-yl)-6-(methylthio)-1,2-dihydro-3*H*-pyrazolo[3,4-*d*]pyrimidin-3-one (**I-1-g**) (84 mg, 0.236 mmol) in 15 mL of toluene, and the obtained solution was stirred at room temperature for 1 hour to obtain a solution of 2-allyl-1-(7-hydroxy-6,7-dihydro-5*H*-cyclopenta[*b*]pyridin-2-yl)-6-(methylsulfinyl)-1,2-dihydro-3*H*-pyrazolo[3,4-*d*]pyrimidin-3-one in toluene. *Tert*-butyl 3-((3a*R*,6a*S*)-5-(4-aminophenyl)hexahydropyrrolo[3,4-c]pyrrol-2(1*H*)-yl)azetidine-1-carboxylate (**I-39-b**) (93 mg, 0.26 mmol) and 0.1 mL of *N*,*N*-diisopropylethylamine were added to the obtained solution, and the mixture was stirred at room temperature for 16 hours. The mixture was concentrated under reduced pressure, and the obtained crude product was separated and purified by thin layer chromatography (7M ammonia methanol: dichloromethane =0-10%) to obtain the target compound protected by Boc, the target compound protected by Boc was dissolved in 20 mL of dichloromethane, 0.5 mL of 4 N dioxane solution of hydrogen chloride was added, and the mixture was stirred at room temperature for 16 hours. Then the mixture was concentrated under reduced pressure, the obtained crude product was first dissolved with water and then extracted with dichloromethane. The aqueous layer was taken and its pH was adjusted to 10 with 2N NaOH solution and filtered to obtain 76 mg of the target compound as a yellow solid (**I-39**) with a yield of 57 %. ¹H NMR (400 MHz, MeOD) δ 8.85 - 8.72 (m, 1H), 7.97 (t, J = 7.9 Hz, 1H), 7.78 (d, J = 7.8 Hz, 1H), 7.64 (dd, J = 7.7, 0.7 Hz, 1H), 7.48 (d, J = 8.3 Hz, 2H), 6.70 (d, J = 9.0 Hz, 2H), 5.72 (ddt, J = 16.4, 10.3, 6.1 Hz, 1H), 5.05 (dd, J = 10.2, 1.1 Hz, 1H), 4.94 (d, J = 1.3 Hz, 1H), 4.82 (d, J = 6.1 Hz, 1H), 3.80 - 3.44 (m, 2H), 3.44 - 3.36 (m, 0H), 3.31 - 3.15 (m, 2H), 2.97 (s, 1H), 2.89 - 2.71 (m, 1H), 2.37 (dd, J = 9.1, 4.1 Hz, 1H), 1.59 (s, 3H). LC-MS: m / z: (M+H)⁺=566.3.

### Embodiment 40

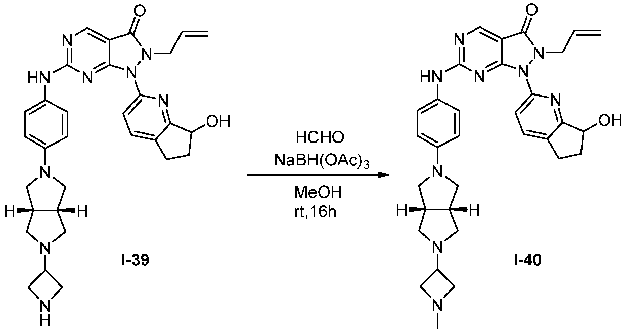

2-Allyl-6-(((4-((3a*R*,6a*S*)-5-(azetidin-3-yl)hexahydropyrrolo[3,4-c]pyrrol-2(1*H*)-yl)phenyl)amino)-1-(7-hydroxy-6,7-dihydro-5*H*-cyclopenta[*b*]pyridin-2-yl)-1,2-dihydro-3*H*-pyrazolo[3,4-*d*]pyrimidin-3-one **(1-39)** (70 mg, 0.124 mmol) was dissolved in methanol (10 mL), then formaldehyde solution (0.1 mL) and sodium triacetoxyborohydride (52 mg, 0.248 mmol) were added thereto, and the mixture was stirred for 16 hours at room temperature. The reaction mixture was concentrated, dissolved in a mixture of dichloromethane and methanol (10/1, 20 mL), washed twice with saturated sodium bicarbonate, the organic layer was dried over anhydrous sodium sulfate, filtered, and the crude product obtained by concentration was separated and purified by thin layer chromatography (7 M ammonia methanol: dichloromethane =0-10%) to obtain 14 mg of the target compound **(1-40)** with a yield of 20 %. ¹H NMR (400 MHz, CDCl₃) δ 8.80 (s, 1H), 7.73 (d, J = 8.0 Hz, 1H), 7.66 (s, 1H), 7.39 (s, 2H), 6.63 (d, J = 8.9 Hz, 2H), 5.71 (ddd, J = 16.5, 11.3, 5.1 Hz, 1H), 5.30 - 5.17 (m, 1H), 5.01 (dd, J = 32.2, 13.5 Hz, 2H), 4.71 (s, 2H), 3.71 (s, 2H), 3.44 - 3.31 (m, 2H), 3.21 (dd, J = 14.9, 6.6 Hz, 4H), 3.10 (ddd, J = 16.4, 9.0, 3.9 Hz, 1H), 2.99 (s, 1H), 2.89 (dd, J = 16.2, 8.0 Hz, 1H), 2.85 - 2.75 (m, 1H), 2.63 (ddd, J = 11.9, 9.9, 5.8 Hz, 1H), 2.49 (s, 1H), 2.35 (d, J = 6.3 Hz, 1H), 2.20 - 2.07 (m, 2H). LC-MS: m / z: (M+H)⁺=580.0.

### Embodiment 41

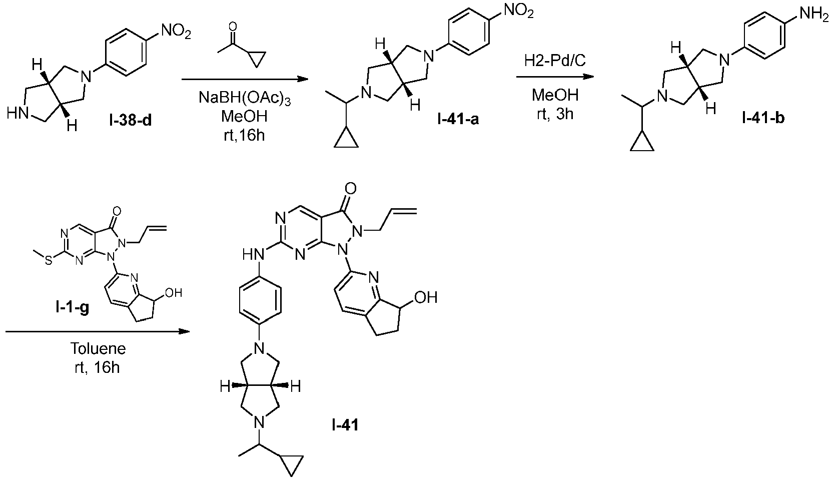

### Step 1:

(3aR, 6a*S*)-2-(4-nitrophenyl)octahydropyrrolo[3,4-*c*]pyrrole **(I-38-d)** (300 mg, 1.29 mmol) was dissolved in methanol (10 mL), then 1-cyclopropylethan-1-one (325 mg, 3.86 mmol) and sodium triacetoxyborohydride (1.37 g, 6.45 mmol) were added thereto, and the mixture was stirred for 16 hours at room temperature. The reaction mixture was concentrated, dissolved with ethyl acetate, washed twice with saturated sodium bicarbonate, the organic layer was dried over anhydrous sodium sulfate, filtered and the crude product obtained by concentration was purified by thin layer chromatography to obtain a yellow solid **(I-41-a)** (250 mg, 0.830 mmol) with a yield of 65 %.

### Step 2:

(3a*R*,6a*S*)-2-(1-cyclopropylethyl)-5-(4-nitrophenyl)octahydropyrrolo[3,4-*c*]pyrrole (**I-41-a**) (250 mg, 0.830 mmol) and palladium carbon (10 mg) were dissolved in 10 mL of methanol and the reaction was stirred for 3 hours at room temperature under a hydrogen balloon. After the reaction was completed, the mixture was filtered by diatomite to obtain a yellow solid **(I-41-b)** (185 mg, 0.682 mmol), which was directly used in the next step with a yield of 82 %. LC-MS: m / z: (M+H)⁺=272.2.

### Step 3:

*m*-Chloroperoxybenzoic acid (55 mg, 0.24 mmol) was added to a solution of 2-allyl-1-(7-hydroxy-6,7-dihydro-5*H*-cyclopenta[*b*]pyridin-2-yl)-6-(methylthio)-1,2-dihydro-3*H*-pyrazolo[3,4-*d*]pyrimidin-3-one (**I-1-g**) (80 mg, 0.22 mmol) in 15 mL of toluene, and the obtained solution was stirred at room temperature for 1 hour to obtain a solution of 2-allyl-1-(7-hydroxy-6,7-dihydro-5*H*-cyclopenta[*b*]pyridin-2-yl)-6-(methylsulfinyl)-1,2-dihydro-3*H*-pyrazolo[3,4-*d*]pyrimidin-3-one in toluene. 4-((3a*R*, 6a*S*)-5-(1-cyclopropylethyl)hexahydropyrrolo[3, 4-*c*]pyrrol-2(1*H*)-yl)aniline (**I-41-b**) (65 mg, 0.24 mmol) and 0.1 mL of N, *N*-diisopropylethylamine were added to the obtained solution, and the mixture was stirred at room temperature for 16 hours. The mixture was concentrated under reduced pressure, the obtained crude product was separated and purified by thin layer chromatography (7M ammonia methanol: dichloromethane = 0-10%) to obtain 25 mg of the target compound as a yellow solid **(1-41)** with a yield of 19 %. ¹H NMR (400 MHz, CDCl₃) δ 8.78 (s, 1H), 7.73 (d, J = 8.2 Hz, 1H), 7.64 (s, 1H), 7.39 (d, J = 5.9 Hz, 2H), 6.64 (d, J = 8.9 Hz, 2H), 5.70 (ddt, J = 16.5, 10.2, 6.2 Hz, 1H), 5.24 (t, J = 6.8 Hz, 1H), 5.00 (dd, J = 34.2, 13.6 Hz, 2H), 4.69 (s, 2H), 3.59 (s, 1H), 3.44 (s, 1H), 3.32 (t, J = 7.7 Hz, 2H), 3.28 - 3.18 (m, 2H), 3.15 - 3.04 (m, 3H), 2.95 - 2.82 (m, 2H), 2.62 (ddd, J = 11.8, 8.7, 4.1 Hz, 2H), 2.53 (s, 3H), 2.19 - 2.06 (m, 1H), 1.70 (s, 1H), 1.34 (d, J = 6.4 Hz, 3H), 0.95 (s, 1H), 0.69 (s, 1H), 0.59 - 0.51 (m, 1H), 0.37 (dd, J = 9.5, 4.7 Hz, 1H), 0.10 (dd, J = 9.8, 4.9 Hz, 1H). LC-MS: m / z: (M+H)⁺=579.0.

### Embodiment 42

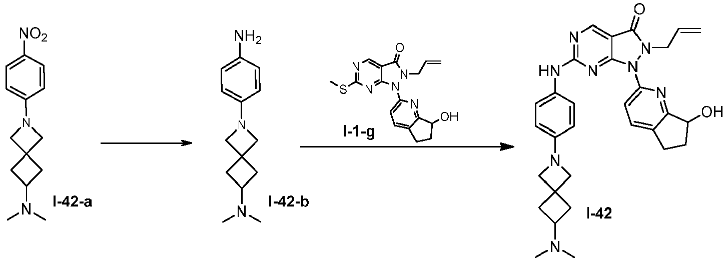

### Step 1:

*N,N*-dimethyl-2-(4-nitrophenyl)-2-azaspiro[3.3]hept-6-amine (100 mg, 0.383 mmol) **(I-42-a)** and 10% palladium carbon (30 mg) were added to 30 mL of dichloromethane, and the reaction flask was ventilated three times with a hydrogen balloon, and the reaction mixture was stirred overnight at room temperature in a hydrogen atmosphere. 5mL of methanol was added to the reaction mixture, then the mixture was filtered and evaporated to dryness to obtain 85 mg of a brown solid **(I-42-b),** which was directly used in the next step. The yield was 96%. LC-MS: m / z: (M+H)⁺ =232.

### Step 2:

*m*-Chloroperoxybenzoic acid (75 mg, 0.37 mmol) with a content of 85 % was added to a solution of 2-allyl-1-(7-hydroxy-6,7-dihydro-5*H*-cyclopenta[*b*]pyridin-2-yl)-6-(methylthio)-1,2-dihydro-3*H-*pyrazolo[3,4-*d*]pyrimidin-3-one (107 mg, 0.3 mmol) **(I-42-b)** in 15 mL of toluene, and the obtained mixture was stirred at room temperature for 1 hour.

2-(4-Aminophenyl)-*N*,*N*-dimethyl-2-azaspiro[3.3]hept-6-amine (**I-42-b**) (85mg, 0.21 mmol), 0.5 mL of *N*,*N*-diisopropylethylamine and 6 mL of dimethyl sulfoxide were added to the above reaction mixture, and the mixture was stirred overnight at room temperature. 5 mL of saturated sodium carbonate aqueous solution and 25 mL of water were added to the above reaction mixture, and the mixture was extracted three times with dichloromethane (3^{∗}20 mL); then the organic phase was combined, washed with 20 mL of water and 20 mL of saturated sodium chloride solution, respectively, dried over anhydrous sodium sulfate, and concentrated to obtain a crude product; the crude product was separated by thin layer chromatography plate {7M ammonia methanol: (dichloromethane: ethyl acetate = 8: 1) = 1:9} to obtain 40 mg of a yellow solid (**I-42**) with a yield of 24 %. LC-MS: m / z: (M+H)⁺ =539, ¹H NMR (400 MHz, CDCl₃₎ δ 8.77 (s, 1H), 7.72 (d, J = 8.2 Hz, 1H), 7.65 (s, 1H), 7.35 (d, J = 6.6 Hz, 2H), 6.40 (d, J = 8.8 Hz, 2H), 5.69 (ddt, J = 16.5, 10.2, 6.2 Hz, 1H), 5.28 - 5.19 (m, 1H), 5.03 (d, J = 10.1 Hz, 1H), 4.95 (d, J = 17.1 Hz, 1H), 4.70 (s, 2H), 3.88 (s, 2H), 3.79 (s, 2H), 3.09 (ddd, J = 16.2, 8.9, 3.8 Hz, 1H), 2.95 - 2.83 (m, 1H), 2.62 (m, 2H), 2.43 - 2.34 (m, 2H), 2.22 - 2.11 (m, 9H).

With reference to the above embodiments, the compounds shown in Table 1 were prepared, and their structural characteristics were as follows:

**Table 1 List of compounds**

| Compoun d | Structure | Characterization data | Refere nce metho d |
|---|---|---|---|
| I-3 | 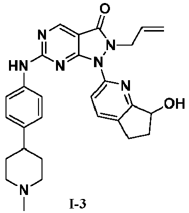 | ¹H NMR (400 MHz, MeOD) δ 8.82 (s, 1H), 7.89 (d, *J* = 8.1 Hz, 1H), 7.70 (d, *J* = 8.1 Hz, 1H), 7.65 (d, *J* = 8.6 Hz, 2H), 7.21 (d, *J* = 8.6 Hz, 2H), 5.72 (m, 1H), 5.12 (dd, *J* = 7.0, 5.6 Hz, 1H), 5.04 (dd, *J* = 10.2, 1.1 Hz, 1H), 4.95 (dd, *J* = 17.1, 1.2 Hz, 1H), 4.73 (d, *J* = 5.1 Hz, 2H), 3.58 (d, *J* = 12.1 Hz, 2H), 3.10 (m, 3H), 2.97 - 2.78 (m, 5H), 2.56 (m, 1H), 2.15 - 1.90 (m, 5H). LC- MS: m / z: (M+H)⁺ =498. | I-5 |
| I-4 | 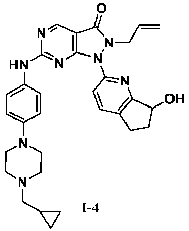 | ¹H NMR (400 MHz, Chloroform-d) δ 8.73 (s, 1H), 8.27 (s, 1H), 7.74 - 7.66 (m, 1H), 7.60 (d, J = 6.8Hz, 1H), 7.38 (d, J = 8.3 Hz, 2H), 6.80 (d, J = 8.8 Hz, 2H), 5.73 - 5.56 (m, 1H), 5.22 (t, J = 6.8 Hz, 1H),5.00 (dd, J = 10.3, 1.4 Hz, 1H), 4.92 (dd, J = 17.1, 1.5 Hz, 1H), 4.66 (d, J = 6.2 Hz, 2H), 3.29 - 3.19 (m,4H), 3.07 (dq, J = 12.8, 4.6, 3.8 Hz, 1H), 2.88 (q, J = 8.1 Hz, 1H), 2.81 (t, J = 5.1 Hz, 4H), 2.61 (dtd, J =16.0, 7.9, 3.8 Hz, 1H), 2.47 - 2.35 (m, 2H), 2.16 - 2.01 (m, 1H), 1.05 - 0.90 (m, 1H), 0.64 - 0.51 (m, 2H),0.19 (p, J = 5.2 Hz, 2H). LC- MS: m / z: (M+H)⁺ =593. | I-5 |
| I-7 | 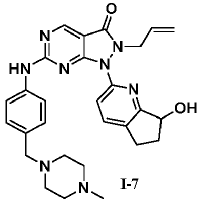 | ¹H NMR (400 MHz, CDC13) δ 8.85 (s, 1H), 7.78 (d, J = 8.2 Hz, 1H), 7.66 (d, J = 8.2 Hz, 1H), 7.55 (d, J = 8.2 Hz, 2H), 7.33 - 7.26 (d, J = 8.2 Hz, 2H), 5.80 - 5.64 (m, 1H), 5.29 - 5.21 (m, 1H), 5.05 (d, J = 10.2 Hz, 1H), 4.97 (d, J = 17.1 Hz, 1H), 4.72 (d, J = 6.1 Hz, 2H), 3.52 (s, 2H), 3.13 (m, 1H), 2.97 - 2.87 (m, 1H), 2.73 - 2.38 (m, 9H), 2.35 (s, 3H), 2.18 - 2.08 (m, 1H). LC- MS: m / z: (M+H)⁺ =513. | I-2 |
| I-8 | 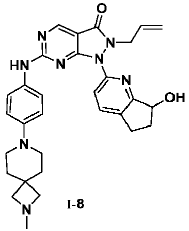 | ¹H NMR (400 MHz, CDC13) δ1H NMR (400 MHz, MeOD) δ 8.80 (s, 1H), 7.91 (d, J = 8.2 Hz, 1H), 7.72 (d, J = 8.1 Hz, 1H), 7.55 (d, J= 8.8 Hz, 2H), 6.97 (d, J = 9.1 Hz, 2H), 5.74 (ddd, J = 23.1, 10.2, 6.0 Hz, 1H), 5.14 (dd, J = 7.2, 5.5 Hz,1H), 5.06 (dd, J = 10.2, 1.2 Hz, 1H), 5.01 - 4.94 (m, 1H), 4.76 (d, J = 6.0 Hz, 2H), 3.18 - 3.03 (m, 4H),3.00 - 2.87 (m, 1H), 2.64 - 2.47 (m, 4H), 2.26 - 2.17 (m, 1H), 2.05 (dt, J = 13.9, 8.5 Hz, 2H), 1.99 - 1.84(m, 3H), 1.39 (d, J = 28.9 Hz, 2H), 1.31 -1.21 (m, 2H). LC-MS: m / z: (M+H)⁺ =539. | I-5 |
| 1-10 | 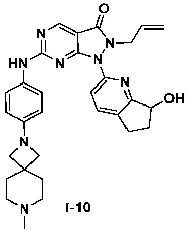 | ¹H NMR (400 MHz, CDC13) δ1H NMR (400 MHz, MeOD) δ 8.75 (s, 1H), 8.59 (s, 1H), 7.85 (d, *J=* 8.2 Hz, 1H), 7.69 (d, *J* = 8.1 Hz,1H), 7.44 (d, *J* = 7.9 Hz, 2H), 6.44 (d, *J* = 8.9 Hz, 2H), 5.73 (dd, *J* = 17.0, 10.3 Hz, 1H), 5.13 (dd, *J* = 7.2,5.5 Hz, 1H), 5.05 (dd, *J* = 10.2, 1.1 Hz, 1H), 4.98 (d, *J* = 1.3 Hz, 1H), 4.75 (d, *J* = 5.4 Hz, 2H), 3.64 (s,4H), 3.04-3.11 (m, 4H), 2.94 - 2.83 (m, 1H), 2.71 (s, 3H), 2.61 - 2.50 (m, 1H), 2.15 -1.93 (m, 5H). LC-MS: m / z: (M+H)⁺ =539. | I-5 |
| I-11 | 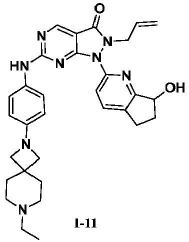 | LC-MS: m / z: (M+H)⁺ =553. 1H NMR (400 MHz, CDCl3) δ1H NMR (400 MHz, MeOD) δ 8.78 (s, 1H), 7.87 (d, J = 8.1 Hz, 1H), 7.71 (d, J = 8.1 Hz, 1H), 7.47 (d, J= 7.3 Hz, 2H), 6.48 (d, J = 8.8 Hz, 2H), 5.83 - 5.65 (m, 1H), 5.14 (dd, J = 7.2, 5.5 Hz, 1H), 5.09 - 5.04 (m,1H), 4.99 (d, J = 1.2 Hz, 1H), 4.76 (d, J = 5.6 Hz, 2H), 3.66 (s, 4H), 3.26 - 2.72 (m, 8H), 2.67 - 2.49 (m,1H), 2.17 - 1.95 (m, 5H), 1.30 (t, J = 7.2 Hz, 3H). | I-5 |
| I-15 | 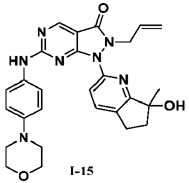 | ¹H NMR (400 MHz, CDCl₃) δ 8.75 (s, 1H), 8.27 (s, 1H), 7.67 (q, 2H), 7.45 (d, *J* = 8.9 Hz, 2H), 6.86 (d, *J* = 9.0 Hz, 2H), 5.67 (ddt, *J* = 16.7, 10.2, 6.3 Hz, 1H), 4.99 (dd, *J* = 10.2, 1.1 Hz, 1H), 4.89 (dd, *J* = 17.1, 1.3 Hz, 1H), 4.75 (m, 2H), 3.97 - 3.80 (m, 4H), 3.24 - 3.10 (m, 4H), 3.05 (m, 1H), 2.91 - 2.81 (m, 1H), 2.40 - 2.25 (m, 2H), 1.60 (s, 3H). LC- MS: m / z: (M+H)⁺ =500. | I-14 |
| I-22 | 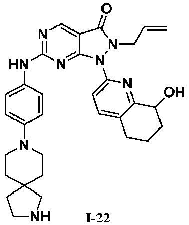 | ¹H NMR (400 MHz, CDCl₃) δ 8.75 (d, J= 11.5 Hz, 1H), 7.65 (d, *J* = 8.0 Hz, 1H), 7.58 (d, *J* = 8.3 Hz, 1H), 7.43 (d, *J* = 8.2 Hz, 2H), 6.88 (dd, *J* = 17.2, 9.1 Hz, 2H), 5.72 (ddt, *J* = 16.4, 10.3, 6.1 Hz, 1H), 5.06 (dd, *J* = 10.2, 1.0 Hz, 1H), 4.98 (dd, *J* = 17.1, 1.2 Hz, 1H), 4.73 (t, *J* = 6.5 Hz, 1H), 4.68 (d, *J* = 5.1 Hz, 2H), 3.23 - 2.95 (m, 6H), 2.86 (m, 4H), 2.34 - 2.19 (m, 1H), 2.12 - 1.98 (m, 1H), 1.94 - 1.79 (m, 2H), 1.79 - 1.58 (m, 6H). LC- MS: m / z: (M+H)⁺ =553. | I-21 |
| I-24 | 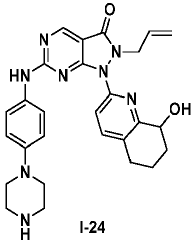 | ¹H NMR (400 MHz, CDC13) δ 8.77 (s, 1H), 7.64 (d, *J* = 8.2 Hz, 1H), 7.57 (d, *J* = 8.3 Hz, 1H), 7.43 (d, *J* = 8.6 Hz, 2H), 6.92 - 6.83 (m, 2H), 5.72 (ddt, *J* = 16.3, 10.2, 6.1 Hz, 1H), 5.06 (dd, *J* = 10.2, 1.1 Hz, 1H), 4.98 (dd, *J =* 17.1, 1.3 Hz, 1H), 4.73 (t, *J* = 6.5 Hz, 1H), 4.68 (d, *J* = 5.5 Hz, 2H), 3.12 (m, 4H), 3.09 - 3.00 (m, 4H), 2.92 - 2.81 (m, 2H), 2.33 - 2.24 (m, 1H), 2.09 - 1.99 (m, 1H), 1.92 - 1.79 (m, 2H). LC- MS: m / z: (M+H)⁺ =499. | I-21 |
| I-25 | 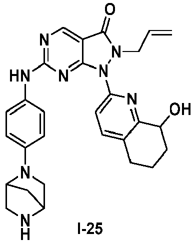 | ¹H NMR (400 MHz, CDCl₃) δ 8.75 (d, *J* = 4.1 Hz, 1H), 8.29 (s, 1H), 7.65 (d, *J=* 7.4 Hz, 1H), 7.56 (d, *J* = 8.2 Hz, 1H), 7.35 (d, *J* = 7.1 Hz, 2H), 6.51 (d, *J* = 8.9 Hz, 2H), 5.73 (ddtd, *J* = 14.3, 10.2, 6.0, 2.1 Hz, 1H), 5.07 (d, *J =* 10.2 Hz, 1H), 5.00 (d, *J* = 17.1 Hz, 1H), 4.73 (t, *J* = 6.5 Hz, 1H), 4.67 (d, *J* = 5.2 Hz, 2H), 4.29 (s, 1H), 3.79 (s, 1H), 3.66 (d, *J* = 8.2 Hz, 1H), 3.12 (d, *J* = 9.7 Hz, 1H), 3.04 (d, *J* = 9.5 Hz, 1H), 2.96 (d, *J* = 7.3 Hz, 1H), 2.92 - 2.76 (m, 2H), 2.35 - 2.19 (m, 2H), 2.11 - 1.99 (m, 1H), 1.96 (d, *J* = 9.3 Hz, 1H), 1.86 (m, 3H). LC-MS: m / z: (M+H)⁺ =511. | I-21 |
| I-30-1 (Retention time = 9.66 min) | 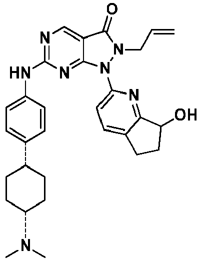 | ¹H NMR (400 MHz, CDCl₃) δ 8.82 (s, 1H), 7.88 (s, 1H), 7.76 (d, *J* = 8.2 Hz, 1H), 7.66 (d, *J* = 8.1 Hz, 1H), 7.50 (d, *J* = 8.4 Hz, 2H), 7.15 (d, *J* = 8.5 Hz, 2H), 5.70 (ddt, J = 16.5, 10.2, 6.2 Hz, 1H), 5.24 (t, *J* = 6.7 Hz, 1H), 5.04 (d, *J* = 10.1 Hz, 1H), 4.96 (d, *J* = 17.1 Hz, 1H), 4.72 (d, *J* = 6.0 Hz, 2H), 3.11 (ddd, *J* = 16.2, 8.9, 3.9 Hz, 1H), 2.97 - 2.84 (m, 1H), 2.69 - 2.57 (m, 1H), 2.54 - 2.42 (m, 1H), 2.36 (m, 7H), 2.18 - 2.04 (m, 3H), 1.99 (d, *J* = 12.5 Hz, 2H), 1.59 - 1.36 (m, 4H). LC- MS: m / z: (M+H)⁺= 526.3. HPLC retention time = 9.66 min, HPLC conditions: gradient elution, 5% mobile phase B → 50% mobile phase B. | I-34 |
| I-30-2 (Retention time = 10.04 min) | 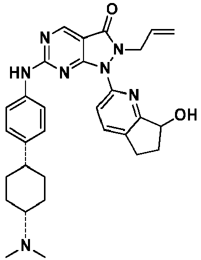 | ¹H NMR (400 MHz, CDCl₃) δ 8.84 (s, 1H), 7.83 (d, *J* = 8.2 Hz, 1H), 7.67 (d, *J* = 8.1 Hz, 2H), 7.52 (d, *J* = 8.5 Hz, 2H), 7.31 (s, 1H), 5.72 (ddt, *J* = 16.4, 10.2, 6.2 Hz, 1H), 5.25 (t, *J* = 6.7 Hz, 1H), 5.05 (dd, *J* = 10.2, 1.0 Hz, 1H), 4.97 (dd, *J* = 17.1, 1.2 Hz, 1H), 4.79 - 4.65 (m, 2H), 3.14 (ddd, J= 16.2, 9.1, 3.8 Hz, 2H), 2.99 - 2.87 (m, 1H), 2.76 - 2.57 (m, 2H), 2.42 (s, 6H), 2.14 (ddd, *J* = 13.6, 7.6, 4.5 Hz, 1H), 2.04 (t, *J* = 15.6 Hz, 4H), 1.67 (s, 4H). LC-MS: m / z: (M+H)⁺= 526.3, HPLC retention time = 10.04 min, HPLC conditions: gradient elution, 5% mobile phase B → 50% mobile phase B. | I-34 |
| I-31 | 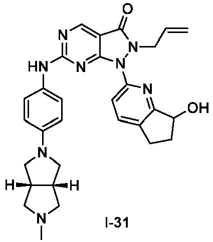 | ¹H NMR (400 MHz, CDCl₃) ¹H NMR (400 MHz, MeOD) δ 8.77 (s, 1H), 8.56 (s, 1H), 7.88 (d, *J* = 8.2 Hz, 1H), 7.71 (d, *J* = 8.1 Hz,1H), 7.49 (d, *J* = 8.1 Hz, 2H), 6.74 (d,J= 8.9 Hz, 2H), 5.74 (ddt, J= 16.4, 10.3, 6.1 Hz, 1H), 5.14 (dd, *J* = 7.2, 5.5 Hz, 1H), 5.06 (dd, *J* = 10.2, 1.0 Hz, 1H), 5.01 - 4.93 (m, 1H), 4.75 (d, *J* = 5.5 Hz, 2H), 3.47 - 3.34(m, 3H), 3.20 - 3.05 (m, 5H), 3.04 - 2.84 (m, 3H), 2.71 (s, 3H), 2.63 - 2.50 (m, 2H), 2.04 (ddd, *J* = 14.0,8.7, 6.3 Hz, 1H). LC- MS: m / z: (M+H)⁺ =525. | I-14 |
| I-36-1 (Retention time = 7.80 min) | 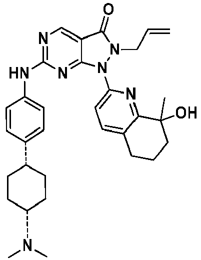 | ¹H NMR (400 MHz, CDCl₃) ¹H NMR (400 MHz, MeOD) δ8.83 (s, 1H), 8.60 (s, 1H), 7.78 - 7.70 (m, 2H), 7.62 (d, *J* = 8.5 Hz, 2H),7.20 (d, *J* = 8.6 Hz, 2H), 5.82 - 5.63 (m, 1H), 5.05 (dd, *J* = 15.4, 6.1 Hz, 1H), 5.00 - 4.92 (m, 2H), 4.79(dd, J= 16.2,6.3 Hz, 1H), 4.62 (s, 1H), 3.10 (s, 1H), 2.96 - 2.82 (m, 2H), 2.77 (s, 6H), 2.61 (d, *J* = 26.0Hz, 1H), 2.19 (dt, *J* = 30.8, 15.5 Hz, 2H), 2.12 -1.95 (m, 5H), 1.87 (dd, *J* = 12.5, 6.4 Hz, 1H), 1.85 (s,7H). LC- MS: m / z: (M+H)⁺= 554.3, HPLC retention time = 7.80 min, HPLC conditions: gradient elution, 5% mobile phase B → 95% mobile phase B. | I-34 |
| I-36-2 (Retention time = 7.97 min) | 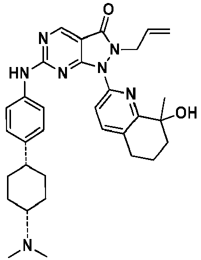 | ¹H NMR (400 MHz, CDCl₃) ¹H NMR (400 MHz, MeOD) δ8.82 (s, 1H), 8.59 (s, 1H), 7.73 (t, J = 6.0 Hz, 2H), 7.64 (d, J = 8.6 Hz,2H), 7.30 (d, J = 8.6 Hz, 2H), 5.74 (s, 1H), 5.05 (dd, J = 10.2, 1.0 Hz, 1H), 4.95 (dd, J = 12.5, 6.0 Hz, 2H), 4.81 (d, J = 6.2 Hz, 1H), 2.89 (ddd, J = 16.1, 13.1, 8.3 Hz, 4H), 2.81 - 2.62 (m, 6H), 2.20 - 1.95 (m, 7H),1.95 - 1.78 (m, 5H), 1.63 (s, 3H). LC-MS: m / z: (M+H)⁺= 554.3, HPLC retention time = 7.97 min, HPLC conditions: gradient elution, 5% mobile phase B → 95% mobile phase B. | I-34 |
| I-37 | 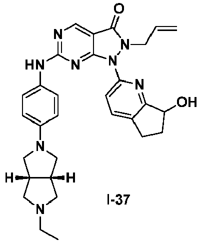 | ¹H NMR (400 MHz, Chloroform-*d*) δ 8.74 (s, 1H), 7.73 (d, *J* = 8.1 Hz, 1H), 7.62 (s, 1H), 7.35 (s, 2H), 6.64 - 6.56 (m, 2H), 5.69 (ddt, *J* = 16.6, 10.2, 6.2 Hz, 1H), 5.24 (dd, *J* = 7.5, 6.1 Hz, 1H), 5.03 (dd, *J* = 10.3, 1.3 Hz, 1H), 4.95 (dq, *J* = 17.1, 1.4 Hz, 1H), 4.68 (s, 2H), 3.29 - 3.20 (m, 5H), 3.13 - 3.06 (m, 3H), 2.89 (dt, *J* = 16.1, 7.8 Hz, 2H), 2.71 (q, *J=* 7.2 Hz, 2H), 2.65 - 2.59 (m, 1H), 2.52 (dd, *J* = 10.3, 4.6 Hz, 2H), 2.15 - 2.06 (m, 1H), 1.28 - 1.22 (m, 3H). LC-MS: m/z: [M+1]⁺= 539.3 | I-14 |
| I-43 | 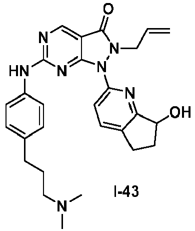 | ¹H NMR (400 MHz, Chloroform-d) δ 8.99 (d, J = 21.8 Hz, 1H), 8.79 (s, 1H), 8.12 - 7.83 (m, 1H), 7.74(d, J = 8.1 Hz, 1H), 7.62 (d, J = 8.2 Hz, 1H), 7.46 (d, J = 8.1 Hz, 2H), 7.11 (d, J = 8.2 Hz, 2H), 5.67 (ddt,J = 16.6, 10.2, 6.2 Hz, 1H), 5.22 (t, J = 6.8 Hz, 1H), 5.02 (d, J = 10.2 Hz, 1H), 4.93 (d, J = 17.1 Hz, 1H),4.67 (q, J = 9.4, 7.1 Hz, 2H), 3.09 (ddd, J = 16.4, 9.0, 3.9 Hz, 1H), 2.88 (dt, J = 16.0, 7.8 Hz, 1H), 2.61 (t,J = 7.9 Hz, 4H), 2.43 - 2.35 (m, 2H), 2.30 (s, 6H), 2.10 (ddt, J = 13.4, 8.9, 6.7 Hz, 1H), 1.83 (p, J = 7.7Hz, 2H), 1.35-1.22 (m, 2H). LC-MS: m/z: [M+1]⁺= 541.4. | I-17 |

### Effect embodiment 1

### I. Inhibitory effect of compound on WEE1 kinase in vitro

### Test method:

The tested compounds were screened on WEE1 kinase with ATP concentration of Km by ELISA. Three compounds were screened on WEE1 kinase to evaluate the kinase inhibitory activity of the tested compounds. In the detection process, the initial concentration of the tested compounds was all selected as 100 nM, and each compound was selected with 6 gradient dilution concentrations, the gradient dilution ratio was 4-fold, and two replicate wells were detected for each concentration, MK1775 was used as the standard control.

WEE1, purchased from CarnaBiosciences, Inc., Item No. 05-177; dimethyl sulfoxide, purchased from Sigma-Aldrich, Item No. D8418; ATP, purchased from Sigma-Aldrich, Item No. A7699; DTT solution, purchased from Sigma-Aldrich, Item No. 43816; protein tyrosine kinase (PTK) substrate (poly-Glu-Tyr), purchased from Sigma-Aldrich, Item No. P4476; P-Tyr (PY99), purchased from Santa Cruz, Item No. sc-7020; Anti-mouse IgG HRP-linked Antibody, purchased from Santa Cruz, Item No. 7076S; TMB liquid Substrate System, purchased from Sigma-Aldrich, Item No. T0440; Costar Stripwell Microplate No Lid 1× 8 Flat Bottom, Certified High Binding, purchased from Sigma-Aldrich, Item No. 42592; 96-well compound plate, purchased from Thermo Scientific, Item No. 267245.

### Test steps:

1. Coating substrate: 1) An appropriate volume of substrate storage solution protein tyrosinase (PTK) substrate (poly-Glu-Tyr) was taken, diluted 10 times with PBS, and the concentration was diluted from 250 mg/mL to 25 mg/mL. The mixture was added to a high adsorption 96-well plate at 125 µL per well. The plate was placed in an incubator at 37 °C for overnight coating. 2) After 24 hours, the 96-well plate was taken out, the liquid in the 96-well plate was poured out, cleaned with washing buffer for 3 times, and the incubator at 37 was inverted and dried for 2 hours.
2. Compound preparation and transfer: 1) compound dilution: 10 mM of the test compound storage solution was taken, the compound in 96-well compound plate was diluted with DMSO in multiple steps to obtain the initial concentration of 100× compound as the first concentration, and then DMSO was used for 4-fold gradient dilution, for a total of 6 concentrations; after that, 2 µL of the gradient dilution solution was added to 48 µL of 1× reaction buffer respectively to prepare 4× compound; 2) 4× compounds were transferred: 10 µL of 4× compounds from the 96-well compound plate configured in the previous step were transferred into the dried high adsorption 96-well plate; 10 µL of the following liquids were added to the compound-free control wells and ATP-control wells: 2 µL of DMSO was added to 48 µL of 1× reaction buffer.
3. Enzyme reaction stage: 1) WEE1 kinase and ATP were prepared into 2× enzyme solution and 4× ATP solution respectively with 1× reaction buffer. In this screening, the final concentration of WEE1 kinase was: 0.15 ng/µL and the final concentration of ATP was: 12 µM; 2) 20 µL of enzyme solution of 2 was added to the high adsorption 96-well plate; 3) 10 µL of 4×ATP solution was added to the high adsorption 96-well plate and 10 µL of 1×reaction buffer was added to the ATP-control group; 4) the plate was placed in HERAEUS Multifuge X1R centrifuge at 2000 rpm for 20 s and then placed at room temperature and the reaction was carried out for 60 min.
4. Reaction termination stage: 1) the reaction mixture in the plate was poured out, 200 µL of washing buffer was added to each well, and washed for 5 times; the primary antibody P-Tyr (PY99) (dilution ratio 1: 2000) was added, 100 µL per well, at room temperature for 30 min. 2) The primary antibody in the plate was poured out, 200 µL of washing buffer was added to each well, and washed for 5 times; the second antibody Anti-mouse IgG HRP-linked Antibody (dilution ratio 1: 2000) was added, 100 µL per well, at room temperature for 30min. 3) The secondary antibody in the plate was poured out, washed 5 times with washing buffer, and TMB was added, 100 µL per well, and colored for 10-30 min, depending on the color depth. The reaction was terminated with IN sulfuric acid before reading.
5. Detection and data processing: 1) The light absorption at 450 nM was read on ThermoScientific MultiScan GO, and the background was read at 650 nM at the same time. 2) Graphpad Prism 5.0 was used to fit Log(inhibitor) vs. response-Variable slope (four parameters) curves to the data, and the corresponding IC₅₀ (half maximal inhibitory concentration) was calculated.

### II. The data of the test result.

The structure of the control samples used in the tests was shown in Table 2.

**Table 2 Structure of control sample**

| **Control sample number** | **Control** 1 **(AZD1775/MK1775)** |
|---|---|
| **Chemical structure** | 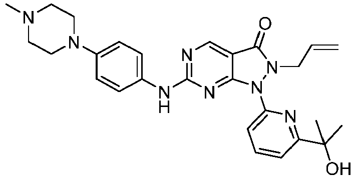 |

The test results were detailed in Table 3.

**Table 3 Test results of WEEE 1 enzyme inhibitory activity**

| Compound number | WEE1 IC₅₀, nM | Compound number | WEE1 IC₅₀, nM |
|---|---|---|---|
| Control 1 | 2.57 | 1-22 | 3.55 |
| 1-1 | 2.85 | 1-23 | 2.87 |
| 1-2 | 2.55 | 1-24 | 3.81 |
| 1-3 | 2.68 | 1-25 | 4.05 |
| 1-4 | 4.43 | 1-28 | 3.58 |
| 1-5 | 3.62 | I-30-1 (Retention time = 9.66 min) | 5.86 |
| 1-7 | 9.69 | 1-30-2 (Retention time = 10.04 min) | 6.60 |
| 1-8 | 11.3 | 1-31 | 3.12 |
| 1-9 | 2.87 | 1-32 | 4.79 |
| 1-10 | 8.59 | 1-33 | 3.48 |
| 1-11 | 11.3 | 1-34 | 4.79 |
| 1-12 | 3.68 | 1-35 | 3.15 |
| 1-13 | 3.41 | I-36-1 (Retention time = 7.80 min) | 4.75 |
| 1-14 | 2.81 | 1-36-2 (Retention time = 7.97 min) | 2.65 |
| 1-15 | 3.85 | 1-37 | 11.8 |
| 1-16 | 3.99 | 1-38 | 2.43 |
| 1-17 | 12.8 | 1-41 | 10.6 |
| 1-18 | 2.03 | 1-43 | 4.91 |
| 1-19 | 1.89 | | |
| 1-20 | 4.41 | | |
| 1-21 | 5.75 | | |

Conclusion: As shown in Table 3, the compounds of the present disclosure have a good inhibitory effect on Wee1 kinase.

### Effect embodiment 2

### I. Proliferation inhibitory of the compound on COLO 205 cells and Hela cells.

### Test method:

Luminescence ATP Detection method was used to detect the inhibitory effects of the compounds on p53-deficient cell line COLO 205 and on the proliferation of Hela cells. COLO205 cells, purchased from SIBS, generation P11; Hela purchased from ATCC, generation P13.

### Test steps:

### 1. Proliferation inhibition of COLO 205 cells

Cell culture and inoculation: on the first day of the experiment, cells in normal culture were taken in their exponential growth state, digested and dispersed, the cell density was adjusted to 4.375×10³ cells/mL, and 40 µL per well were inoculated into 96-well cell culture plates; after inoculation was completed, the microplates were placed at 37 °C and 5 % CO₂ for incubation.

Drug treatment of cells: on the third day of the experiment, microplates were removed from the incubator, 9× compound were added to each well in the microplate, and 5 µL was added to each well, wherein there were 2 replicate wells for each drug concentration and 10 drug concentrations for each compound. According to different cell lines, the initial concentration of each compound was different, after the completion, the microplate was cultured at 37 °C and 5% CO₂ for 72 hours.

Data collection: The microplates were removed from the incubator and equilibrated at room temperature for 30 minutes. 100 µL of ATPlite reaction mixture after room temperature equilibrium was added to each well, the solution was shaken at room temperature of 1300 rpm for 2 minutes, and then the microplate was placed in a HERAEUS Multifuge X1R centrifuge at 2000 rpm for 1 minute; after equilibrium at room temperature for 10 minutes, the fluorescence signal value was measured on EnVisionTM.

The inhibitory activity of the compound *in vitro* was calculated according to the following formula: inhibition rate of cell proliferation: inhibition rate (%) = (signal value of control - signal value of administration)/signal value of control × 100%. According to the inhibition rate of each concentration, 50 % inhibitory concentration (IC₅₀) was calculated by LOGIT method.

Calculation of cell viability: Cell viability(%)= signal value of administration/signal value of control× 100%. Graphpad Prism 5.0 was used to fit Log(inhibitor) vs. response-Variable slope (four parameters) curves to the fluorescence signal values at each concentration, and the corresponding IC₅₀ (half maximal inhibitory concentration) was calculated.

### 2. Proliferation inhibition of Hela cells

Cell culture and inoculation: on the first day of the experiment, cells in normal culture were taken in their exponential growth state, digested and dispersed, the cell density was adjusted to 9×10³ cells/mL, and 40 µL per well were inoculated into 384-well cell culture plates; after inoculation was completed, the microplates were placed at 37 °C and 5 % CO₂ for incubation.

Drug treatment of cells: on the second day of the experiment, microplates were removed from the incubator, 9× compound were added to each well in the microplate, and 5 µL was added to each well, wherein there were 2 replicate wells for each drug concentration and 10 drug concentrations for each compound. According to different cell lines, the initial concentration of each compound was different, after the completion, the microplate was cultured at 37 °C and 5% CO₂ for 72 hours.

Data collection: the microplates were removed from the incubator and equilibrated at room temperature for 30 minutes. 40 µL of ATPlite reaction mixture after room temperature equilibrium was added to each well, the solution was shaken at room temperature of 1300 rpm for 2 minutes, and then the microplate was placed in a HERAEUS Multifuge X1R centrifuge at 2000 rpm for 1 minute; after equilibrium at room temperature for 10 minutes, the fluorescence signal value was measured on EnVisionTM.

The inhibitory activity of the compound *in vitro* was calculated according to the following formula: inhibition rate of cell proliferation: inhibition rate (%) = (signal value of control - signal value of administration)/signal value of control × 100%. According to the inhibition rate of each concentration, 50 % inhibitory concentration (IC₅₀) was calculated by LOGIT method.

Calculation of cell viability: Cell viability(%)= signal value of administration/signal value of control× 100%. Graphpad Prism 5.0 was used to fit Log(inhibitor) vs. response-Variable slope (four parameters) curves to the fluorescence signal values at each concentration, and the corresponding IC₅₀ (half maximal inhibitory concentration) was calculated.

### II. Experimental result

**Table 4**

| Compound number | COLO 205 IC₅₀(nM) | Hela IC₅₀ (nM) |
|---|---|---|
| 1-1 | 1882 | 495 |
| 1-2 | 1735 | 345 |
| 1-3 | 5152 | 434 |
| 1-4 | 1971 | 395 |
| 1-5 | 2552 | 392 |
| 1-7 | - | 2903 |
| 1-9 | 614 | 191 |
| 1-10 | 920 | 206 |
| 1-11 | 1194 | 317 |
| 1-13 | 886 | 190 |
| 1-14 | 363 | 192 |
| 1-16 | 1615 | 316 |
| 1-19 | 944 | 170 |
| 1-23 | 1669 | 534 |
| 1-32 | 541 | 148 |
| 1-33 | 891 | 200 |
| 1-34 | 1581 | 214 |
| 1-35 | 473 | 147 |
| 1-38 | 451 | 134 |
| 1-41 | 786 | 224 |
| 1-42 | - | 322 |

Although the specific embodiments of the present disclosure have been described above, those skilled in the art should understand that these are only embodiments, various changes or modifications can be made to these embodiments without departing from the principle and essence of the present invention. Therefore, the protection scope of the present disclosure is defined by the appended claims.

## Claims

1. A pyrazolone-fused pyrimidine compound represented by formula I, a pharmaceutically acceptable salt thereof, a solvate thereof or a solvate of the pharmaceutically acceptable salt thereof; 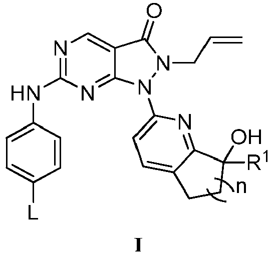 wherein, R¹ is hydrogen or methyl;
n is 1 or 2;
L is -(CH₂)ₘ-R², C₃-C₂₀ cycloalkyl, "C₃-C₂₀ cycloalkyl substituted by one or two R³", C₃-C₁₄ heterocycloalkyl or "C₃-C₁₄ heterocycloalkyl substituted by one or two R⁴";
m is 0, 1, 2, 3 or 4;
R², R³ and R⁴ are independently -CN, -NR²⁻¹R²⁻², -(C=O)R²⁻³, -(C=NR²⁻⁴)R²⁻⁵, -S(=O)₂R²⁻⁶, -OR²⁻⁷, - SR²⁻⁸ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁹;
R²⁻¹ and R²⁻² are independently hydrogen, -(C=O)R²⁻¹⁻¹, -S(=O)₂R²⁻¹⁻², -(C=NR²⁻¹⁻³)R²⁻¹⁻⁴ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻¹⁻⁵;
or, R²⁻¹ and R²⁻² together with the nitrogen atom they are attached to form a C₃-C₁₄ heterocycloalkyl optionally substituted by one, two or three R²⁻¹⁻⁶; one or more methylenes in the C₃-C₁₄ heterocycloalkyl optionally and independently substituted by oxygen atom, sulfur atom, sulfinyl, sulfonyl, carbonyl, vinylidene or -N(R²⁻¹⁻⁷)-; R²⁻¹⁻⁷ is independently C₁-C₇ alkyl or C₃-C₁₄ cycloalkyl;
R²⁻¹⁻¹, R²⁻¹⁻² and R²⁻¹⁻⁴ are independently hydrogen, -NR²⁻¹⁻¹⁻¹R²⁻¹⁻¹⁻² or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻¹⁻¹⁻³;
R²⁻¹⁻¹⁻¹ and R²⁻¹⁻¹⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻¹⁻¹⁻³ is independently halogen, hydroxyl, mercapto, cyano, amino, C₁-C₇ alkyl, C₁-C₇ alkoxy, C₁-C₇ alkylthio, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻¹⁻³ is selected from hydrogen, cyano, -OH, C₁-C₇ alkyl or C₁-C₇ alkoxy;
R²⁻¹⁻⁵ and R²⁻¹⁻⁶ are independently cyano, halogen, -NR²⁻¹⁻⁵⁻¹R²⁻¹⁻⁵⁻², -OR²⁻¹⁻⁵⁻³, -SR²⁻¹⁻⁵⁻⁴ or C₁-C₇ alkyl;
R²⁻¹⁻⁵⁻¹, R²⁻¹⁻⁵⁻², R²⁻¹⁻⁵⁻³ and R²⁻¹⁻⁵⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻³ is independently hydrogen, -NR²⁻³⁻¹R²⁻³⁻², -OR²⁻³⁻³ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻³⁻⁴;
R²⁻³⁻¹ and R²⁻³⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻³⁻¹ and R²⁻³⁻² together with the nitrogen atom they are attached to form a C₃-C₁₄ heterocycloalkyl optionally substituted by one, two or three R²⁻³⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻³⁻¹⁻²)-; R²⁻³⁻¹⁻² is independently C₁-C₇ alkyl or C₃-C₁₄ cycloalkyl;
R²⁻³⁻¹⁻¹ is independently cyano, halogen, -NR²⁻³⁻¹⁻¹⁻¹R²⁻³⁻¹⁻¹⁻², -OR²⁻³⁻¹⁻¹⁻³, -SR²⁻³⁻¹⁻¹⁻⁴ or C₁-C₇ alkyl;
R²⁻³⁻¹⁻¹⁻¹, R²⁻³⁻¹⁻¹⁻², R²⁻³⁻¹⁻¹⁻³ and R²⁻³⁻¹⁻¹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻³⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻³⁻⁴ is independently cyano, halogen, -NR²⁻³⁻⁴⁻¹R^{2-3-4-2,} -OR^{2-3-4-3,} -SR²⁻³⁻⁴⁻⁴ or C₁-C₇ alkyl;
R²⁻³⁻⁴⁻¹, R²⁻³⁻⁴⁻², R²⁻³⁻⁴⁻³ and R²⁻³⁻⁴⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁴ is independently hydrogen, -CN, -OR²⁻⁴⁻¹ or C₁-C₇ alkyl;
R²⁻⁴⁻¹ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁵ is independently -NR²⁻⁵⁻¹R^{2-5-2,} -OR²⁻⁵⁻³ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁵⁻⁴;
R²⁻⁵⁻¹ and R²⁻⁵⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻⁵⁻¹ and R²⁻⁵⁻² together with the nitrogen atom they are attached to form a C₃-C₁₄ heterocycloalkyl optionally substituted by one, two or three R²⁻⁵⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻⁵⁻¹⁻²)-; R²⁻⁵⁻¹⁻² is independently C₁-C₇ alkyl or C₃-C₁₄ cycloalkyl;
R²⁻⁵⁻¹⁻¹ is independently cyano, halogen, -NR²⁻⁵⁻¹⁻¹⁻¹R^{2-S-1-1-2}, -OR²⁻⁵⁻¹⁻¹⁻³, -SR²⁻⁵⁻¹⁻¹⁻⁴ or C₁-C₇ alkyl;
R²⁻⁵⁻¹⁻¹⁻¹, R²⁻⁵⁻¹⁻¹⁻², R²⁻⁵⁻¹⁻¹⁻³ and R²⁻⁵⁻¹⁻¹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁵⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁵⁻⁴ is independently cyano, halogen, -NR²⁻⁵⁻⁴⁻¹R^{2-5-4-2,} -OR^{2-5-4-3,} -SR²⁻⁵⁻⁴⁻⁴ or C₁-C₇ alkyl;
R²⁻⁵⁻⁴⁻¹, R²⁻⁵⁻⁴⁻², R²⁻⁵⁻⁴⁻³ and R²⁻⁵⁻⁴⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁶ is independently -NR²⁻⁶⁻¹R²⁻⁶⁻², -OR²⁻⁶⁻³ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁶⁻⁴;
R²⁻⁶⁻¹ and R²⁻⁶⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻⁶⁻¹ and R²⁻⁶⁻² together with the nitrogen atom they are attached to form a C₃-C₁₄ heterocycloalkyl optionally substituted by one, two or three R²⁻⁶⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻⁶⁻¹⁻²)-; R²⁻⁶⁻¹⁻² is independently C₁-C₇ alkyl or C₃-C₁₄ cycloalkyl;
R²⁻⁶⁻¹⁻¹ is independently cyano, halogen, -NR²⁻⁶⁻¹⁻¹⁻¹R²⁻⁶⁻¹⁻¹⁻², -OR²⁻⁶⁻¹⁻¹⁻³, -SR²⁻⁶⁻¹⁻¹⁻⁴ or C₁-C₇ alkyl;
R²⁻⁶⁻¹⁻¹⁻¹, R²⁻⁶⁻¹⁻¹⁻², R²⁻⁶⁻¹⁻¹⁻³ and R²⁻⁶⁻¹⁻¹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁶⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁶⁻⁴ is independently cyano, halogen, -NR²⁻⁶⁻⁴⁻¹R²⁻⁶⁻⁴⁻², -OR²⁻⁶⁻⁴⁻³, -SR²⁻⁶⁻⁴⁻⁴ or C₁-C₇ alkyl;
R²⁻⁶⁻⁴⁻¹, R²⁻⁶⁻⁴⁻², R²⁻⁶⁻⁴⁻³ and R²⁻⁶⁻⁴⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁷ and R²⁻⁸ are independently hydrogen or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁷⁻¹;
R²⁻⁷⁻¹ is independently cyano, halogen, -NR²⁻⁷⁻¹⁻¹R²⁻⁷⁻¹⁻², -OR²⁻⁷⁻¹⁻³, -SR²⁻⁷⁻¹⁻⁴ or C₁-C₇ alkyl;
R²⁻⁷⁻¹⁻¹, R²⁻⁷⁻¹⁻², R²⁻⁷⁻¹⁻³ and R²⁻⁷⁻¹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹ is independently -OR²⁻⁹⁻¹, -NR²⁻⁹⁻²R²⁻⁹⁻³, -SR²⁻⁹⁻⁴, halogen, cyano, -(C=O)R²⁻⁹⁻⁵, -(C=NR²⁻⁹⁻⁶)R²⁻⁹⁻⁷, -S(=O)₂R²⁻⁹⁻⁸, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻¹, R²⁻⁹⁻², R²⁻⁹⁻³ and R²⁻⁹⁻⁴ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁵ is independently hydrogen, -NR²⁻⁹⁻⁵⁻¹R²⁻⁹⁻⁵⁻², -OR²⁻⁹⁻⁵⁻³ or "C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl" optionally substituted by one, two or three R²⁻⁹⁻⁵⁻⁴;
R²⁻⁹⁻⁵⁻¹, R²⁻⁹⁻⁵⁻² and R²⁻⁹⁻⁵⁻³ are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁵⁻⁴ is independently hydrogen, halogen, hydroxyl, mercapto, cyano, amino, C₁-C₇ alkyl, C₁-C₇ alkoxy, C₁-C₇ alkylthio, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁶ is independently hydrogen, -CN, -OR²⁻⁹⁻⁶⁻¹ or C₁-C₇ alkyl;
R²⁻⁹⁻⁶⁻¹ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁷ is independently -NR²⁻⁹⁻⁷⁻¹R²⁻⁹⁻⁷⁻², -OR²⁻⁹⁻⁷⁻³, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁷⁻¹ and R²⁻⁹⁻⁷⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻⁹⁻⁷⁻¹ and R²⁻⁹⁻⁷⁻² together with the nitrogen atom they are attached to form a C₃-C₁₄ heterocycloalkyl optionally substituted by one, two or three R²⁻⁹⁻⁷⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻⁹⁻⁷⁻¹⁻²)-; R²⁻⁹⁻⁷⁻¹⁻² is independently C₁-C₇ alkyl or C₃-C₁₄ cycloalkyl;
R²⁻⁹⁻⁷⁻¹⁻¹ is independently halogen, hydroxyl, mercapto, cyano, amino, C₁-C₇ alkyl, C₁-C₇ alkoxy, C₁-C₇ alkylthio, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁷⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁸ is independently -NR²⁻⁹⁻⁸⁻¹R²⁻⁹⁻⁸⁻², -OR²⁻⁹⁻⁸⁻³, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁸⁻¹ and R²⁻⁹⁻⁸⁻² are independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
or, R²⁻⁹⁻⁸⁻¹ and R²⁻⁹⁻⁸⁻² together with the nitrogen atom they are attached to form a C₃-C₁₄ heterocycloalkyl optionally substituted by one, two or three R²⁻⁹⁻⁸⁻¹⁻¹; one or more methylenes in the C₃-C₁₄ heterocycloalkyl optionally and independently substituted by oxygen atom, sulfur atom, sulfinyl, sulfonyl, carbonyl, vinylidene or -N(R²⁻⁹⁻⁸⁻¹⁻²)-; R²⁻⁹⁻⁸⁻¹⁻² is independently C₁-C₇ alkyl or C₃-C₁₄ cycloalkyl;
R²⁻⁹⁻⁸⁻¹⁻¹ is independently halogen, hydroxyl, mercapto, cyano, amino, C₁-C₇ alkyl, C₁-C₇ alkoxy, C₁-C₇ alkylthio, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
R²⁻⁹⁻⁸⁻³ is independently hydrogen, C₁-C₇ alkyl, C₂-C₇ alkenyl, C₂-C₇ alkynyl, C₃-C₁₄ cycloalkyl, C₃-C₁₄ heterocycloalkyl, C₆-C₁₀ aryl or C₁-C₇ heteroaryl;
in any one of the above cases, the heteroatoms in the C₃-C₁₄ heterocycloalkyl, C₁-C₇ heteroaryl are independently selected from one or more of boron, silicon, oxygen, sulfur, selenium, nitrogen and phosphorus; the number of heteroatoms is independently 1, 2, 3 or 4.

2. The pyrazolone-fused pyrimidine compound represented by formula I according to claim 1, the pharmaceutically acceptable salt thereof, the solvate thereof or the solvate of the pharmaceutically acceptable salt thereof, wherein, when L is C₃-C₂₀ cycloalkyl substituted by one or two R³, the C₃-C₂₀ cycloalkyl is C₃-C₂₀ monocyclic cycloalkyl, C₃-C₂₀ spiro cycloalkyl, C₃-C₂₀ fused cycloalkyl or C₃-C₂₀ bridged cycloalkyl;
and/or, when L is C₃-C₂₀ cycloalkyl substituted by one or two R³, the C₃-C₂₀ cycloalkyl is C₃-C₂₀ saturated cycloalkyl;
and/or, when R²⁻¹ and R²⁻² are independently C₁-C₇ alkyl, the C₁-C₇ alkyl is C₁-C₃ alkyl;
and/or, when L is C₃-C₁₄ heterocycloalkyl, the C₃-C₁₄ heterocycloalkyl is C₃-C₁₄ monocyclic heterocycloalkyl, C₃-C₁₄ spiro heterocycloalkyl, C₃-C₁₄ fused heterocycloalkyl or C₃-C₁₄ bridged heterocycloalkyl;
and/or, when L is C₃-C₁₄ heterocycloalkyl, the C₃-C₁₄ heterocycloalkyl is C₃-C₁₄ saturated heterocycloalkyl;
and/or, when L is C₃-C₁₄ heterocycloalkyl, the heteroatom of the C₃-C₁₄ heterocycloalkyl is not substituted;
and/or, when L is C₃-C₁₄ heterocycloalkyl, the methylene in the C₃-C₁₄ heterocycloalkyl is not substituted or replaced;
and/or, when L is C₃-C₁₄ heterocycloalkyl substituted by one or two R⁴, the C₃-C₁₄ heterocycloalkyl is C₃-C₁₄ monocyclic heterocycloalkyl, C₃-C₁₄ spiro heterocycloalkyl, C₃-C₁₄ fused heterocycloalkyl or C₃-C₁₄ bridged heterocycloalkyl;
and/or, when L is C₃-C₁₄ heterocycloalkyl substituted by one or two R⁴, the C₃-C₁₄ heterocycloalkyl is C₃-C₁₄ saturated heterocycloalkyl;
and/or, when L is C₃-C₁₄ heterocycloalkyl optionally substituted by one or two R⁴, the heteroatom of the C₃-C₁₄ heterocycloalkyl is not substituted except R⁴;
and/or, when L is C₃-C₁₄ heterocycloalkyl optionally substituted by one or two R⁴, the methylene in the C₃-C₁₄ heterocycloalkyl is not substituted or replaced;
and/or, when R⁴ is C₁-C₇ alkyl optionally substituted by one R²⁻⁹, the C₁-C₇ alkyl is C₁-C₃ alkyl;
and/or, when R⁴ is C₃-C₁₄ cycloalkyl, the C₃-₁₄ cycloalkyl is C₃-C₇ cycloalkyl;
and/or, when R⁴ is C₃-C₁₄ cycloalkyl, the C₃-C₁₄ cycloalkyl is C₃-C₁₄ saturated cycloalkyl;
and/or, when R⁴ is C₃-C₁₄ heterocycloalkyl optionally substituted by one R²⁻⁹, the C₃-C₁₄ heterocycloalkyl is C₃-C₇ heterocycloalkyl;
and/or, when R⁴ is C₃-C₁₄ heterocycloalkyl optionally substituted by one R²⁻⁹, the C₃-C₁₄ heterocycloalkyl is C₃-C₁₄ saturated heterocycloalkyl;
and/or, when R⁴ is C₃-C₁₄ heterocycloalkyl optionally substituted by one R²⁻⁹, the heteroatom of the C₃-C₁₄ heterocycloalkyl is not substituted except R²⁻⁹;
and/or, when R⁴ is C₃-C₁₄ heterocycloalkyl optionally substituted by one R²⁻⁹, the methylene in the C₃-C₁₄ heterocycloalkyl is not substituted or replaced;
and/or, when R²⁻⁹ is independently C₃-C₁₄ cycloalkyl, the C₃-₁₄ cycloalkyl is C₃-C₁₄ monocyclic cycloalkyl;
and/or, when R²⁻⁹ is independently C₃-C₁₄ cycloalkyl, the C₃-C₁₄ cycloalkyl is C₃-C₁₄ saturated cycloalkyl;
and/or, when R²⁻⁹⁻² and R²⁻⁹⁻³ are independently C₁-C₇ alkyl, the C₁-C₇ alkyl is C₁-C₃ alkyl;
and/or, when R²⁻³ is independently C₃-C₁₄ heterocycloalkyl, the C₃-C₁₄ heterocycloalkyl is C₃-C₁₄ monocyclic heterocycloalkyl;
and/or, when R²⁻³ is independently C₃-C₁₄ heterocycloalkyl, the C₃-C₁₄ heterocycloalkyl is C₃-C₁₄ saturated heterocycloalkyl;
and/or, when R²⁻³ is independently C₃-C₁₄ heterocycloalkyl, the heteroatom of the C₃-C₁₄ heterocycloalkyl is not substituted;
and/or, when R²⁻³ is independently C₃-C₁₄ heterocycloalkyl, the methylene in the C₃-C₁₄ heterocycloalkyl is not substituted or replaced;
and/or, when R² is C₃-C₁₄ heterocycloalkyl optionally substituted by one R²⁻⁹, the C₃-C₁₄ monocyclic heterocycloalkyl is "C₃-C₉ monocyclic heterocycloalkyl having one or two heteroatoms selected from one or two of N, O and S";
and/or, when R² is C₃-C₁₄ heterocycloalkyl substituted by one R²⁻⁹, the C₃-C₁₄ heterocycloalkyl is C₃-C₁₄ saturated heterocycloalkyl;
and/or, when R² is C₃-C₁₄ heterocycloalkyl optionally substituted by one R²⁻⁹, the heteroatom of the C₃-C₁₄ heterocycloalkyl is not substituted except R²⁻⁹;
and/or, when R² is C₃-C₁₄ heterocycloalkyl optionally substituted by one R²⁻⁹, the methylene in the C₃-C₁₄ heterocycloalkyl is not substituted or replaced;
and/or, when R²⁻⁹ is independently C₁-C₇ alkyl, the C₁-C₇ alkyl is C₁-C₃ alkyl;
and/or, the ratio of each isomer in the pyrazolone-fused pyrimidine compound represented by formula I is equal;
and/or, the atoms in the pyrazolone-fused pyrimidine compound represented by formula I, the pharmaceutically acceptable salt thereof, the solvate thereof or the solvate of the pharmaceutically acceptable salt thereof all exist in their natural abundance.

3. The pyrazolone-fused pyrimidine compound represented by formula I according to claim 2, the pharmaceutically acceptable salt thereof, the solvate thereof or the solvate of the pharmaceutically acceptable salt thereof, wherein, when L is C₃-C₂₀ cycloalkyl substituted by one or two R³, the C₃-C₂₀ cycloalkyl is C₃-C₂₀ monocyclic cycloalkyl; the C₃-C₂₀ monocyclic cycloalkyl is cyclopropyl, cyclobutyl, cyclopentyl or cyclohexyl;
and/or, when L is C₃-C₂₀ cycloalkyl substituted by one or two R³, the C₃-C₂₀ cycloalkyl is C₃-C₂₀ bridged cycloalkyl; the C₃-C₂₀ bridged cycloalkyl is 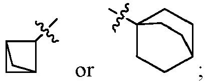
and/or, when R²⁻¹ and R²⁻² are independently C₁-C₇ alkyl, the C₁-C₇ alkyl is methyl, ethyl, *n*-propyl or isopropyl;
and/or, when L is C₃-C₁₄ heterocycloalkyl, the C₃-C₁₄ heterocycloalkyl is C₃-C₁₄ monocyclic heterocycloalkyl; the C₃-C₁₄ monocyclic heterocycloalkyl is 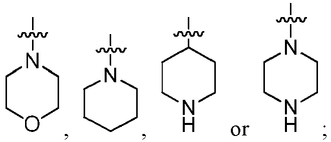
and/or, when L is C₃-C₁₄ heterocycloalkyl, the C₃-C₁₄ heterocycloalkyl is C₃-C₁₄ spiro heterocycloalkyl; the C₃-C₁₄ spiro heterocycloalkyl is 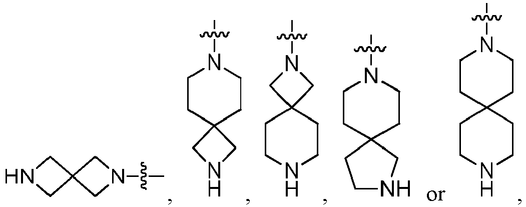 can also be 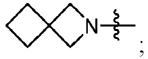
and/or, when L is independently C₃-C₁₄ heterocycloalkyl, the C₃-C₁₄ heterocycloalkyl is C₃-C₁₄ fused heterocycloalkyl; the C₃-C₁₄ fused heterocycloalkyl is 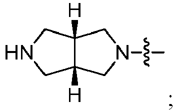
and/or, when L is C₃-C₁₄ heterocycloalkyl, the C₃-C₁₄ heterocycloalkyl is C₃-C₁₄ bridged heterocycloalkyl; the C₃-C₁₄ bridged heterocycloalkyl is 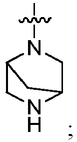
and/or, when L is C₃-C₁₄ heterocycloalkyl substituted by one or two R⁴, the C₃-C₁₄ heterocycloalkyl is C₃-C₁₄ monocyclic heterocycloalkyl; the C₃-C₁₄ monocyclic heterocycloalkyl is 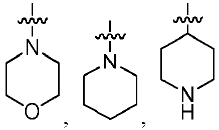 or 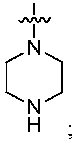
and/or, when L is C₃-C₁₄ heterocycloalkyl substituted by one or two R⁴, the C₃-C₁₄ heterocycloalkyl is C₃-C₁₄ spiro heterocycloalkyl; the C₃-C₁₄ spiro heterocycloalkyl is 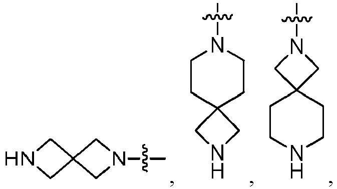 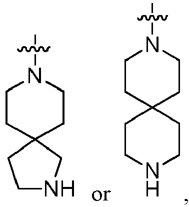 can also be 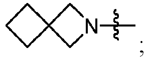
and/or, when L is C₃-C₁₄ heterocycloalkyl substituted by one or two R⁴, the C₃-C₁₄ heterocycloalkyl is C₃-C₁₄ fused heterocycloalkyl; the C₃-C₁₄ fused heterocycloalkyl is 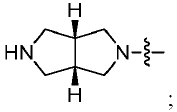
and/or, when L is C₃-C₁₄ heterocycloalkyl substituted by one or two R⁴, the C₃-C₁₄ heterocycloalkyl is C₃-C₁₄ bridged heterocycloalkyl; the C₃-C₁₄ bridged heterocycloalkyl is 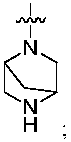
and/or, when R⁴ is C₁-C₇ alkyl optionally substituted by one R²⁻⁹, the C₁-C₇ alkyl is methyl, ethyl, n-propyl or isopropyl;
and/or, when R⁴ is C₃-C₁₄ cycloalkyl, the C₃-C₁₄ cycloalkyl is C₃-C₇ monocyclic cycloalkyl, for example cyclobutyl;
and/or, when R⁴ is C₃-C₁₄ heterocycloalkyl substituted by one R²⁻⁹, the C₃-C₁₄ heterocycloalkyl is C₃-C₇ monocyclic heterocycloalkyl, for example azetidinyl, for example 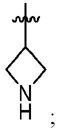
and/or, when R²⁻⁹ is independently C₃-C₁₄ cycloalkyl, the C₃-C₁₄ cycloalkyl is cyclopropyl;
and/or, when R²⁻⁹⁻² and R²⁻⁹⁻³ are independently C₁-C₇ alkyl, the C₁-C₇ alkyl is methyl, ethyl, *n*-propyl or isopropyl;
and/or, when R²⁻³ is independently C₃-C₁₄ heterocycloalkyl, the C₃-C₁₄ heterocycloalkyl is 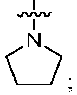
and/or, when R² is C₃-C₁₄ heterocycloalkyl optionally substituted by one R²⁻⁹, the C₃-C₁₄ monocyclic heterocycloalkyl is 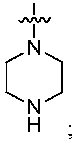
and/or, when R²⁻⁹ is independently C₁-C₇ alkyl, the C₁-C₇ alkyl is methyl, ethyl, *n*-propyl or isopropyl.

4. The pyrazolone-fused pyrimidine compound represented by formula I according to claim 3, the pharmaceutically acceptable salt thereof, the solvate thereof or the solvate of the pharmaceutically acceptable salt thereof, wherein, when L is C₃-C₂₀ cycloalkyl substituted by one R³, R³ is -NR²⁻¹R²⁻²; the "C₃-C₂₀ cycloalkyl substituted by one R³" is 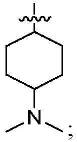
and/or, when L is C₃-C₁₄ heterocycloalkyl substituted by one R⁴, R⁴ is C₁-C₇ alkyl optionally substituted by one R²⁻⁹, the "C₃-C₁₄ heterocycloalkyl substituted by one R⁴" is 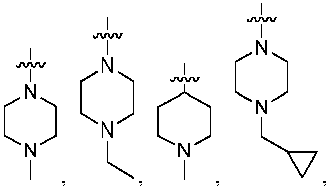 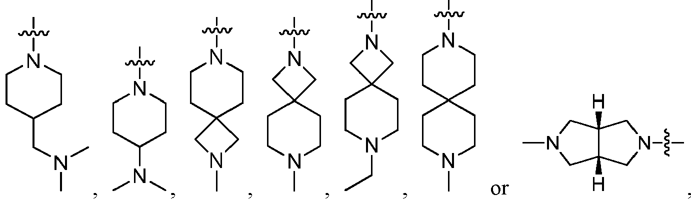 , and can also be 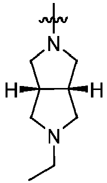 or 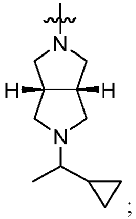
and/or, when L is C₃-C₁₄ heterocycloalkyl substituted by one R⁴, R⁴ is C₃-C₁₄ cycloalkyl optionally substituted by one R²⁻⁹, the "C₃-C₁₄ heterocycloalkyl substituted by one R⁴" is 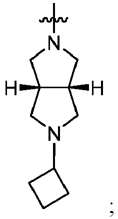
and/or, when L is C₃-C₁₄ heterocycloalkyl substituted by one R⁴, R⁴ is C₃-C₁₄ heterocycloalkyl optionally substituted by one R²⁻⁹, the "C₃-C₁₄ heterocycloalkyl substituted by one R⁴" is 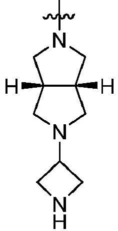 or 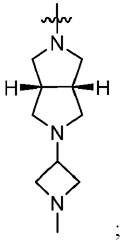
and/or, when L is C₃-C₁₄ heterocycloalkyl substituted by one R⁴, R⁴ is -NR²⁻¹R²⁻²; the "C₃-C₁₄ heterocycloalkyl substituted by one R⁴" is

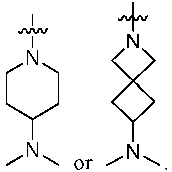

5. The pyrazolone-fused pyrimidine compound represented by formula I according to claim 1, the pharmaceutically acceptable salt thereof, the solvate thereof or the solvate of the pharmaceutically acceptable salt thereof, wherein, R¹ is hydrogen or methyl;
and/or, n is 1 or 2;
and/or, L is -(CH₂)ₘ-R², "C₃-C₂₀ cycloalkyl substituted by one R³", C₃-C₁₄ heterocycloalkyl or "C₃-C₁₄ heterocycloalkyl substituted by one R⁴"; m is 1 or 2, and can also be 3;
and/or, R², R³ and R⁴ are independently -NR²⁻¹R²⁻², -(C=O)R²⁻³ or "C₁-C₇ alkyl or C₃-C₁₄ heterocycloalkyl" optionally substituted by one R²⁻⁹; R²⁻¹ and R²⁻² are independently C₁-C₇ alkyl; R²⁻³ is independently C₃-C₁₄ heterocycloalkyl; R²⁻⁹ is independently -NR²⁻⁹⁻²R²⁻⁹⁻³, C₁-C₇ alkyl or C₃-C₁₄ cycloalkyl; R²⁻⁹⁻² and R²⁻⁹⁻³ are independently C₁-C₇ alkyl;
and/or, the pyrazolone-fused pyrimidine compounds represented by formula I are not

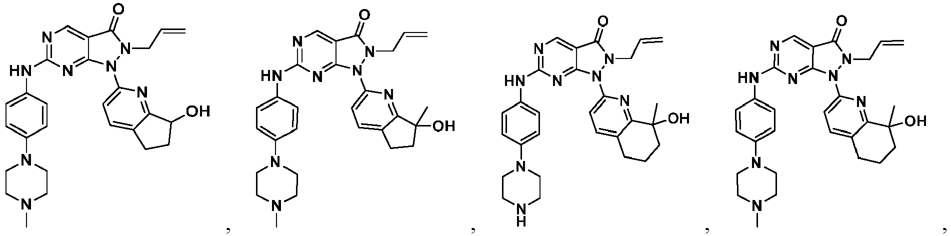

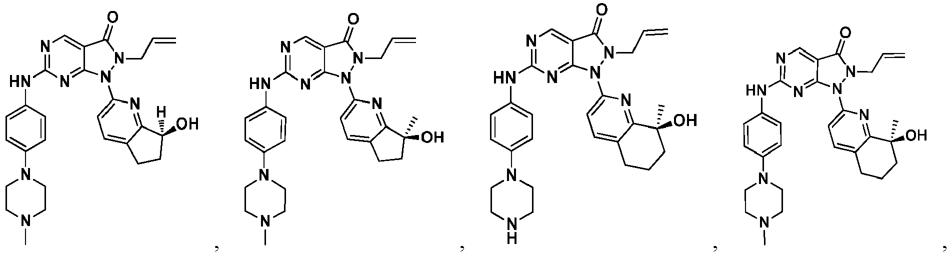

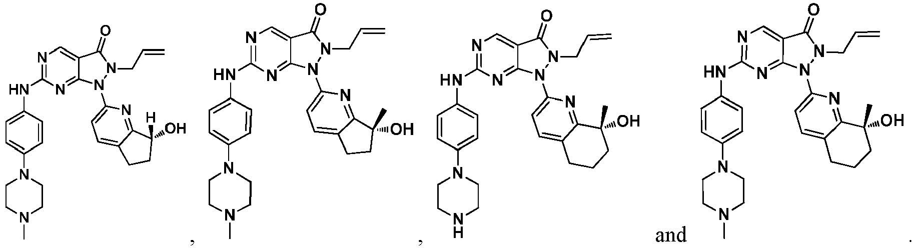

6. The pyrazolone-fused pyrimidine compound represented by formula I according to claim 5, the pharmaceutically acceptable salt thereof, the solvate thereof or the solvate of the pharmaceutically acceptable salt thereof, wherein, L is "C₃-C₂₀ cycloalkyl substituted by one R³", C₃-C₁₄ heterocycloalkyl or "C₃-C₁₄ heterocycloalkyl substituted by one R⁴";
and/or, R³ and R⁴ are independently -NR²⁻¹R²⁻² or "C₁-C₇ alkyl optionally substituted by one R²⁻⁹"; R²⁻¹ and R²⁻² are independently C₁-C₇ alkyl; R²⁻⁹ is independently -NR²⁻⁹⁻²R²⁻⁹⁻³ or C₃-C₁₄ cycloalkyl; R²⁻⁹⁻² and R²⁻⁹⁻³ are independently C₁-C₇ alkyl.

7. The pyrazolone-fused pyrimidine compound represented by formula I according to claim 2, the pharmaceutically acceptable salt thereof, the solvate thereof or the solvate of the pharmaceutically acceptable salt thereof, wherein, L is -(CH₂)ₘ-R², "C₃-C₁₄ fused heterocycloalkyl substituted by one or two R⁴" or "C₃-C₁₄ spiro heterocycloalkyl substituted by one or two R⁴";
when L is C₃-C₁₄ fused heterocycloalkyl, the C₃-C₁₄ fused heterocycloalkyl is C₅-C₁₄ fused heterocycloalkyl;
when L is C₃-C₁₄ spiro heterocycloalkyl, the C₃-C₁₄ spiro heterocycloalkyl is C₅-C₁₄ spiro heterocycloalkyl.

8. The pyrazolone-fused pyrimidine compound represented by formula I according to claim 7, the pharmaceutically acceptable salt thereof, the solvate thereof or the solvate of the pharmaceutically acceptable salt thereof, wherein, R² is -NR²⁻¹R²⁻²;
and/or, R⁴ is -NR²⁻¹R²⁻² or "C₁-C₇ alkyl, C₃-C₁₄ cycloalkyl or C₃-C₁₄ heterocycloalkyl" optionally substituted by one, two or three R²⁻⁹;
and/or, R²⁻¹ and R²⁻² are independently C₁-C₇ alkyl;
and/or, R²⁻⁹ is independently C₁-C₇ alkyl or C₃-C₁₄ heterocycloalkyl.

9. The pyrazolone-fused pyrimidine compound represented by formula I according to claim 8, the pharmaceutically acceptable salt thereof, the solvate thereof or the solvate of the pharmaceutically acceptable salt thereof, wherein, L is

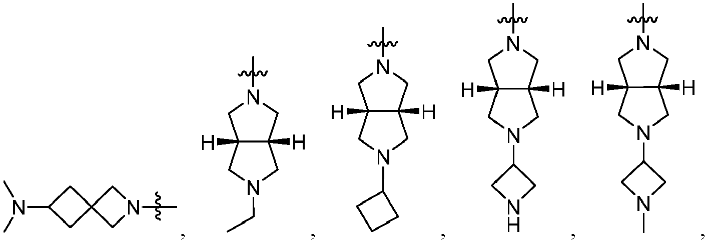

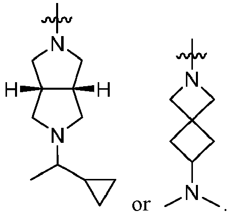

10. The pyrazolone-fused pyrimidine compound represented by formula I according to claim 1, the pharmaceutically acceptable salt thereof, the solvate thereof or the solvate of the pharmaceutically acceptable salt thereof, wherein, the pyrazolone-fused pyrimidine compound represented by formula I is any of the following schemes:
scheme (1):
R¹ is hydrogen or methyl;
n is 1 or 2;
L is -(CH₂)ₘ-R², C₃-C₂₀ cycloalkyl, "C₃-C₂₀ cycloalkyl substituted by one or two R³", C₃-C₁₄ heterocycloalkyl or "C₃-C₁₄ heterocycloalkyl substituted by one or two R⁴";
m is 1, 2, 3 or 4;
R², R³ and R⁴ are independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻².
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl;
scheme (2):
R¹ is hydrogen or methyl;
n is 1 or 2;
L is -(CH₂)ₘ-R², "C₃-C₂₀ cycloalkyl substituted by one R3", C₃-C₁₄ heterocycloalkyl or "C₃-C₁₄ heterocycloalkyl substituted by one R⁴"; m is 1 or 2, and can also be 3;
R², R³ and R⁴ are independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl;
scheme (3):
R¹ is hydrogen or methyl;
n is 1 or 2;
L is "C₃-C₂₀ cycloalkyl substituted by one R³", C₃-C₁₄ heterocycloalkyl or "C₃-C₁₄ heterocycloalkyl substituted by one R⁴;
R³ and R⁴ are independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl;
scheme (4):
R¹ is hydrogen or methyl;
n is 1 or 2;
L is -(CH₂)ₘ-R², "C₃-C₁₄ fused heterocycloalkyl substituted by one or two R⁴" or "C₃-C₁₄ spiro heterocycloalkyl substituted by one or two R⁴";
when L is C₃-C₁₄ fused heterocycloalkyl, the C₃-C₁₄ fused heterocycloalkyl is C₅-C₁₄ fused heterocycloalkyl;
when L is C₃-C₁₄ spiro heterocycloalkyl, the C₃-C₁₄ spiro heterocycloalkyl is C₅-C₁₄ spiro heterocycloalkyl;
R² is -NR²⁻¹R²⁻²;
R⁴ is -NR²⁻¹R²⁻² or "C₁-C₇ alkyl, C₃-C₁₄ cycloalkyl or C₃-C₁₄ heterocycloalkyl" optionally substituted by one, two or three R²⁻⁹;
scheme (5):
R¹ is hydrogen;
n is 1;
L is -(CH₂)ₘ-R², "C₃-C₁₄ fused heterocycloalkyl substituted by one or two R⁴" or "C₃-C₁₄ spiro heterocycloalkyl substituted by one or two R⁴";
when L is C₃-C₁₄ fused heterocycloalkyl, the C₃-C₁₄ fused heterocycloalkyl is C₅-C₁₄ fused heterocycloalkyl;
when L is C₃-C₁₄ spiro heterocycloalkyl, the C₃-C₁₄ spiro heterocycloalkyl is C₅-C₁₄ spiro heterocycloalkyl; R² is -NR²⁻¹R²⁻²;
R⁴ is -NR²⁻¹R²⁻² or "C₁-C₇ alkyl, C₃-C₁₄ cycloalkyl or C₃-C₁₄ heterocycloalkyl" optionally substituted by one, two or three R²⁻⁹;
R²⁻¹ and R²⁻² are independently C₁-C₇ alkyl;
R²⁻⁹ is independently C₁-C₇ alkyl or C₃-C₁₄ heterocycloalkyl;
scheme (6):
R¹ is hydrogen or methyl;
n is 1 or 2;
L is -(CH₂)ₘ-R², "C₃-C₂₀ cycloalkyl substituted by one or two R³" or "C₃-C₁₄ heterocycloalkyl substituted by one or two R⁴";
m is 1, 2, 3 or 4;
R², R³ and R⁴ are independently -NR²⁻¹R²⁻²;
R²⁻¹ and R²⁻² are independently hydrogen, C₁-C₇ alkyl, or C₃-C₁₄ cycloalkyl;
scheme (7):
R¹ is hydrogen or methyl;
n is 1 or 2;
L is -(CH₂)ₘ-R², "C₃-C₂₀ cycloalkyl substituted by one R3", "C₃-C₁₄ heterocycloalkyl substituted by one R⁴"; m is 1 or 2, and can also be 3;
R², R³ and R⁴ are independently -NR²⁻¹R²⁻²;
R²⁻¹ and R²⁻² are independently C₁-C₇ alkyl;
scheme (8):
R¹ is hydrogen or methyl;
n is 1 or 2;
L is "C₃-C₂₀ cycloalkyl substituted by one R³" or "C₃-C₁₄ heterocycloalkyl substituted by one R⁴;
R³ and R⁴ are independently -NR²⁻¹R²⁻²;
R²⁻¹ and R²⁻² are independently C₁-C₇ alkyl;
scheme (9):
R¹ is hydrogen or methyl;
n is 1 or 2;
L is C₃-C₂₀ cycloalkyl or "C₃-C₂₀ cycloalkyl substituted by one or two R³";
R³ is independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻¹ are independently hydrogen or C₁-C₇ alkyl;
R²⁻⁹⁻⁸ is independently -NR²⁻⁹⁻⁸⁻¹R²⁻⁹⁻⁸⁻²;
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl;
scheme (10):
R¹ is hydrogen or methyl;
n is 1 or 2;
L is C₃-C₂₀ cycloalkyl substituted by one R³;
R³ is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl;
scheme (11):
R¹ is hydrogen or methyl;
n is 1 or 2;
L is C₃-C₂₀ cycloalkyl substituted by one R³;
R³ is independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl;
scheme (12):
R¹ is hydrogen or methyl;
n is 1 or 2;
L is C₃-C₁₄ heterocycloalkyl or "C₃-C₁₄ heterocycloalkyl substituted by one or two R⁴"; the C₃-C₁₄ heterocycloalkyl is C₃-C₁₄ spiro heterocycloalkyl;
R⁴ is independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻².
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl;
scheme (13):
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ spiro heterocycloalkyl;
R⁴ is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl;
scheme (14):
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ spiro heterocycloalkyl;
R⁴ is independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl;
scheme (15):
R¹ is hydrogen or methyl;
n is 1 or 2;
L is C₃-C₁₄ heterocycloalkyl or "C₃-C₁₄ heterocycloalkyl substituted by one or two R⁴"; the C₃-C₁₄ heterocycloalkyl is C₃-C₁₄ fused heterocycloalkyl;
R⁴ is independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻².
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl;
scheme (16):
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ fused heterocycloalkyl;
R⁴ is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl;
scheme (17):
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ fused heterocycloalkyl;
R⁴ is independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl;
scheme (18):
R¹ is hydrogen or methyl;
n is 1 or 2;
L is C₃-C₁₄ heterocycloalkyl or "C₃-C₁₄ heterocycloalkyl substituted by one or two R⁴"; the C₃-C₁₄ heterocycloalkyl is C₃-C₁₄ bridged heterocycloalkyl;
R⁴ is independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl, or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²;
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻²;
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl;
scheme (19):
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ bridged heterocycloalkyl;
R⁴ is independently -NR²⁻¹R²⁻², -(C=O)R²⁻³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl;
scheme (20):
R¹ is hydrogen or methyl;
n is 1 or 2;
L is C₃-C₁₄ heterocycloalkyl or "C₃-C₁₄ heterocycloalkyl substituted by one R⁴"; the C₃-C₁₄ heterocycloalkyl is C₃-C₁₄ bridged heterocycloalkyl;
R⁴ is independently -NR²⁻¹R²⁻² or "C₁-C₇ alkyl optionally substituted by one R²⁻⁹";
R²⁻¹ and R²⁻² are independently C₁-C₇ alkyl;
R²⁻⁹ is independently -NR²⁻⁹⁻²R²⁻⁹⁻³ or C₃-C₁₄ cycloalkyl;
R²⁻⁹⁻² and R²⁻⁹⁻³ are independently C₁-C₇ alkyl;
scheme (21):
R¹ is hydrogen or methyl;
n is 1 or 2;
L is (CH₂)ₘ-R²;
m is 1, 2, 3 or 4;
R², R³ and R⁴ are independently -CN, -NR²⁻¹R²⁻², -(C=O)R²⁻³, -(C=NR²⁻⁴)R²⁻⁵, -S(=O)₂R²⁻⁶, -OR²⁻⁷ or "C₁-C₇ alkyl, C₃-C₁₄ cycloalkyl, C₃-C₁₄ heterocycloalkyl or C₁-C₇ heteroaryl" optionally substituted by one, two or three R²⁻⁹;
R²⁻¹ and R²⁻² are independently hydrogen, C₁-C₇ alkyl or C₃-C₁₄ cycloalkyl;
R²⁻³ is independently -NR²⁻³⁻¹R²⁻³⁻², -OR²⁻³⁻³, C₁-C₇ alkyl or C₃-C₁₄ heterocycloalkyl;
R²⁻³⁻¹ and R²⁻³⁻² are independently hydrogen or C₁-C₇ alkyl;
R²⁻³⁻³ is independently hydrogen or C₁-C₇ alkyl;
R²⁻⁴ is independently hydrogen, -CN or -OR²⁻⁴⁻¹;
R²⁻⁴⁻¹ is independently hydrogen or C₁-C₇ alkyl;
R²⁻⁵ is independently -NR²⁻⁵⁻¹R²⁻⁵⁻² or C₁-C₇ cycloalkyl;
R²⁻⁵⁻¹ and R²⁻⁵⁻² are independently hydrogen or C₁-C₇ alkyl;
R²⁻⁶ is independently -NR²⁻⁶⁻¹R²⁻⁶⁻²
R²⁻⁶⁻¹ and R²⁻⁶⁻² are independently hydrogen, C₁-C₇ alkyl, C₃-C₁₄ cycloalkyl or C₃-C₁₄ heterocycloalkyl;
R²⁻⁹ is independently -NR²⁻⁹⁻²R²⁻⁹⁻³, halogen, cyano, -(C=O)R²⁻⁹⁻⁵, -S(=O)₂R²⁻⁹⁻⁸, C₁-C₇ alkyl, C₃-C₁₄ cycloalkyl or C₃-C₁₄ heterocycloalkyl;
R²⁻⁹⁻² and R²⁻⁹⁻³ are independently C₁-C₇ alkyl;
R²⁻⁹⁻⁵ is independently hydrogen, -NR²⁻⁹⁻⁵⁻¹R²⁻⁹⁻⁵⁻², -OR²⁻⁹⁻⁵⁻³ or C₁-C₇ alkyl;
R²⁻⁹⁻⁵⁻¹, R²⁻⁹⁻⁵⁻² and R²⁻⁹⁻⁵⁻³ are independently hydrogen or C₁-C₇ alkyl;
R²⁻⁹⁻⁸ is independently -NR²⁻⁹⁻⁸⁻¹R²⁻⁹⁻⁸⁻²;
R²⁻⁹⁻⁸⁻¹ and R²⁻⁹⁻⁸⁻² are independently hydrogen, C₁-C₇ alkyl, C₃-C₁₄ cycloalkyl or C₃-C₁₄ heterocycloalkyl;
scheme (22):
R¹ is hydrogen or methyl;
n is 1 or 2;
L is (CH₂)ₘ-R²; m is 1 or 2, and can also be 3;
R² is independently -NR²⁻¹R²⁻², -(C=O)R²³ or "C₁-C₇ alkyl or C₃-C₁₄ heterocycloalkyl" optionally substituted by one R²⁻⁹;
R²⁻¹ and R²⁻² are independently C₁-C₇ alkyl;
R²⁻³ is independently C₃-C₁₄ heterocycloalkyl;
R²⁻⁹ is independently -NR²⁻⁹⁻²R²⁻⁹⁻³, C₁-C₇ alkyl or C₃-C₁₄ cycloalkyl;
R²⁻⁹⁻² and R²⁻⁹⁻³ are independently C₁-C₇ alkyl.

11. The pyrazolone-fused pyrimidine compound represented by formula I according to claim 1, the pharmaceutically acceptable salt thereof, the solvate thereof or the solvate of the pharmaceutically acceptable salt thereof, wherein, the pyrazolone-fused pyrimidine compound represented by formula I is any of the following structures: 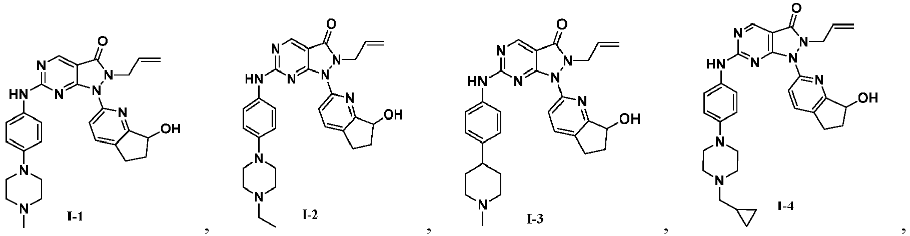 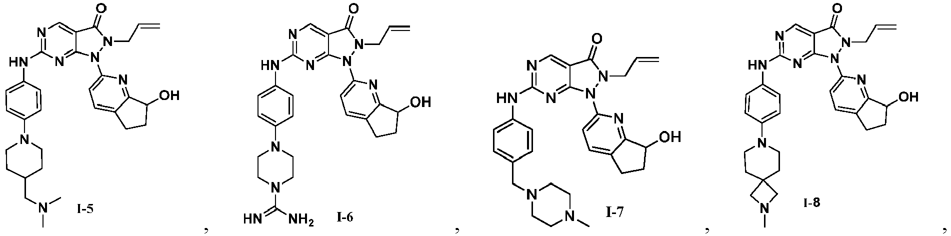 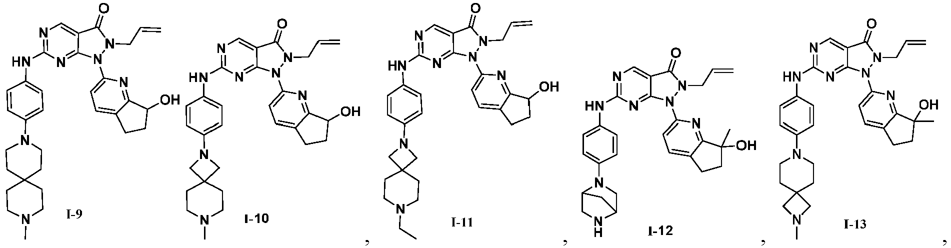 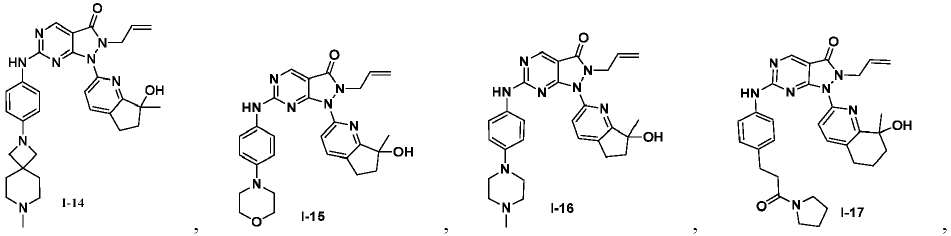 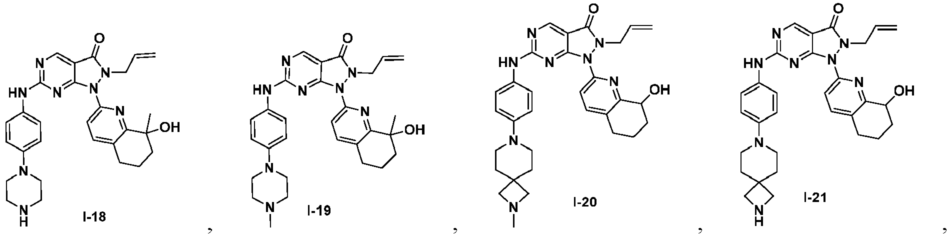 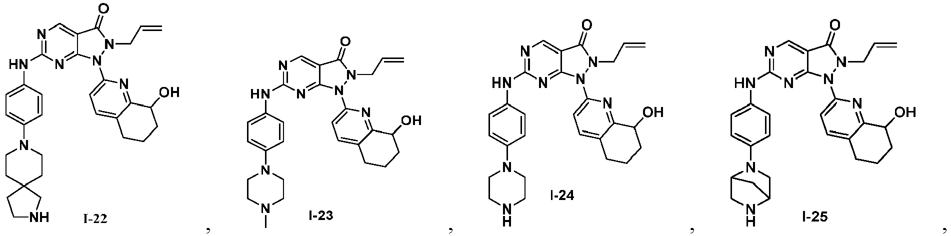 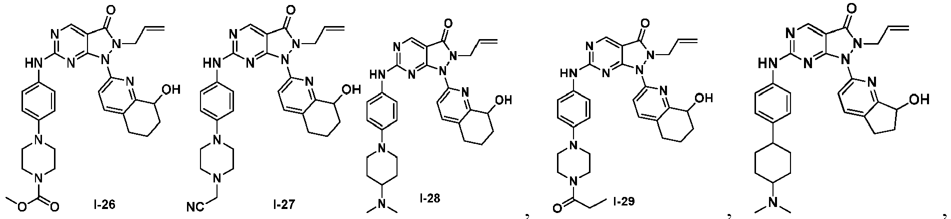 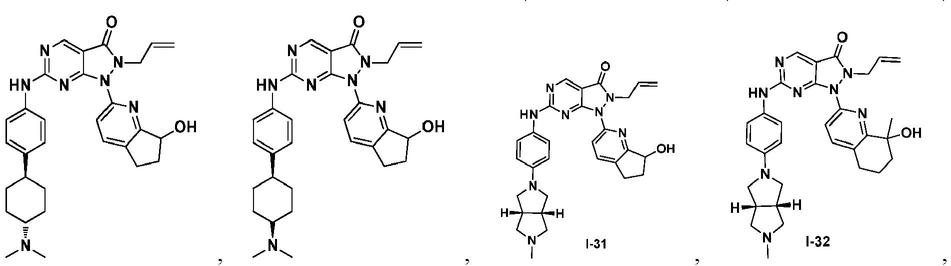 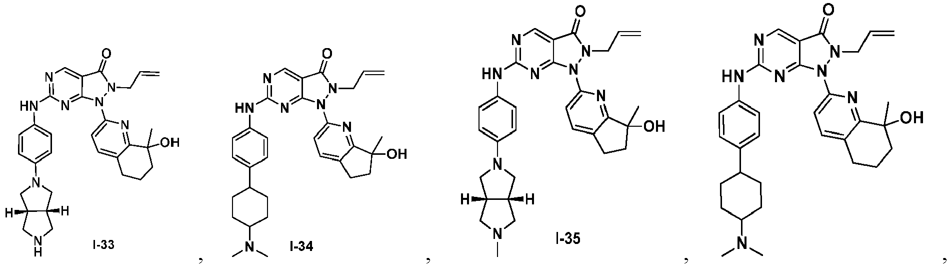 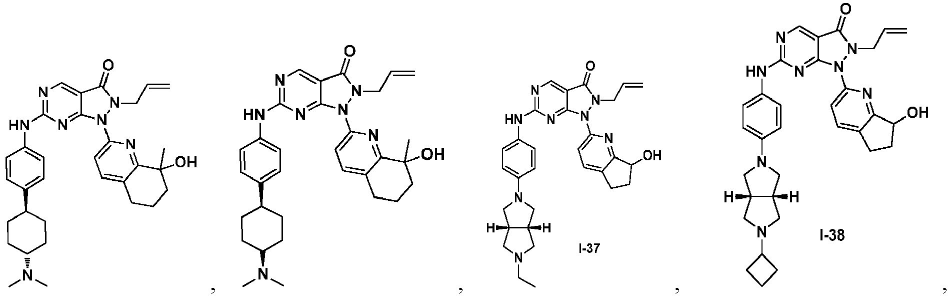 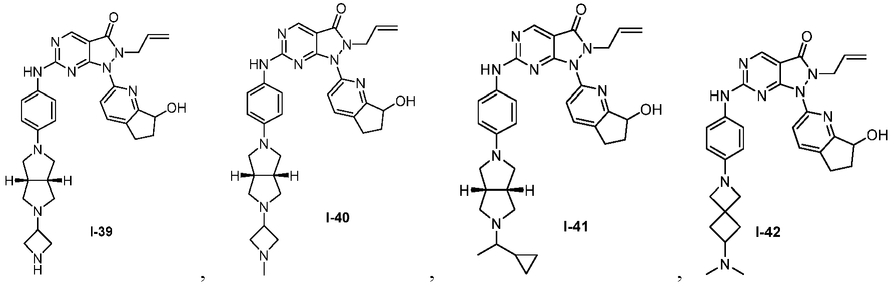 and

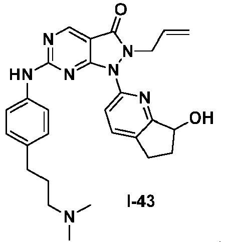

12. The pyrazolone-fused pyrimidine compound represented by formula I according to claim 1, the pharmaceutically acceptable salt thereof, the solvate thereof or the solvate of the pharmaceutically acceptable salt thereof, wherein, the pyrazolone-fused pyrimidine compound represented by formula I is any of the following compounds: 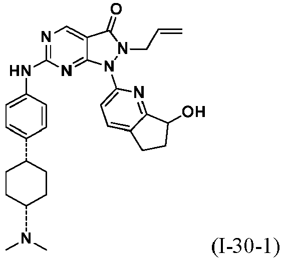 with a retention time of 9.66 min under the following conditions: Agilent 1260 high performance liquid chromatograph; mobile phase A: water (0.1 % formic acid), mobile phase B: acetonitrile; column time: 15 min; column type: Waters' Xselect, 5 µm, 4.6×250 mm; gradient elution, 5 % mobile phase B→50 % mobile phase B; 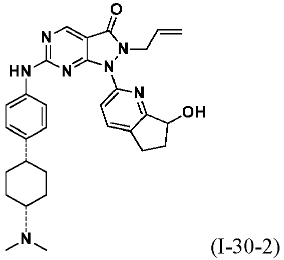 with a retention time of 10.04 min under the following conditions: Agilent 1260 high performance liquid chromatograph; mobile phase A: water (0.1 % formic acid), mobile phase B: acetonitrile; column time: 15 min; column type: Waters' Xselect, 5 µm, 4.6×250 mm; gradient elution, 5 % mobile phase B→50 % mobile phase B; 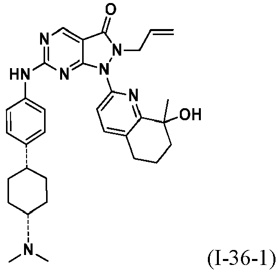 with a retention time of 7.80 min under the following conditions: Agilent 1260 high performance liquid chromatograph; mobile phase A: water (0.1 % formic acid), mobile phase B: acetonitrile; column time: 15 min; column type: Waters' Xselect, 5 µm, 4.6×250 mm; gradient elution, 5 % mobile phase B→95 % mobile phase B; 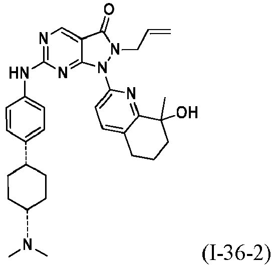 with a retention time of 7.97 min under the following conditions: Agilent 1260 high performance liquid chromatograph; mobile phase A: water (0.1 % formic acid), mobile phase B: acetonitrile; column time: 15 min; column type: Waters' Xselect, 5 µm, 4.6×250 mm; gradient elution, 5 % mobile phase B→95 % mobile phase B;
wherein, 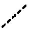 means that the *cis-trans* conformation is uncertain.

13. A preparation method of the pyrazolone-fused pyrimidine compound represented by formula I according to at least one of claims 1-12, the method comprises the following steps: step I, a C-N coupling reaction is carried out between compound 1A and compound 1B to obtain compound 1C; step II, an intermediate sulfoxide with higher activity is formed by compound 1C under the action of an oxidant, and then a substitution reaction is carried out between the intermediate sulfoxide with compound ID to obtain compound I;

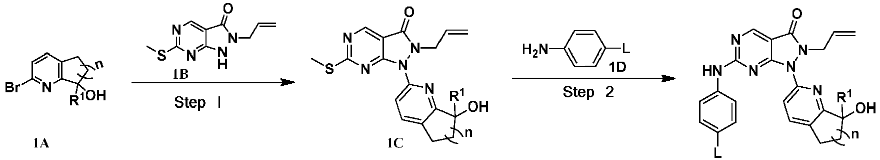

14. A compound represented by formula 1C: 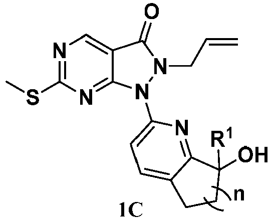 wherein, R¹ is hydrogen or methyl; n is 1 or 2;
preferably any of the following structures: 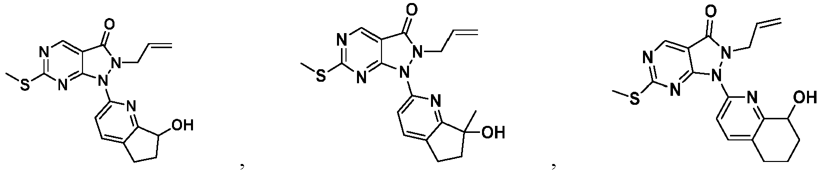 and

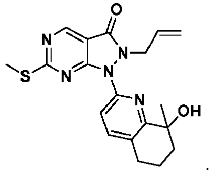

15. An application of the substance **X** in the preparation of kinase inhibitors or medicaments;
the substance **X** is the pyrazolone-fused pyrimidine compound represented by formula **I** according to at least one of claims 1-12, the pharmaceutically acceptable salt thereof, the solvate thereof or the solvate of the pharmaceutically acceptable salt thereof.

16. An application of the substance **X** in the preparation of a medicament; the medicament is used for treating and/or preventing diseases related to WEE1 kinase, or the medicament is used for treating and/or preventing cancer;
the substance **X** is the pyrazolone-fused pyrimidine compound represented by formula **I** according to at least one of claims 1-12, the pharmaceutically acceptable salt thereof, the solvate thereof or the solvate of the pharmaceutically acceptable salt thereof.

17. A pharmaceutical composition comprising substance **X** and pharmaceutical excipients;
the substance **X** is the pyrazolone-fused pyrimidine compound represented by formula **I** according to at least one of claims 1-12, the pharmaceutically acceptable salt thereof, the solvate thereof or the solvate of the pharmaceutically acceptable salt thereof.

18. A combination comprising substance **X** and an anticancer drug,
the substance **X** is the pyrazolone-fused pyrimidine compound represented by formula **I** according to at least one of claims 1-12, the pharmaceutically acceptable salt thereof, the solvate thereof or the solvate of the pharmaceutically acceptable salt thereof.

19. An application of the combination according to claim 18 in the preparation of a medicament for preventing and/or treating cancer.

20. An application of the substance **X** in the preparation of a medicament, the medicament in combination with an anticancer drug used for preventing and/or treating cancer;
the substance **X** is the pyrazolone-fused pyrimidine compound represented by formula **I** according to at least one of claims 1-12, the pharmaceutically acceptable salt thereof, the solvate thereof or the solvate of the pharmaceutically acceptable salt thereof.

21. An application of an anticancer drug in the preparation of a medicament, the medicament in combination with the substance **X** used for preventing and/or treating cancer;
the substance **X** is the pyrazolone-fused pyrimidine compound represented by formula **I** according to at least one of claims 1-12, the pharmaceutically acceptable salt thereof, the solvate thereof or the solvate of the pharmaceutically acceptable salt thereof.

22. A pharmaceutical composition comprising the combination according to claim 18 and pharmaceutical excipients.

23. A combination drug kit comprising a pharmaceutical composition A and a pharmaceutical composition B;
the pharmaceutical composition A comprises the substance **X,** and pharmaceutical excipients;
the pharmaceutical composition B comprises the anticancer drugs and pharmaceutical excipients;
the substance **X** is the pyrazolone-fused pyrimidine compound represented by formula **I** according to at least one of claims 1-12, the pharmaceutically acceptable salt thereof, the solvate thereof or the solvate of the pharmaceutically acceptable salt thereof.

24. A method for treating and/or preventing diseases comprising administering a therapeutically effective amount of substance **X** to a patient; the disease is a disease or cancer related to WEE1 kinase;
the substance **X** is the pyrazolone-fused pyrimidine compound represented by formula I according to at least one of claims 1-12, the pharmaceutically acceptable salt thereof, the solvate thereof, the solvate of the pharmaceutically acceptable salt thereof, the metabolite thereof or the prodrug thereof.

25. A method for treating and/or preventing cancer comprising administering a therapeutically effective amount of the combination according to claim 18 to a patient.
